# EUROPEAN PATENT APPLICATION

(11) **EP 4 471 031 A1**
(43) Date of publication of application: **04.12.2024**
(21) Application number: 23746453.2
(22) Date of filing: 29.01.2023
(51) Int. Cl.: C07D 471/22, C07D 487/22, A61K 31/55, A61K 31/519, A61P 35/00

(54) **CRYSTALLINE FORM OR AMORPHOUS FORM OF MACROCYCLIC COMPOUND OR SALT OR SOLVATE THEREOF**

(30) Priority: 30.01.2022 WO PCT/CN2022/075258
(71) Applicant: Ascentage Pharma (Suzhou) Co., Ltd., Suzhou, Jiangsu 215127 (CN); Ascentage Pharma Group Corp Limited, Hong Kong 999077 (CN)
(72) Inventor: LIN, Yanqiong, Suzhou, Jiangsu 215127 (CN); WEN, Jianfeng, Suzhou, Jiangsu 215127 (CN); FENG, Jianpeng, Suzhou, Jiangsu 215127 (CN); LI, Weidong, Suzhou, Jiangsu 215127 (CN); WANG, Chuanshen, Suzhou, Jiangsu 215127 (CN); LI, Zongbin, Suzhou, Jiangsu 215127 (CN)
(74) Representative: Plasseraud IP
(86) International application number: PCT/CN2023/073719
(87) International publication number: WO 2023/143576

(57) **Abstract**

Disclosed are a crystalline form or an amorphous form of a macrocyclic compound or a salt or solvate thereof, as well as a preparation method therefor and an application thereof. The structure of the macrocyclic compound is represented by formula I.

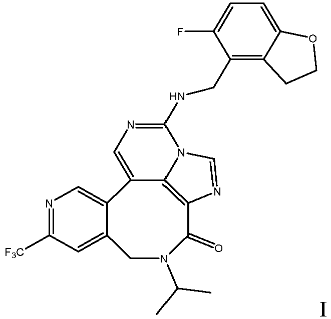

## Description

The present application claims the priority to the Chinese patent application PCT/CN2022/075258 filed on Jan. 1, 2022, which is incorporated herein by reference in its entirety.

### TECHNICAL FIELD

The present invention belongs to the field of pharmaceutical chemistry, and particularly relates to a crystalline form or an amorphous form of a macrocyclic compound, or a salt or solvate thereof, a preparation method therefor and use thereof.

### BACKGROUND

Polycomb group (PcG) proteins are a class of chromatin-modifying enzymes that are dysregulated in many human cancers. Polycomb repressive complex2 (PRC2) comprises SUZ12 (an inhibitor of zeste 12), EED, and a catalytic subunit EZH2 (an enhancer factor of zeste homolog 2), and inhibits genes by methylating the promoter region of the target gene and its surrounding core histone H3 lysine 27 (H3K27me3). PRC2 is an important component of cellular mechanisms involved in epigenetic regulation of gene transcription, playing a key role in development, tissue differentiation and regeneration (Moritz and Trievel, J. Biol. Chem 293(36):13805-13814 (2018); Fiskus et al., Mol Cancer Ther 5(12):3096-3014 (2006)).

The methyltransferase activity of PRC2 requires the involvement of at least EED and SUZ12. EED, SUZ12, and EZH2 are overexpressed in many cancers, including but not limited to breast cancer, prostate cancer, and hepatocellular carcinoma. There is a need in the art for small molecules that inhibit the activity of EED for the treatment of cancer and other diseases.

In WO2021011713A1, a compound of formula I is disclosed, which is a potent inhibitor of EED and can be used for the treatment or prevention of one or more diseases mediated by the overexpression of EED. 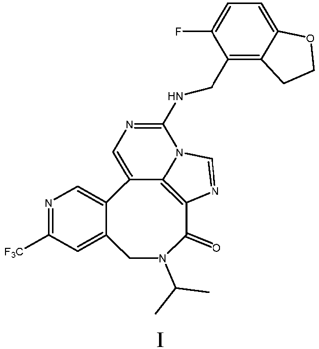 Generally, the low purity, low solubility and poor stability of the pharmaceutically active ingredient will affect its absorption in the body, which results in low bioavailability and is not conducive to further drug development. The structure of the pharmaceutically active ingredient such as salt form, solvate or crystalline form thereof often affects the physical and chemical properties of the pharmaceutically active ingredient.

To date, no salt of the compound of formula I or crystalline form thereof has been reported. Therefore, it is necessary to improve various properties of the compound of formula I by preparing salts or crystalline forms thereof.

### SUMMARY

The present invention aims to provide a crystalline form or an amorphous form of a macrocyclic compound, or a salt or solvate thereof to overcome the defects of poor crystallinity, solubility, purity and/or stability of the existing EED inhibitor, thereby solving the technical problem. The crystalline form or the amorphous form of the macrocyclic compound, or the salt or solvate thereof of the present invention has one or more of the following advantages: high crystallinity, good stability, low hygroscopicity, easy formation of solvates, and difficulty in preparation.

The present invention solves the above technical problems by the following technical solutions.

The present invention provides a crystalline form A of a compound of formula I, which has an X-ray powder diffraction (XRPD) pattern having at least three, at least four, at least five, at least six, or at least seven characteristic peaks at the following 2Θ angles: 5.181° ± 0.2°, 6.254° ± 0.2°, 8.708° ± 0.2°, 11.496° ± 0.2°, 11.743° ± 0.2°, 16.538° ± 0.2°, and 20.361° ± 0.2°;

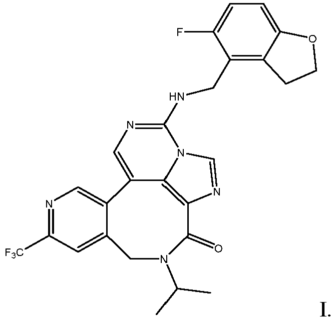

In one embodiment, the crystalline form A of the compound of formula I has an X-ray powder diffraction pattern having characteristic peaks at the following 2Θ angles: 5.181° ± 0.2°, 16.538° ± 0.2°, and 20.361° ± 0.2°.

In one embodiment, the crystalline form A of the compound of formula I has an X-ray powder diffraction pattern having characteristic peaks at the following 2Θ angles: 5.181° ± 0.2°, 11.743° ± 0.2°, 16.538° ± 0.2°, and 20.361° ± 0.2°.

In one embodiment, the crystalline form A of the compound of formula I has an X-ray powder diffraction pattern having characteristic peaks at the following 2Θ angles: 5.181° ± 0.2°, 6.254° ± 0.2°, 11.743° ± 0.2°, 16.538° ± 0.2°, and 20.361° ± 0.2°.

In one embodiment, the crystalline form A of the compound of formula I has an X-ray powder diffraction pattern having characteristic peaks at the following 2Θ angles: 5.181° ± 0.2°, 6.254° ± 0.2°, 11.496° ± 0.2°, 11.743° ± 0.2°, 16.538° ± 0.2°, and 20.361° ± 0.2°.

In one embodiment, the crystalline form A of the compound of formula I has an X-ray powder diffraction pattern having characteristic peaks at the following 2Θ angles: 5.181° ± 0.2°, 6.254° ± 0.2°, 8.708° ± 0.2°, 11.496° ± 0.2°, 11.743° ± 0.2°, 16.538° ± 0.2°, and 20.361° ± 0.2°.

In one embodiment, the crystalline form A of the compound of formula I has an X-ray powder diffraction pattern having characteristic peaks at the following 2Θ angles: 5.181° ± 0.2°, 6.254° ± 0.2°, 8.708° ± 0.2°, 10.304° ± 0.2°, 11.496° ± 0.2°, 11.743° ± 0.2°, 16.538° ± 0.2°, 18.275° ± 0.2°, 18.58° ± 0.2°, 20.361° ± 0.2°, 21.113° ± 0.2°, 23.495° ± 0.2°, 24.232° ± 0.2°, 26.337° ± 0.2°, and 26.767° ± 0.2°.

In one embodiment, the crystalline form A of the compound of formula I has an X-ray powder diffraction pattern having characteristic peaks at the following 2Θ angles: 5.181° ± 0.2°, 6.254° ± 0.2°, 7.052° ± 0.2°, 8.708° ± 0.2°, 10.304° ± 0.2°, 10.633° ± 0.2°, 11.496° ± 0.2°, 11.743° ± 0.2°, 12.466° ± 0.2°, 12.849° ± 0.2°, 13.224° ± 0.2°, 14.047° ± 0.2°, 14.784° ± 0.2°, 15.004° ± 0.2°, 15.917° ± 0.2°, 16.538° ± 0.2°, 17.529° ± 0.2°, 17.726° ± 0.2°, 18.275° ± 0.2°, 18.58° ± 0.2°, 19.083° ± 0.2°, 19.291° ± 0.2°, 19.848° ± 0.2°, 20.361° ± 0.2°, 21.113° ± 0.2°, 22.221° ± 0.2°, 22.458° ± 0.2°, 23.066° ± 0.2°, 23.495° ± 0.2°, 23.743° ± 0.2°, 24.232° ± 0.2°, 25.19° ± 0.2°, 25.885° ± 0.2°, 26.337° ± 0.2°, 26.767° ± 0.2°, 27.119° ± 0.2°, 27.832° ± 0.2°, 28.188° ± 0.2°, 29.263° ± 0.2°, and 30.363° ± 0.2°.

In one embodiment, the crystalline form A of the compound of formula I has X-ray powder diffraction pattern analysis data substantially as shown in Table 1:

**Table 1. X-ray powder diffraction pattern analysis data for the crystalline form A of the compound of formula I**

| Position [°2θ] ± 0.2° | d-spacing [Å] | Relative intensity [%] |
|---|---|---|
| 5.181 | 17.042 | 100 |
| 6.254 | 14.1202 | 25.2 |
| 7.052 | 12.5243 | 6 |
| 8.708 | 10.1458 | 22.9 |
| 10.304 | 8.5777 | 18.8 |
| 10.633 | 8.3134 | 7 |
| 11.496 | 7.691 | 23.7 |
| 11.743 | 7.5296 | 29.6 |
| 12.466 | 7.0947 | 9.4 |
| 12.849 | 6.8842 | 4.3 |
| 13.224 | 6.6896 | 6.2 |
| 14.047 | 6.2996 | 6.5 |
| 14.784 | 5.9869 | 3.8 |
| 15.004 | 5.9 | 7.2 |
| 15.917 | 5.5632 | 8.8 |
| 16.538 | 5.3557 | 43 |
| 17.529 | 5.0553 | 9.2 |
| 17.726 | 4.9996 | 5.4 |
| 18.275 | 4.8505 | 13.6 |
| 18.58 | 4.7716 | 14.4 |
| 19.083 | 4.647 | 7.2 |
| 19.291 | 4.5973 | 4.6 |
| 19.848 | 4.4695 | 6.5 |
| 20.361 | 4.358 | 32.2 |
| 21.113 | 4.2044 | 13.9 |
| 22.221 | 3.9973 | 4.8 |
| 22.458 | 3.9556 | 5.4 |
| 23.066 | 3.8527 | 8.8 |
| 23.495 | 3.7834 | 17.4 |
| 23.743 | 3.7443 | 7.7 |
| 24.232 | 3.6699 | 13.8 |
| 25.19 | 3.5325 | 7.2 |
| 25.885 | 3.4392 | 8.3 |
| 26.337 | 3.3812 | 10.6 |
| 26.767 | 3.3278 | 12.4 |
| 27.119 | 3.2855 | 6.4 |
| 27.832 | 3.2028 | 3.7 |
| 28.188 | 3.1631 | 3.7 |
| 29.263 | 3.0494 | 4.3 |
| 30.363 | 2.9414 | 5.6 |

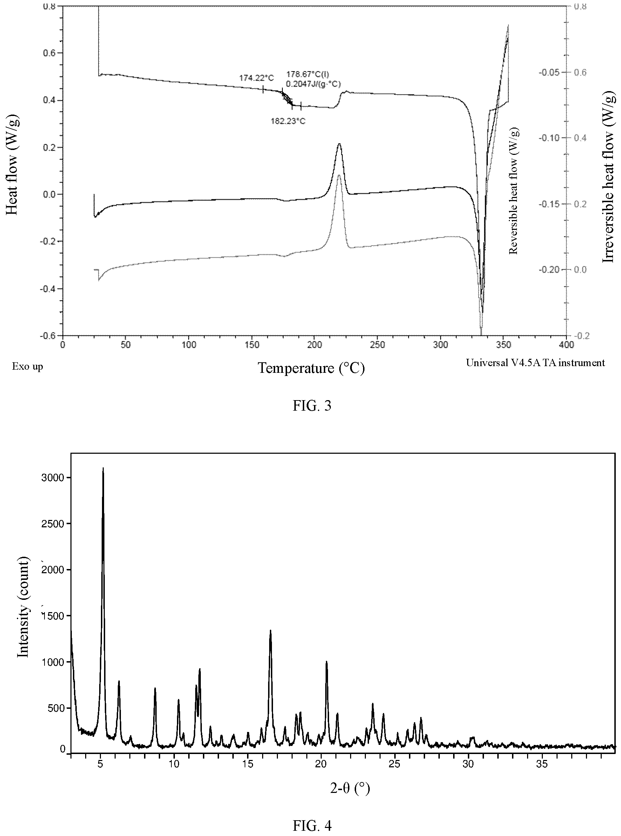

In one embodiment, the crystalline form A of the compound of formula I has an X-ray powder diffraction pattern substantially as shown in FIG. 4.

In one embodiment, the crystalline form A of the compound of formula I has a thermogravimetric analysis (TGA) curve showing a weight loss of 0.1264% ± 0.2% during heating from 29.6 °C ± 3 °C to 150.14 °C ± 3 °C.

In one embodiment, the crystalline form A of the compound of formula I has a thermogravimetric analysis curve showing a weight loss of 0.1264% during heating from 29.6 °C to 150.14 °C.

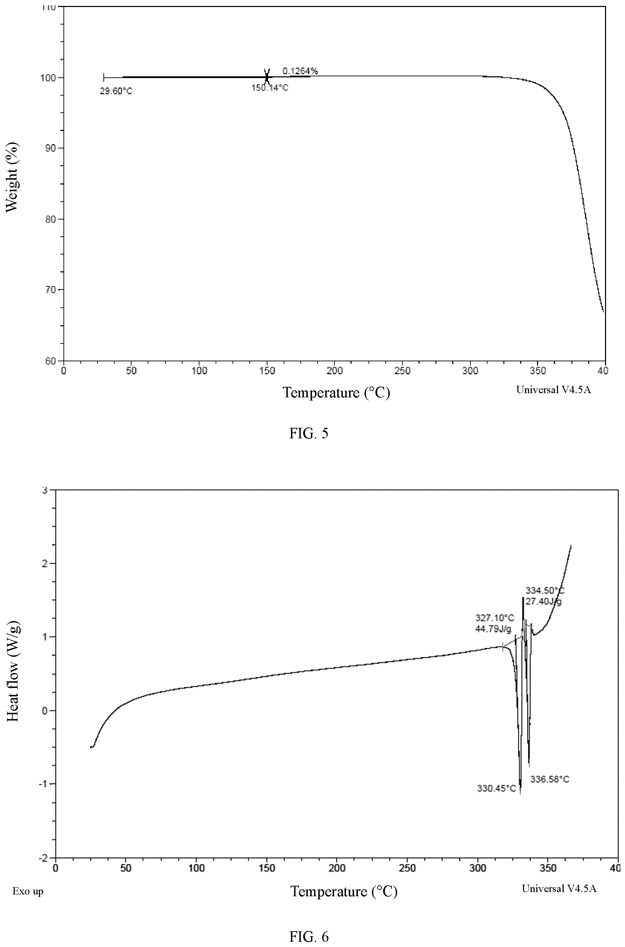

In one embodiment, the crystalline form A has a thermogravimetric analysis curve substantially as shown in FIG. 5.

In one embodiment, the crystalline form A of the compound of formula I has a differential scanning calorimetry (DSC) curve having endothermic peaks with initial temperatures of 327.1 °C ± 3 °C and 334.5 °C ± 3 °C, respectively.

In one embodiment, the crystalline form A of the compound of formula I has a differential scanning calorimetry curve having endothermic peaks with initial temperatures of 327.1 °C and 334.5 °C, respectively.

In one embodiment, the crystalline form A of the compound of formula I has a differential scanning calorimetry curve having endothermic peaks with peak temperatures of 330.45 °C ± 3 °C and 336.58 °C ± 3 °C, respectively.

In one embodiment, the crystalline form A of the compound of formula I has a differential scanning calorimetry curve having endothermic peaks with peak temperatures of 330.45 °C and 336.58 °C, respectively.

In one embodiment, the crystalline form A of the compound of formula I has a differential scanning calorimetry curve substantially as shown in FIG. 6.

In one embodiment, the crystalline form A of the compound of formula I has a dynamic vapor sorption (DVS) curve showing a hygroscopic weight gain of 0.44% ± 0.2% at 25 °C and 80% RH.

In one embodiment, the crystalline form A of the compound of formula I has a dynamic vapor sorption curve showing a hygroscopic weight gain of 0.44% at 25 °C and 80% RH.

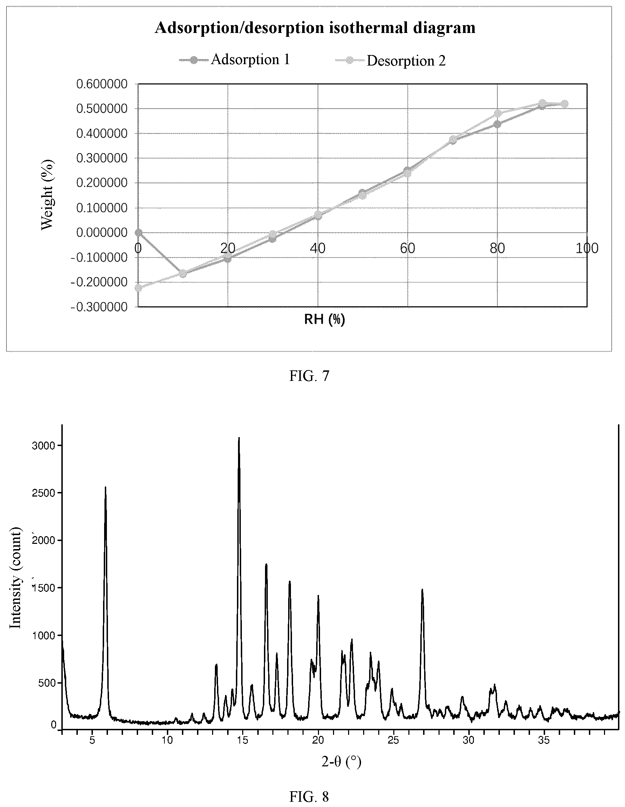

In one embodiment, the crystalline form A of the compound of formula I has a dynamic vapor sorption curve as shown in FIG. 7.

The present invention further provides a preparation method for the crystalline form A of the compound of formula I, preferably any one of the following methods:
method I comprising the following steps: stirring a crystalline form B of a compound of formula I in a solvent to precipitate a solid, and separating and drying the solid to obtain the crystalline form A of the compound of formula I, wherein the solvent is an organic solvent or a mixed solvent of an organic solvent and water; the organic solvent is selected from one or more of methanol, ethanol, dichloromethane, and acetonitrile; the stirring is preferably performed at a temperature of 20-60 °C; the stirring is preferably performed for a period of 1.5-2.5 days; the crystalline form B of the compound of formula I and the solvent are preferably in a mass-to-volume ratio of (250 mg-350 mg):1 mL; when the solvent is a mixed solvent of an organic solvent and water, the organic solvent and the water are preferably in a volume ratio of (0.5-3.5):1; wherein when the organic solvent is ethanol, the stirring is performed at a temperature of 40-60 °C; when the solvent is a mixed solvent of methanol and water in a volume ratio of (2.5-3.5):1, the stirring is performed at a temperature of 20-30 °C; and when the solvent is a mixed solvent of methanol and water in a volume ratio of (0.5-1.5):1, the stirring is performed at a temperature of 40-60 °C;
method II comprising the following steps: stirring a crystalline form B of a compound of formula I in dichloromethane to obtain a mixed solution, adding an anti-solvent and stirring the solution to precipitate a solid, and separating and drying the solid to obtain the crystalline form A of the compound of formula I, wherein the anti-solvent is selected from one or more of ethanol, tetrahydrofuran, and ethyl acetate; the stirring is preferably performed at a temperature of 20-60 °C; the stirring is preferably performed for a period of 1.5-2.5 days; the crystalline form B of the compound of formula I and the dichloromethane are preferably in a mass-to-volume ratio of (50 mg-200 mg):1 mL; and the dichloromethane and the anti-solvent are preferably in a volume ratio of (0.5-2.5):1;
method III comprising the following steps: stirring an amorphous form of a compound of formula I in a solvent to precipitate a solid, and separating and drying the solid to obtain the crystalline form A of the compound of formula I, wherein the solvent is an organic solvent or a mixed solvent of an organic solvent and water; the organic solvent is selected from one or more of methanol, ethanol, acetonitrile, isopropanol, and dichloromethane; the stirring is preferably performed at a temperature of 20-60 °C; the stirring is preferably performed for a period of 1.5-2.5 days; the amorphous form of the compound of formula I and the solvent are preferably in a mass-to-volume ratio of (200 mg-350 mg):1 mL; when the solvent is a mixed solvent of an organic solvent and water, the organic solvent and the water are preferably in a volume ratio of (0.5-3.5):1; wherein when the solvent is a mixed solvent of methanol and water, the methanol and the water are in a volume ratio of (2.5-3.5):1; and when the solvent is ethanol or a mixed solvent of ethanol and water in a volume ratio of 1:1, the stirring is performed at a temperature of 40-60 °C;
method IV comprising the following steps: stirring a crystalline form A of a hydrochloride salt of a compound of formula I in a mixed solvent of an organic solvent and water to precipitate a solid, and separating and drying the solid to obtain the crystalline form A of the compound of formula I, wherein the organic solvent is selected from one or two of methanol and ethanol; the stirring is preferably performed at a temperature of 35-45 °C; the stirring is preferably performed for a period of 2.5-3.5 days; the hydrochloride salt of the compound of formula I and the mixed solvent are preferably in a mass-to-volume ratio of (100 mg-200 mg):1 mL; and the organic solvent and the water are preferably in a volume ratio of (2.5-3.5):1;
method V comprising the following steps: stirring a crystalline form A of a sulfate salt of a compound of formula I in a solvent to precipitate a solid, and separating and drying the solid to obtain the crystalline form A of the compound of formula I, wherein the solvent is a mixed solvent of methanol, an organic solvent and water or a mixed solvent of methanol and dichloromethane; the organic solvent is selected from one or more of methanol, ethanol, and acetonitrile; the stirring is preferably performed at a temperature of 35-45 °C; the stirring is preferably performed for a period of 2.5-3.5 days; the crystalline form A of the sulfate salt of the compound of formula I and the solvent are preferably in a mass-to-volume ratio of (100 mg-200 mg):1 mL; and the organic solvent and the water are preferably in a volume ratio of (0.5-3.5):1;
method VI comprising the following steps: mixing a crystalline form A of a sulfate salt of a compound of formula I with methanol, adding heptane to precipitate a solid and stirring, and separating and drying the solid to obtain the crystalline form A of the compound of formula I, wherein the stirring is preferably performed at a temperature of 20-30 °C; the stirring is preferably performed for a period of 0.5-1.5 days; and the crystalline form A of the sulfate salt of the compound of formula I and the methanol are preferably in a mass-to-volume ratio of (30 mg-40 mg):1 mL;
method VII comprising the following steps: stirring a crystalline form A of a methanesulfonate salt of a compound of formula I in a solvent to precipitate a solid, and separating and drying the solid to obtain the crystalline form A of the compound of formula I, wherein the solvent is water or a mixed solvent of an organic solvent and water; the organic solvent is selected from one or two of methanol and acetonitrile; the stirring is preferably performed at a temperature of 35-45 °C; the stirring is preferably performed for a period of 2.5-3.5 days; and the crystalline form A of the methanesulfonate salt of the compound of formula I and the solvent are preferably in a mass-to-volume ratio of (100 mg-200 mg):1 mL;
method VIII comprising the following steps: mixing a crystalline form A of a methanesulfonate salt of a compound of formula I with DMF, adding acetonitrile to precipitate a solid and stirring, and separating and drying the solid to obtain the crystalline form A of the compound of formula I, wherein the stirring is preferably performed at a temperature of 20-30 °C; the stirring is preferably performed for a period of 12-16 h; and the methanesulfonate salt of the compound of formula I and DMF are preferably in a mass-to-volume ratio of (30 mg-70 mg):1 mL; and
method IX comprising the following steps: stirring a crystalline form A of a methanesulfonate salt of a compound of formula I in a solvent until a clear solution is obtained, and volatilizing and drying the solution to obtain the crystalline form A of the compound of formula I, wherein the solvent is methanol or a mixed solvent of ethanol and methyl acetate; the organic solvent is selected from one or two of methanol and acetonitrile; the volatilizing and drying are preferably performed at a temperature of 20-30 °C; and the ethanol and the methyl acetate are preferably in a volume ratio of (1.5-2.5):1.

In one embodiment, in the method I, the stirring is preferably performed at a temperature of 25 °C or 50 °C. The stirring is preferably performed for a period of 2 days. The crystalline form B of the compound of formula I and the solvent are preferably in a mass-to-volume ratio of 300 mg:1 mL. When the solvent is a mixed solvent of an organic solvent and water, the organic solvent and the water are preferably in a volume ratio of 1:1 or 3:1. When the organic solvent is ethanol, the stirring is preferably performed at a temperature of 50 °C. When the solvent is a mixed solvent of methanol and water in a volume ratio of 3:1, the stirring is preferably performed at a temperature of 25 °C. When the solvent is a mixed solvent of methanol and water in a volume ratio of 1:1, the stirring is preferably performed at a temperature of 50 °C.

In one embodiment, in the method II, the stirring is preferably performed at a temperature of 25 °C or 50 °C. The stirring is preferably performed for a period of 2 days. The crystalline form B of the compound of formula I and the dichloromethane are preferably in a mass-to-volume ratio of 100 mg:1 mL or 180 mg:1 mL. The dichloromethane and the anti-solvent are preferably in a volume ratio of 1:1 or 2:1.

In one embodiment, in the method III, the stirring is preferably performed at a temperature of 25 °C or 50 °C. The stirring is preferably performed for a period of 2 days. The amorphous form of the compound of formula I and the solvent are preferably in a mass-to-volume ratio of 250 mg:1 mL or 300 mg:1 mL. When the solvent is a mixed solvent of an organic solvent and water, the organic solvent and the water are preferably in a volume ratio of 1:1 or 3:1. When the solvent is a mixed solvent of methanol and water, the methanol and water are preferably in a volume ratio of 3:1. When the solvent is ethanol or a mixed solvent of ethanol and water in a volume ratio of 1:1, the stirring is preferably performed at a temperature of 50 °C.

In one embodiment, in the method IV, the stirring is preferably performed at a temperature of 40 °C. The stirring is preferably performed for a period of 3 days. The hydrochloride salt of the compound of formula I and the mixed solvent are preferably in a mass-to-volume ratio of 150 mg:1 mL. The organic solvent and the water are preferably in a volume ratio of 3:1.

In one embodiment, in the method V, the stirring is preferably performed at a temperature of 40 °C. The stirring is preferably performed for a period of 3 days. The sulfate salt of the compound of formula I and the solvent are preferably in a mass-to-volume ratio of 150 mg:1 mL. The organic solvent and the water are preferably in a volume ratio of 1:1 or 3:1.

In one embodiment, in the method VI, the stirring is preferably performed at a temperature of 25 °C. The stirring is preferably performed for a period of 1 day. The sulfate salt of the compound of formula I and the methanol are preferably in a mass-to-volume ratio of 35.71 mg:1 mL.

In one embodiment, in the method VII, the stirring is preferably performed at a temperature of 40 °C. The stirring is preferably performed for a period of 3 days. The methanesulfonate salt of the compound of formula I and the solvent are preferably in a mass-to-volume ratio of 150 mg:1 mL.

In one embodiment, in the method VIII, the stirring is preferably performed at a temperature of 25 °C. The stirring is preferably performed for a period of 14 h. The methanesulfonate salt of the compound of formula I and the DMF are preferably in a mass-to-volume ratio of 50 mg:1 mL.

In one embodiment, in the method IX, the volatilizing and drying are preferably performed at a temperature of 25 °C. The ethanol and the methyl acetate are preferably in a volume ratio of 2:1.

The present invention provides a crystalline form B of a compound of formula I, which has an X-ray powder diffraction (XRPD) pattern having at least three, at least four, at least five, at least six, or at least seven characteristic peaks at the following 2Θ angles: 5.884° ± 0.2°, 14.747° ± 0.2°, 16.575° ± 0.2°, 18.116° ± 0.2°, 19.987° ± 0.2°, 22.225° ± 0.2°, and 26.884° ± 0.2°;

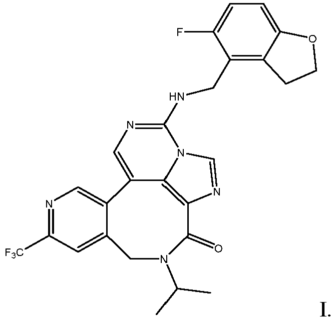

In one embodiment, the crystalline form B of the compound of formula I has an X-ray powder diffraction pattern having characteristic peaks at the following 2Θ angles: 5.884° ± 0.2°, 14.747° ± 0.2°, and 16.575° ± 0.2°.

In one embodiment, the crystalline form B of the compound of formula I has an X-ray powder diffraction pattern having characteristic peaks at the following 2Θ angles: 5.884° ± 0.2°, 14.747° ± 0.2°, 16.575° ± 0.2°, and 18.116° ± 0.2°.

In one embodiment, the crystalline form B of the compound of formula I has an X-ray powder diffraction pattern having characteristic peaks at the following 2Θ angles: 5.884° ± 0.2°, 14.747° ± 0.2°, 16.575° ± 0.2°, 18.116° ± 0.2°, and 26.884° ± 0.2°.

In one embodiment, the crystalline form B of the compound of formula I has an X-ray powder diffraction pattern having characteristic peaks at the following 2Θ angles: 5.884° ± 0.2°, 14.747° ± 0.2°, 16.575° ± 0.2°, 18.116° ± 0.2°, 19.987° ± 0.2°, and 26.884° ± 0.2°.

In one embodiment, the crystalline form B of the compound of formula I has an X-ray powder diffraction pattern having characteristic peaks at the following 2Θ angles: 5.884° ± 0.2°, 14.747° ± 0.2°, 16.575° ± 0.2°, 18.116° ± 0.2°, 19.987° ± 0.2°, 22.225° ± 0.2°, and 26.884° ± 0.2°.

In one embodiment, the crystalline form B of the compound of formula I has an X-ray powder diffraction pattern having characteristic peaks at the following 2Θ angles: 5.884° ± 0.2°, 13.263° ± 0.2°, 14.747° ± 0.2°, 16.575° ± 0.2°, 17.259° ± 0.2°, 18.116° ± 0.2°, 19.558° ± 0.2°, 19.987° ± 0.2°, 21.566° ± 0.2°, 22.225° ± 0.2°, 23.456° ± 0.2°, 23.999° ± 0.2°, and 26.884° ± 0.2°.

In one embodiment, the crystalline form B of the compound of formula I has an X-ray powder diffraction pattern having characteristic peaks at the following 2Θ angles: 5.884° ± 0.2°, 13.263° ± 0.2°, 13.868° ± 0.2°, 14.299° ± 0.2°, 14.747° ± 0.2°, 15.622° ± 0.2°, 16.575° ± 0.2°, 17.259° ± 0.2°, 18.116° ± 0.2°, 19.558° ± 0.2°, 19.987° ± 0.2°, 21.566° ± 0.2°, 22.225° ± 0.2°, 23.258° ± 0.2°, 23.456° ± 0.2°, 23.999° ± 0.2°, 24.894° ± 0.2°, 26.884° ± 0.2°, 29.551° ± 0.2°, 31.442° ± 0.2°, and 31.695° ± 0.2°.

In one embodiment, the crystalline form B of the compound of formula I has an X-ray powder diffraction pattern having characteristic peaks at the following 2Θ angles: 5.884° ± 0.2°, 10.54° ± 0.2°, 11.629° ± 0.2°, 12.427° ± 0.2°, 13.263° ± 0.2°, 13.868° ± 0.2°, 14.299° ± 0.2°, 14.747° ± 0.2°, 15.622° ± 0.2°, 16.575° ± 0.2°, 17.259° ± 0.2°, 18.116° ± 0.2°, 19.558° ± 0.2°, 19.987° ± 0.2°, 21.566° ± 0.2°, 22.225° ± 0.2°, 23.258° ± 0.2°, 23.456° ± 0.2°, 23.999° ± 0.2°, 24.894° ± 0.2°, 25.501° ± 0.2°, 26.884° ± 0.2°, 27.328° ± 0.2°, 27.7° ± 0.2°, 28.048° ± 0.2°, 28.557° ± 0.2°, 29.551° ± 0.2°, 30.497° ± 0.2°, 30.859° ± 0.2°, 31.442° ± 0.2°, 31.695° ± 0.2°, 32.436° ± 0.2°, 33.328° ± 0.2°, 34.071° ± 0.2°, 34.715° ± 0.2°, 35.553° ± 0.2°, 35.842° ± 0.2°, and 36.347° ± 0.2°.

In one embodiment, the crystalline form B of the compound of formula I has X-ray powder diffraction pattern analysis data substantially as shown in Table 2:

**Table 2. X-ray powder diffraction pattern analysis data for the crystalline form B of the compound of formula I**

| Position [°2θ] ± 0.2° | d-spacing [Å] | Relative intensity [%] |
|---|---|---|
| 5.884 | 15.0089 | 83.1 |
| 10.54 | 8.3861 | 3.9 |
| 11.629 | 7.6036 | 5.5 |
| 12.427 | 7.1168 | 5.5 |
| 13.263 | 6.67 | 22.3 |
| 13.868 | 6.3803 | 11.6 |
| 14.299 | 6.1889 | 13.8 |
| 14.747 | 6.0021 | 100 |
| 15.622 | 5.6676 | 15.3 |
| 16.575 | 5.3439 | 56.6 |
| 17.259 | 5.1336 | 26.1 |
| 18.116 | 4.8927 | 50.7 |
| 19.558 | 4.5351 | 24 |
| 19.987 | 4.4388 | 45.8 |
| 21.566 | 4.1172 | 27 |
| 22.225 | 3.9966 | 30.9 |
| 23.258 | 3.8213 | 15.7 |
| 23.456 | 3.7896 | 26.4 |
| 23.999 | 3.7051 | 23.3 |
| 24.894 | 3.5738 | 14 |
| 25.501 | 3.4901 | 8.8 |
| 26.884 | 3.3136 | 47.9 |
| 27.328 | 3.2607 | 8.4 |
| 27.7 | 3.2178 | 6.8 |
| 28.048 | 3.1787 | 6.9 |
| 28.557 | 3.1231 | 7.9 |
| 29.551 | 3.0203 | 11.2 |
| 30.497 | 2.9288 | 6 |
| 30.859 | 2.8952 | 6.3 |
| 31.442 | 2.8428 | 14 |
| 31.695 | 2.8207 | 15.4 |
| 32.436 | 2.758 | 9.8 |
| 33.328 | 2.6861 | 8.2 |
| 34.071 | 2.6293 | 7.3 |
| 34.715 | 2.5819 | 8.1 |
| 35.553 | 2.523 | 7.5 |
| 35.842 | 2.5033 | 7.2 |
| 36.347 | 2.4697 | 7 |

In one embodiment, the crystalline form B of the compound of formula I has an X-ray powder diffraction pattern substantially as shown in FIG. 8.

In one embodiment, the crystalline form B of the compound of formula I has a thermogravimetric analysis (TGA) curve showing a weight loss of 0.1817% ± 0.2% during heating from 28.73 °C ± 3 °C to 149.63 °C ± 3 °C.

In one embodiment, the crystalline form B of the compound of formula I has a thermogravimetric analysis curve showing a weight loss of 0.1817% during heating from 28.73 °C to 149.63 °C.

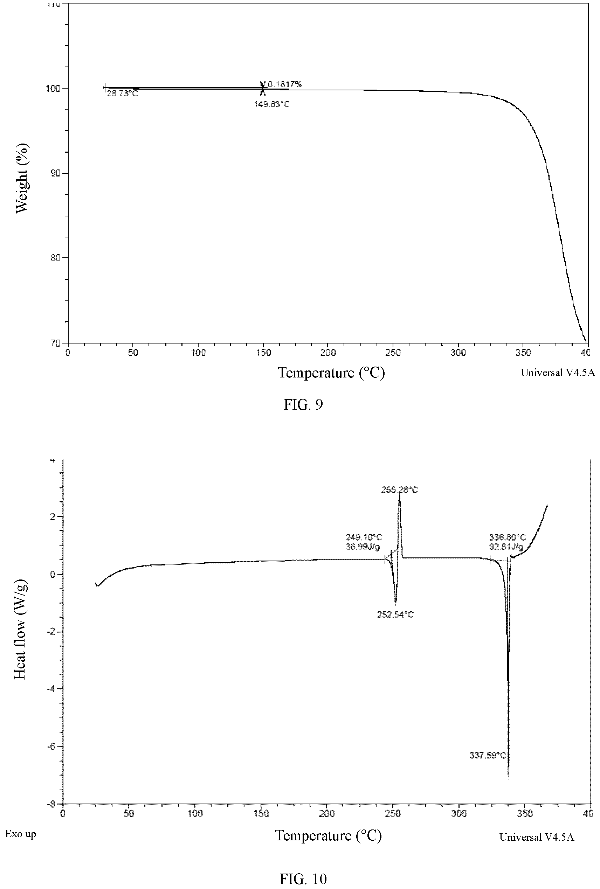

In one embodiment, the crystalline form B of the compound of formula I has a thermogravimetric analysis curve substantially as shown in FIG. 9.

In one embodiment, the crystalline form B of the compound of formula I has a differential scanning calorimetry (DSC) curve having endothermic peaks with initial temperatures of 249.1 °C ± 3 °C and 336.8 °C ± 3 °C, respectively.

In one embodiment, the crystalline form B of the compound of formula I has a differential scanning calorimetry curve having endothermic peaks with initial temperatures of 249.1 °C and 336.8 °C, respectively.

In one embodiment, the crystalline form B of the compound of formula I has a differential scanning calorimetry curve having endothermic peaks with peak temperatures of 252.54 °C ± 3 °C and 337.59 °C ± 3 °C, respectively.

In one embodiment, the crystalline form B of the compound of formula I has a differential scanning calorimetry curve having endothermic peaks with peak temperatures of 252.54 °C and 337.59 °C, respectively.

In one embodiment, the crystalline form B of the compound of formula I has a differential scanning calorimetry curve having an exothermic peak with a peak temperature of 255.28 °C ± 3 °C.

In one embodiment, the crystalline form B of the compound of formula I has a differential scanning calorimetry curve having an exothermic peak with a peak temperature of 255.28 °C.

In one embodiment, the crystalline form B of the compound of formula I has a differential scanning calorimetry curve substantially as shown in FIG. 10.

In one embodiment, the crystalline form B of the compound of formula I has a dynamic vapor sorption (DVS) curve showing a hygroscopic weight gain of 0.84% ± 0.2% at 25 °C and 80% RH.

In one embodiment, the crystalline form B of the compound of formula I has a dynamic vapor sorption curve showing a hygroscopic weight gain of 0.84% at 25 °C and 80% RH.

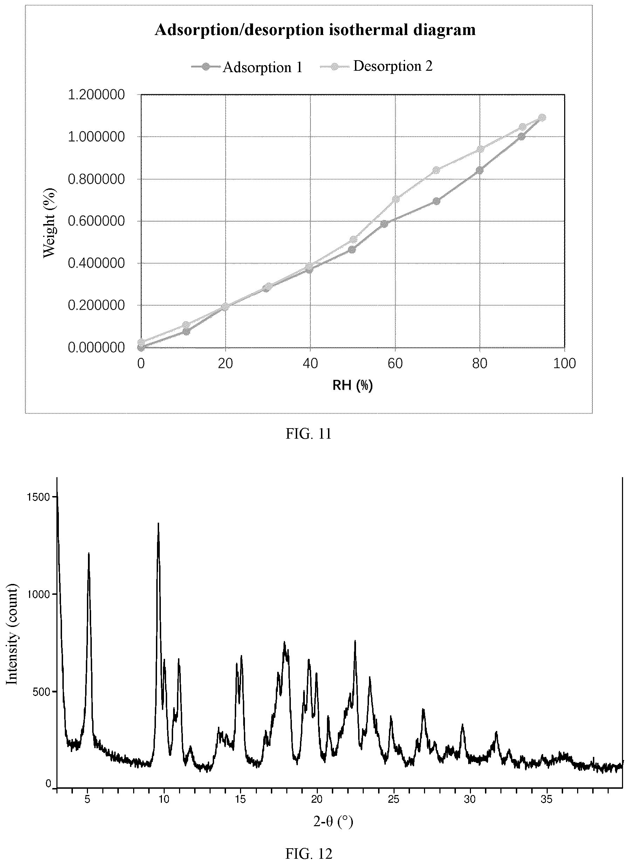

In one embodiment, the crystalline form B of the compound of formula I has a dynamic vapor sorption curve as shown in FIG. 11.

The present invention further provides a preparation method for the crystalline form B of the compound of formula I, preferably any one of the following methods:
method I comprising the following steps: stirring a crystalline form A of a compound of formula I in an organic solvent to precipitate a solid, and separating and drying the solid to obtain the crystalline form B of the compound of formula I, wherein the organic solvent is selected from one or more of ethyl acetate, acetone, 88% acetone, and tetrahydrofuran; the stirring is preferably performed at 20-60 °C; the stirring is preferably performed for a period of 1.5-2.5 days; the crystalline form A of the compound of formula I and the organic solvent are preferably in a mass-to-volume ratio of (80 mg-250 mg):1 mL; wherein when the organic solvent is tetrahydrofuran, the stirring is performed at a temperature of 35-45 °C;
method II comprising the following steps: stirring an amorphous form of a compound of formula I in a solvent to precipitate a solid, and separating and drying the solid to obtain the crystalline form B of the compound of formula I, wherein the solvent is an organic solvent or a mixed solvent of acetone and water in a volume ratio of (0.25-0.75):1; the organic solvent is selected from one or more of ethanol, ethyl acetate, acetone, methyl isobutyl ketone, and methyl tert-butyl ether; the stirring is preferably performed at a temperature of 20-60 °C; the stirring is preferably performed for a period of 1.5-2.5 days; the amorphous form of the compound of formula I and the solvent are preferably in a mass-to-volume ratio of (250 mg-350 mg):1 mL; wherein when the organic solvent is ethyl acetate or methyl *tert*-butyl ether, the stirring is performed at a temperature of 40-60 °C; and when the organic solvent is ethanol or methyl isobutyl ketone, the stirring is performed at a temperature of 20-30 °C;
method III comprising the following steps: stirring a crystalline form A of a hydrochloride salt of a compound of formula I or a crystalline form A of a sulfate salt of a compound of formula I in 88% acetone to precipitate a solid, and separating and drying the solid to obtain the crystalline form B of the compound of formula I, wherein the stirring is preferably performed at a temperature of 35-45 °C; the stirring is preferably performed for a period of 2.5-3.5 days; the crystalline form A of the hydrochloride salt of the compound of formula I or the crystalline form A of the sulfate salt of the compound of formula I and the 88% acetone are preferably in a mass-to-volume ratio of (100 mg-200 mg):1 mL;
method IV comprising the following steps: mixing a crystalline form A of a sulfate salt of a compound of formula I with methanol, adding acetone to precipitate a solid and stirring, and separating and drying the solid to obtain the crystalline form B of the compound of formula I, wherein the stirring is preferably performed at a temperature of 20-30 °C; the stirring is preferably performed for a period of 0.5-1.5 days; and the crystalline form A of the sulfate salt of the compound of formula I and the methanol are preferably in a mass-to-volume ratio of (30 mg-40 mg):1 mL; and
method V comprising the following steps: stirring a crystalline form A of a methanesulfonate salt of a compound of formula I in a solvent until a clear solution is obtained, and volatilizing and drying the solution to obtain the crystalline form B of the compound of formula I, wherein the solvent is ethanol or a mixed solvent of methanol and an organic solvent; the organic solvent is selected from one or two of methyl acetate and dichloromethane; the volatilizing and drying are preferably performed at a temperature of 20-30 °C; and the ethanol and the methyl acetate are preferably in a volume ratio of (1.5-2.5):1.

In one embodiment, in the method I, the stirring is preferably performed at a temperature of 25 °C, 40 °C, or 50 °C. The stirring is preferably performed for a period of 2 days. The crystalline form A of the compound of formula I and the organic solvent are preferably in a mass-to-volume ratio of 100 mg:1 mL, 150 mg:1 mL, or 200 mg:1 mL. When the organic solvent is tetrahydrofuran, the stirring is preferably performed at a temperature of 40 °C.

In one embodiment, in the method II, the acetone and the water are preferably in a volume ratio of 1:2. The stirring is preferably performed at a temperature of 25 °C or 50 °C. The stirring is preferably performed for a period of 2 days. The amorphous form of the compound of formula I and the solvent are preferably in a mass-to-volume ratio of 300 mg:1 mL. When the organic solvent is ethyl acetate or methyl tert-butyl ether, the stirring is preferably performed at a temperature of 50 °C. When the organic solvent is ethanol or methyl isobutyl ketone, the stirring is preferably performed at a temperature of 25 °C.

In one embodiment, in the method III, the stirring is preferably performed at a temperature of 40 °C. The stirring is preferably performed for a period of 3 days. The crystalline form A of the hydrochloride salt of the compound of formula I or the crystalline form A of the sulfate salt of the compound of formula I and the 88% acetone are preferably in a mass-to-volume ratio of 150 mg:1 mL.

In one embodiment, in the method IV, the stirring is preferably performed at a temperature of 25 °C. The stirring is preferably performed for a period of 1 day. The crystalline form A of the sulfate salt of the compound of formula I and the methanol are preferably in a mass-to-volume ratio of 35.71 mg:1 mL.

In one embodiment, in the method V, the volatilizing and drying are preferably performed at a temperature of 25 °C. The methanol and the organic solvent are preferably in a volume ratio of 2:1.

The present invention provides a crystalline form C of a compound of formula I, which has an X-ray powder diffraction (XRPD) pattern having at least three, at least four, at least five, at least six, at least seven, or at least eight characteristic peaks at the following 2Θ angles: 5.085° ± 0.2°, 9.623° ± 0.2°, 10.012° ± 0.2°, 10.95° ± 0.2°, 15.058° ± 0.2°, 18.077° ± 0.2°, 19.423° ± 0.2°, and 22.479° ± 0.2°;

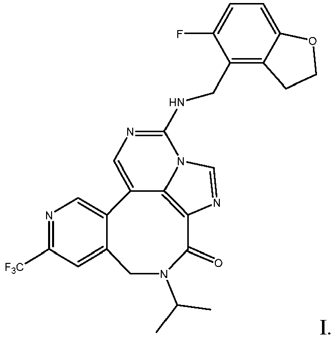

In one embodiment, the crystalline form C of the compound of formula I has an X-ray powder diffraction pattern having characteristic peaks at the following 2Θ angles: 5.085° ± 0.2°, 9.623° ± 0.2°, and 22.479° ± 0.2°.

In one embodiment, the crystalline form C of the compound of formula I has an X-ray powder diffraction pattern having characteristic peaks at the following 2Θ angles: 5.085° ± 0.2°, 9.623° ± 0.2°, 18.077° ± 0.2°, and 22.479° ± 0.2°.

In one embodiment, the crystalline form C of the compound of formula I has an X-ray powder diffraction pattern having characteristic peaks at the following 2Θ angles: 5.085° ± 0.2°, 9.623° ± 0.2°, 15.058° ± 0.2°, 18.077° ± 0.2°, and 22.479° ± 0.2°.

In one embodiment, the crystalline form C of the compound of formula I has an X-ray powder diffraction pattern having characteristic peaks at the following 2Θ angles: 5.085° ± 0.2°, 9.623° ± 0.2°, 10.95° ± 0.2°, 15.058° ± 0.2°, 18.077° ± 0.2°, and 22.479° ± 0.2°.

In one embodiment, the crystalline form C of the compound of formula I has an X-ray powder diffraction pattern having characteristic peaks at the following 2Θ angles: 5.085° ± 0.2°, 9.623° ± 0.2°, 10.012° ± 0.2°, 10.95° ± 0.2°, 15.058° ± 0.2°, 18.077° ± 0.2°, and 22.479° ± 0.2°.

In one embodiment, the crystalline form C of the compound of formula I has an X-ray powder diffraction pattern having characteristic peaks at the following 2Θ angles: 5.085° ± 0.2°, 9.623° ± 0.2°, 10.012° ± 0.2°, 10.95° ± 0.2°, 15.058° ± 0.2°, 18.077° ± 0.2°, 19.423° ± 0.2°, and 22.479° ± 0.2°.

In one embodiment, the crystalline form C of the compound of formula I has an X-ray powder diffraction pattern having characteristic peaks at the following 2Θ angles: 5.085° ± 0.2°, 9.623° ± 0.2°, 10.012° ± 0.2°, 10.637° ± 0.2°, 10.95° ± 0.2°, 13.558° ± 0.2°, 14.065° ± 0.2°, 15.058° ± 0.2°, 17.435° ± 0.2°, 18.077° ± 0.2°, 19.129° ± 0.2°, 19.423° ± 0.2°, 19.947° ± 0.2°, 20.709° ± 0.2°, 22.147° ± 0.2°, 22.479° ± 0.2°, 23.435° ± 0.2°, 24.781° ± 0.2°, 26.883° ± 0.2°, 29.492° ± 0.2°, and 31.713° ± 0.2°.

In one embodiment, the crystalline form C of the compound of formula I has an X-ray powder diffraction pattern having characteristic peaks at the following 2Θ angles: 5.085° ± 0.2°, 9.623° ± 0.2°, 10.012° ± 0.2°, 10.637° ± 0.2°, 10.95° ± 0.2°, 11.707° ± 0.2°, 13.558° ± 0.2°, 14.065° ± 0.2°, 15.058° ± 0.2°, 17.435° ± 0.2°, 18.077° ± 0.2°, 19.129° ± 0.2°, 19.423° ± 0.2°, 19.947° ± 0.2°, 20.709° ± 0.2°, 22.147° ± 0.2°, 22.479° ± 0.2°, 23.435° ± 0.2°, 24.781° ± 0.2°, 25.439° ± 0.2°, 26.548° ± 0.2°, 26.883° ± 0.2°, 28.552° ± 0.2°, 28.853° ± 0.2°, 29.492° ± 0.2°, 31.713° ± 0.2°, 32.514° ± 0.2°, and 33.392° ± 0.2°.

In one embodiment, the crystalline form C of the compound of formula I has X-ray powder diffraction pattern analysis data substantially as shown in Table 3:

**Table 3. XRPD diffraction data for the crystalline form C of the compound of formula 1**

| Position [°2θ] ± 0.2° | d-spacing [Å] | Relative intensity [%] |
|---|---|---|
| 5.085 | 17.3658 | 88.7 |
| 9.623 | 9.1834 | 100 |
| 10.012 | 8.8274 | 48.5 |
| 10.637 | 8.3103 | 30.3 |
| 10.95 | 8.0734 | 48.7 |
| 11.707 | 7.553 | 15.8 |
| 13.558 | 6.5254 | 22.9 |
| 14.065 | 6.2914 | 20.3 |
| 15.058 | 5.8787 | 49.9 |
| 17.435 | 5.0823 | 43.6 |
| 18.077 | 4.9031 | 52.1 |
| 19.129 | 4.6359 | 36.7 |
| 19.423 | 4.5663 | 48.4 |
| 19.947 | 4.4476 | 43.3 |
| 20.709 | 4.2855 | 27.3 |
| 22.147 | 4.0104 | 35.8 |
| 22.479 | 3.952 | 55.6 |
| 23.435 | 3.7928 | 41.8 |
| 24.781 | 3.5898 | 27.2 |
| 25.439 | 3.4984 | 15.2 |
| 26.548 | 3.3547 | 18.6 |
| 26.883 | 3.3137 | 29.8 |
| 28.552 | 3.1237 | 16.2 |
| 28.853 | 3.0918 | 15.6 |
| 29.492 | 3.0262 | 24 |
| 31.713 | 2.8192 | 21.3 |
| 32.514 | 2.7515 | 14.6 |
| 33.392 | 2.6812 | 12.1 |

In one embodiment, the crystalline form C of the compound of formula I has an X-ray powder diffraction pattern substantially as shown in FIG. 12.

The present invention further provides a preparation method for the crystalline form C of the compound of formula I, which preferably comprises the following steps: stirring a crystalline form A of a compound of formula I in tetrahydrofuran to precipitate a solid, and separating and drying the solid to obtain the crystalline form C of the compound of formula I, wherein the stirring is performed at a temperature of 20-30 °C; the stirring is preferably performed for a period of 0.5-1.5 days; the crystalline form A of the compound of formula I and the tetrahydrofuran are preferably in a mass-to-volume ratio of 20 mg:1 mL-50 mg:1 mL.

In one embodiment, in the preparation method for the crystalline form C of the compound of formula I, the stirring is preferably performed at a temperature of 25 °C. The stirring is preferably performed for a period of 1 day. The crystalline form A of the compound of formula I and the tetrahydrofuran are preferably in a mass-to-volume ratio of 33.33 mg:1 mL.

The present invention provides a crystalline form of a toluene solvate of a compound of formula I, which has an X-ray powder diffraction (XRPD) pattern having at least three, at least four, or at least five characteristic peaks at the following 2Θ angles: 5.298° ± 0.2°, 14.611° ± 0.2°, 18.098° ± 0.2°, 20.024° ± 0.2°, and 20.9° ± 0.2°;

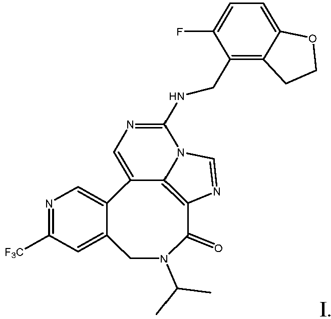

In one embodiment, the crystalline form of the toluene solvate of the compound of formula I has an X-ray powder diffraction pattern having characteristic peaks at the following 2Θ angles: 5.298° ± 0.2°, 14.611° ± 0.2°, and 20.9° ± 0.2°.

In one embodiment, the crystalline form of the toluene solvate of the compound of formula I has an X-ray powder diffraction pattern having characteristic peaks at the following 2Θ angles: 5.298° ± 0.2°, 14.611° ± 0.2°, 18.098° ± 0.2°, and 20.9° ± 0.2°.

In one embodiment, the crystalline form of the toluene solvate of the compound of formula I has an X-ray powder diffraction pattern having characteristic peaks at the following 2Θ angles: 5.298° ± 0.2°, 14.611° ± 0.2°, 18.098° ± 0.2°, 20.024° ± 0.2°, and 20.9° ± 0.2°.

In one embodiment, the crystalline form of the toluene solvate of the compound of formula I has an X-ray powder diffraction pattern having characteristic peaks at the following 2Θ angles: 5.298° ± 0.2°, 10.439° ± 0.2°, 11.496° ± 0.2°, 13.324° ± 0.2°, 14.611° ± 0.2°, 18.098° ± 0.2°, 20.024° ± 0.2°, 20.9° ± 0.2°, 21.837° ± 0.2°, 22.303° ± 0.2°, 23.122° ± 0.2°, 24.506° ± 0.2°, 25.886° ± 0.2°, and 27.506° ± 0.2°.

In one embodiment, the crystalline form of the toluene solvate of the compound of formula I has X-ray powder diffraction pattern analysis data substantially as shown in Table 4: 5.298° ± 0.2°, 5.745° ± 0.2°, 9.662° ± 0.2°, 10.439° ± 0.2°, 11.496° ± 0.2°, 13.324° ± 0.2°, 14.101° ± 0.2°, 14.611° ± 0.2°, 14.98° ± 0.2°, 15.64° ± 0.2°, 16.769° ± 0.2°, 17.628° ± 0.2°, 18.098° ± 0.2°, 19.258° ± 0.2°, 20.024° ± 0.2°, 20.9° ± 0.2°, 21.232° ± 0.2°, 21.837° ± 0.2°, 22.303° ± 0.2°, 22.698° ± 0.2°, 23.122° ± 0.2°, 24.506° ± 0.2°, 25.128° ± 0.2°, 25.886° ± 0.2°, 26.977° ± 0.2°, 27.506° ± 0.2°, 28.28° ± 0.2°, 29.357° ± 0.2°, 30.232° ± 0.2°, 31.28° ± 0.2°, 31.492° ± 0.2°, 33.412° ± 0.2°, 33.764° ± 0.2°, 35.277° ± 0.2°, and 35.683° ± 0.2°.

**Table 4. X-ray powder diffraction pattern analytical data for the crystalline form of the toluene solvate of the compound of formula I**

| Position [°2θ] ± 0.2° | d-spacing [Å] | Relative intensity [%] |
|---|---|---|
| 5.298 | 16.6667 | 67.5 |
| 5.745 | 15.3703 | 25.7 |
| 9.662 | 9.1463 | 21.2 |
| 10.439 | 8.4671 | 36.8 |
| 11.496 | 7.6911 | 36.5 |
| 13.324 | 6.6398 | 34.4 |
| 14.101 | 6.2755 | 16.9 |
| 14.611 | 6.0574 | 100 |
| 14.98 | 5.9093 | 27.7 |
| 15.64 | 5.6611 | 22.1 |
| 16.769 | 5.2827 | 20.9 |
| 17.628 | 5.0271 | 24.8 |
| 18.098 | 4.8976 | 59.7 |
| 19.258 | 4.605 | 16.9 |
| 20.024 | 4.4306 | 50.6 |
| 20.9 | 4.2469 | 60.2 |
| 21.232 | 4.1812 | 26.2 |
| 21.837 | 4.0666 | 48.8 |
| 22.303 | 3.9828 | 32.9 |
| 22.698 | 3.9144 | 25 |
| 23.122 | 3.8435 | 40.9 |
| 24.506 | 3.6295 | 31.7 |
| 25.128 | 3.541 | 18.8 |
| 25.886 | 3.439 | 31.5 |
| 26.977 | 3.3024 | 18.4 |
| 27.506 | 3.24 | 36.7 |
| 28.28 | 3.1531 | 16.2 |
| 29.357 | 3.0399 | 13.1 |
| 30.232 | 2.9538 | 13 |
| 31.28 | 2.8572 | 13.6 |
| 31.492 | 2.8385 | 13.1 |
| 33.412 | 2.6796 | 12.1 |
| 33.764 | 2.6525 | 15.2 |
| 35.277 | 2.5421 | 12.2 |
| 35.683 | 2.5141 | 12.9 |

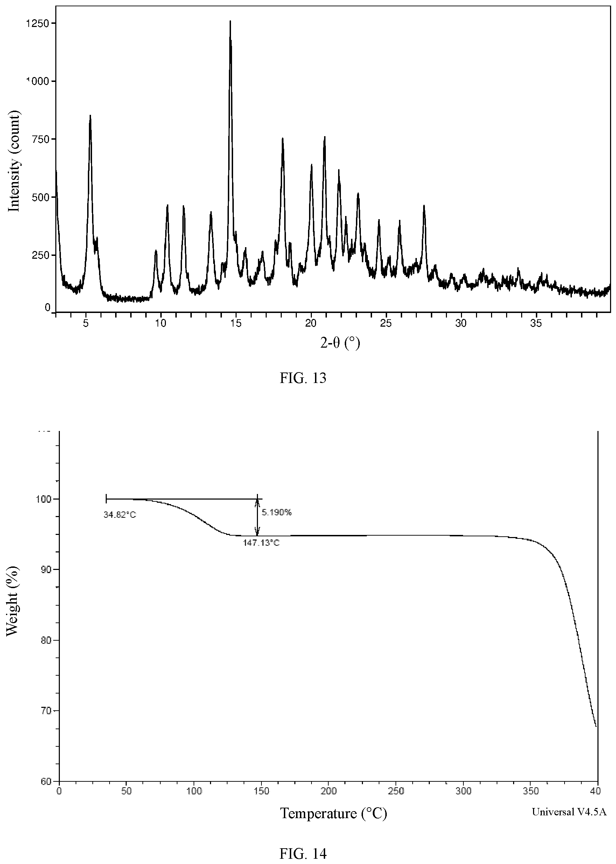

In one embodiment, the crystalline form of the toluene solvate of the compound of formula I has an X-ray powder diffraction pattern substantially as shown in FIG. 13.

In one embodiment, the crystalline form of the toluene solvate of the compound of formula I has a thermogravimetric analysis (TGA) curve showing a weight loss of 5.19% ± 0.5% during heating from 34.82 °C ± 3 °C to 147.13 °C ± 3 °C.

In one embodiment, the crystalline form of the toluene solvate of the compound of formula I has a thermogravimetric analysis curve showing a weight loss of 5.19% during heating from 34.82 °C to 147.13 °C. In one embodiment, the crystalline form of the toluene solvate of the compound of formula I has a thermogravimetric analysis curve substantially as shown in FIG. 14.

In one embodiment, the crystalline form of the toluene solvate of the compound of formula I has a differential scanning calorimetry (DSC) curve having endothermic peaks with initial temperatures of 120.68 °C ± 3 °C and 334.15 °C ± 3 °C, respectively.

In one embodiment, the crystalline form of the toluene solvate of the compound of formula I has a differential scanning calorimetry curve having endothermic peaks with initial temperatures of 120.68 °C and 334.15 °C, respectively.

In one embodiment, the crystalline form of the toluene solvate of the compound of formula I has a differential scanning calorimetry curve having endothermic peaks with peak temperatures of 151.28 °C ± 3 °C and 336.25 °C ± 3 °C, respectively.

In one embodiment, the crystalline form of the toluene solvate of the compound of formula I has a differential scanning calorimetry curve having endothermic peaks with peak temperatures of 151.28 °C and 336.25 °C, respectively.

In one embodiment, the crystalline form of the toluene solvate of the compound of formula I has a differential scanning calorimetry curve having an exothermic peak with a peak temperature of 238.88 °C ± 3 °C.

In one embodiment, the crystalline form of the toluene solvate of the compound of formula I has a differential scanning calorimetry curve having an exothermic peak with a peak temperature of 238.88 °C.

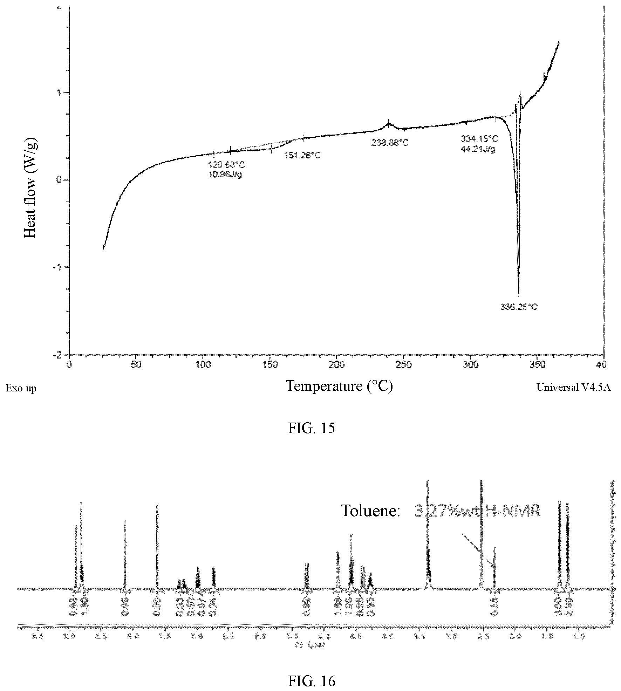

In one embodiment, the crystalline form of the toluene solvate of the compound of formula I has a differential scanning calorimetry curve substantially as shown in FIG. 15.

In one embodiment, the crystalline form of the toluene solvate of the compound of formula I has a nuclear magnetic resonance hydrogen spectrum (¹H-NMR) showing the presence of toluene with a weight percentage of 3.27% ± 0.5%.

In one embodiment, the crystalline form of the toluene solvate of the compound of formula I has a nuclear magnetic resonance hydrogen spectrum showing the presence of toluene with a weight percentage of 3.27%. In one embodiment, the crystalline form of the toluene solvate of the compound of formula I has a nuclear magnetic resonance hydrogen spectrum substantially as shown in FIG. 16.

The present invention further provides a preparation method for the crystalline form of the toluene solvate of the compound of formula I, which preferably comprises the following steps: stirring a crystalline form B of a compound of formula I in toluene to precipitate a solid, and separating and drying the solid to obtain the crystalline form of the toluene solvate of the compound of formula I, wherein the stirring is performed at a temperature of 20-30 °C; the stirring is preferably performed for a period of 1.5-2.5 days; the crystalline form B of the compound of formula I and the toluene are preferably in a mass-to-volume ratio of (250 mg-350 mg):1 mL.

In one embodiment, in the preparation method for the crystalline form of the toluene solvate of the compound of formula I, the stirring is preferably performed at a temperature of 25 °C. The stirring is preferably performed for a period of 2 days. The crystalline form B of the compound of formula I and the toluene are preferably in a mass-to-volume ratio of 300 mg:1 mL.

The present invention provides a crystalline form E of a compound of formula I, which has an X-ray powder diffraction (XRPD) pattern having at least three, at least four, at least five, at least six, or at least seven characteristic peaks at the following 2Θ angles: 6.001° ± 0.2°, 10.676° ± 0.2°, 11.203° ± 0.2°, 12.526° ± 0.2°, 13.09° ± 0.2°, 16.891° ± 0.2°, and 21.179° ± 0.2°;

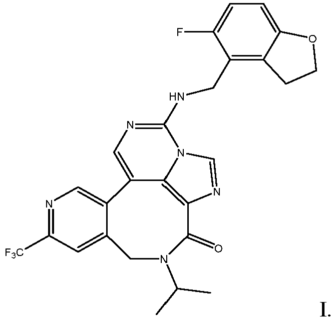

In one embodiment, the crystalline form E of the compound of formula I has an X-ray powder diffraction pattern having characteristic peaks at the following 2Θ angles: 6.001° ± 0.2°, 10.676° ± 0.2°, and 11.203° ± 0.2°.

In one embodiment, the crystalline form E of the compound of formula I has an X-ray powder diffraction pattern having characteristic peaks at the following 2Θ angles: 6.001° ± 0.2°, 10.676° ± 0.2°, 11.203° ± 0.2°, and 12.526° ± 0.2°.

In one embodiment, the crystalline form E of the compound of formula I has an X-ray powder diffraction pattern having characteristic peaks at the following 2Θ angles: 6.001° ± 0.2°, 10.676° ± 0.2°, 11.203° ± 0.2°, 12.526° ± 0.2°, and 16.891° ± 0.2°.

In one embodiment, the crystalline form E of the compound of formula I has an X-ray powder diffraction pattern having characteristic peaks at the following 2Θ angles: 6.001° ± 0.2°, 10.676° ± 0.2°, 11.203° ± 0.2°, 12.526° ± 0.2°, 16.891° ± 0.2°, and 21.179° ± 0.2°.

In one embodiment, the crystalline form E of the compound of formula I has an X-ray powder diffraction pattern having characteristic peaks at the following 2Θ angles: 6.001° ± 0.2°, 10.676° ± 0.2°, 11.203° ± 0.2°, 12.526° ± 0.2°, 13.09° ± 0.2°, 16.891° ± 0.2°, and 21.179° ± 0.2°.

In one embodiment, the crystalline form E of the compound of formula I has an X-ray powder diffraction pattern having characteristic peaks at the following 2Θ angles: 6.001° ± 0.2°, 6.484° ± 0.2°, 8.32° ± 0.2°, 10.676° ± 0.2°, 11.203° ± 0.2°, 12.526° ± 0.2°, 13.09° ± 0.2°, 13.771° ± 0.2°, 14.61° ± 0.2°, 14.982° ± 0.2°, 15.429° ± 0.2°, 16.423° ± 0.2°, 16.891° ± 0.2°, 18.034° ± 0.2°, 18.757° ± 0.2°, 20.239° ± 0.2°, 20.864° ± 0.2°, 21.179° ± 0.2°, 22.618° ± 0.2°, 23.802° ± 0.2°, and 26.217° ± 0.2°.

In one embodiment, the crystalline form E of the compound of formula I has an X-ray powder diffraction pattern having characteristic peaks at the following 2Θ angles: 6.001° ± 0.2°, 6.484° ± 0.2°, 8.32° ± 0.2°, 10.676° ± 0.2°, 11.203° ± 0.2°, 12.526° ± 0.2°, 13.09° ± 0.2°, 13.771° ± 0.2°, 14.61° ± 0.2°, 14.982° ± 0.2°, 15.429° ± 0.2°, 16.423° ± 0.2°, 16.891° ± 0.2°, 18.034° ± 0.2°, 18.757° ± 0.2°, 20.239° ± 0.2°, 20.864° ± 0.2°, 21.179° ± 0.2°, 22.618° ± 0.2°, 23.802° ± 0.2°, 25.594° ± 0.2°, 26.217° ± 0.2°, 27.198° ± 0.2°, 28.009° ± 0.2°, and 30.812° ± 0.2°.

In one embodiment, the crystalline form E of the compound of formula I has X-ray powder diffraction pattern analysis data substantially as shown in Table 5:

**Table 5. X-ray powder diffraction pattern analysis data for the crystalline form E of the compound of formula I**

| Position [°2θ] ± 0.2° | d-spacing [Å] | Relative intensity [%] |
|---|---|---|
| 6.001 | 14.7151 | 100 |
| 6.484 | 13.6199 | 28.5 |
| 8.32 | 10.6185 | 24.1 |
| 10.676 | 8.28 | 52.8 |
| 11.203 | 7.8917 | 60.2 |
| 12.526 | 7.061 | 41.3 |
| 13.09 | 6.7579 | 31 |
| 13.771 | 6.4252 | 21.9 |
| 14.61 | 6.0579 | 22.2 |
| 14.982 | 5.9085 | 21.3 |
| 15.429 | 5.7384 | 24 |
| 16.423 | 5.393 | 25.2 |
| 16.891 | 5.2446 | 38.2 |
| 18.034 | 4.9148 | 21.3 |
| 18.757 | 4.7268 | 23.6 |
| 20.239 | 4.3839 | 28.5 |
| 20.864 | 4.254 | 22.4 |
| 21.179 | 4.1916 | 38.1 |
| 22.618 | 3.928 | 22.4 |
| 23.802 | 3.7353 | 21.4 |
| 25.594 | 3.4776 | 19.7 |
| 26.217 | 3.3964 | 26.9 |
| 27.198 | 3.2761 | 19.7 |
| 28.009 | 3.183 | 15.9 |
| 30.812 | 2.8995 | 14.2 |

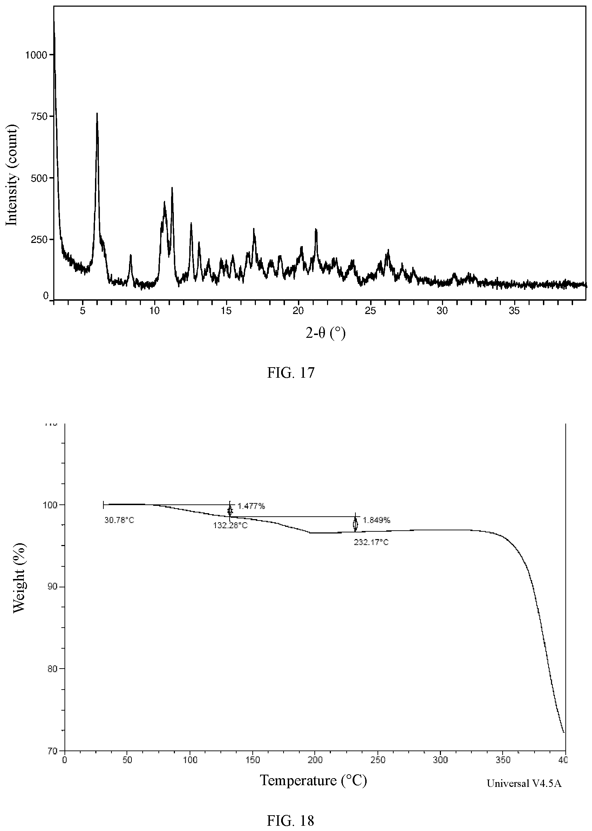

In one embodiment, the crystalline form E of the compound of formula I has an X-ray powder diffraction pattern substantially as shown in FIG. 17.

In one embodiment, the crystalline form E of the compound of formula I has a thermogravimetric analysis (TGA) curve showing a weight loss of 1.477% ± 0.5% during heating from 30.78 °C ± 3 °C to 132.28 °C ± 3 °C; and a weight loss of 1.849% ± 0.5% during heating from 132 °C ± 3 °C from 232 °C ± 3 °C.

In one embodiment, the crystalline form E of the compound of formula I has a thermogravimetric analysis curve showing a weight loss of 1.477% during heating from 30.78 °C to 132.28 °C; and a weight loss of 1.849% during heating from 132.28 °C to 232.17 °C.

In one embodiment, the crystalline form E of the compound of formula I has a thermogravimetric analysis curve substantially as shown in FIG. 18.

In one embodiment, the crystalline form E of the compound of formula I has a differential scanning calorimetry (DSC) curve having endothermic peaks with initial temperatures of 190.41 °C ± 3 °C and 334.9 °C ± 3 °C, respectively.

In one embodiment, the crystalline form E of the compound of formula I has a differential scanning calorimetry curve having endothermic peaks with initial temperatures of 190.41 °C and 334.9 °C, respectively.

In one embodiment, the crystalline form E of the compound of formula I has a differential scanning calorimetry curve having endothermic peaks with peak temperatures of 199.65 °C ± 3 °C and 336.81 °C ± 3 °C, respectively.

In one embodiment, the crystalline form E of the compound of formula I has a differential scanning calorimetry curve having endothermic peaks with peak temperatures of 199.65 °C and 336.81 °C, respectively.

In one embodiment, the crystalline form E of the compound of formula I has a differential scanning calorimetry curve having an exothermic peak with a peak temperature of 205.95 °C ± 3 °C.

In one embodiment, the crystalline form F of the compound of formula I has a differential scanning calorimetry curve having an exothermic peak with a peak temperature of 205.95 °C.

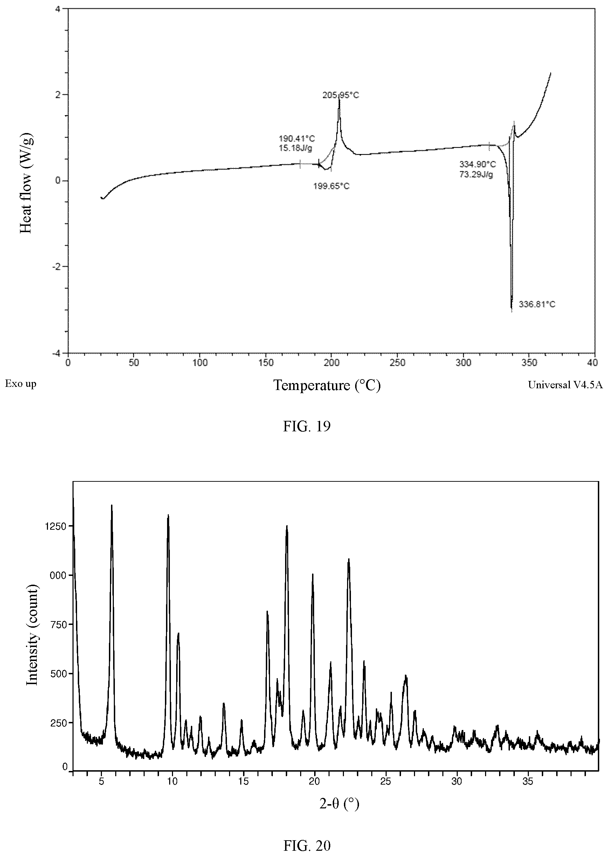

In one embodiment, the crystalline form E of the compound of formula I has a differential scanning calorimetry curve substantially as shown in FIG. 19.

The present invention further provides a preparation method for the crystalline form E of the compound of formula I, preferably any one of the following methods:
method I comprising the following steps: stirring a crystalline form B of a compound of formula I in methyl *tert*-butyl ether to precipitate a solid, and separating and drying the solid to obtain the crystalline form E of the compound of formula I, wherein the stirring is performed at a temperature of 20-30 °C; the stirring is preferably performed for a period of 1.5-2.5 days; and the crystalline form B of the compound of formula I and the methyl *tert*-butyl ether are preferably in a mass-to-volume ratio of (50 mg-350 mg):1 mL; and
method II comprising the following steps: stirring an amorphous form of a compound of formula I in an organic solvent to precipitate a solid, and separating and drying the solid to obtain the crystalline form E of the compound of formula I, wherein the organic solvent is selected from one or two of 2-methyltetrahydrofuran and methyl *tert*-butyl ether; the stirring is performed at a temperature of 20-30 °C; the stirring is preferably performed for a period of 1.5-2.5 days; and the amorphous form of the compound of formula I and the solvent are preferably in a mass-to-volume ratio of (250 mg-350 mg):1 mL.

In one embodiment, in the method I, the stirring is preferably performed at a temperature of 25 °C. The stirring is preferably performed for a period of 2 days. The crystalline form B of the compound of formula I and the methyl *tert*-butyl ether are preferably in a mass-to-volume ratio of 100 mg:1 mL or 300 mg:1 mL.

In one embodiment, in the method II, the stirring is preferably performed at a temperature of 25 °C. The stirring is preferably performed for a period of 2 days. The amorphous form of the compound of formula I and the solvent are preferably in a mass-to-volume ratio of 300 mg: 1 mL.

The present invention provides a crystalline form F of a compound of formula I, which has an X-ray powder diffraction (XRPD) pattern having at least three, at least four, at least five, or at least six characteristic peaks at the following 2Θ angles: 5.728° ± 0.2°, 9.701° ± 0.2°, 16.658° ± 0.2°, 18.038° ± 0.2°, 19.851° ± 0.2°, and 22.382° ± 0.2°;

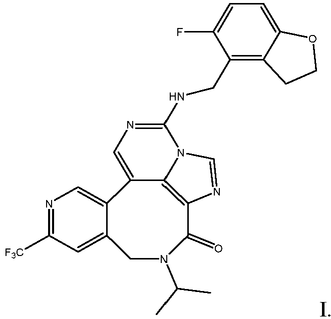

In one embodiment, the crystalline form F of the compound of formula I has an X-ray powder diffraction pattern having characteristic peaks at the following 2Θ angles: 5.728° ± 0.2°, 9.701° ± 0.2°, and 18.038° ± 0.2°.

In one embodiment, the crystalline form F of the compound of formula I has an X-ray powder diffraction pattern having characteristic peaks at the following 2Θ angles: 5.728° ± 0.2°, 9.701° ± 0.2°, 18.038° ± 0.2°, and 22.382° ± 0.2°.

In one embodiment, the crystalline form F of the compound of formula I has an X-ray powder diffraction pattern having characteristic peaks at the following 2Θ angles: 5.728° ± 0.2°, 9.701° ± 0.2°, 18.038° ± 0.2°, 19.851° ± 0.2°, and 22.382° ± 0.2°.

In one embodiment, the crystalline form F of the compound of formula I has an X-ray powder diffraction pattern having characteristic peaks at the following 2Θ angles: 5.728° ± 0.2°, 9.701° ± 0.2°, 16.658° ± 0.2°, 18.038° ± 0.2°, 19.851° ± 0.2°, and 22.382° ± 0.2°.

In one embodiment, the crystalline form F of the compound of formula I has an X-ray powder diffraction pattern having characteristic peaks at the following 2Θ angles: 5.728° ± 0.2°, 9.701° ± 0.2°, 10.421° ± 0.2°, 11.944° ± 0.2°, 13.616° ± 0.2°, 16.658° ± 0.2°, 17.358° ± 0.2°, 18.038° ± 0.2°, 19.17° ± 0.2°, 19.851° ± 0.2°, 21.116° ± 0.2°, 21.815° ± 0.2°, 22.382° ± 0.2°, 23.066° ± 0.2°, 23.454° ± 0.2°, 24.334° ± 0.2°, 24.622° ± 0.2°, 25.362° ± 0.2°, 26.376° ± 0.2°, and 27.038° ± 0.2°.

In one embodiment, the crystalline form F of the compound of formula I has an X-ray powder diffraction pattern having characteristic peaks at the following 2Θ angles: 5.728° ± 0.2°, 8.026° ± 0.2°, 9.701° ± 0.2°, 10.421° ± 0.2°, 10.946° ± 0.2°,11.333° ± 0.2°, 11.944° ± 0.2°, 12.546° ± 0.2°, 13.616° ± 0.2°, 14.847° ± 0.2°, 15.761° ± 0.2°, 16.658° ± 0.2°, 17.358° ± 0.2°, 18.038° ± 0.2°, 19.17° ± 0.2°, 19.851° ± 0.2°, 21.116° ± 0.2°, 21.815° ± 0.2°, 22.382° ± 0.2°, 23.066° ± 0.2°, 23.454° ± 0.2°, 24.334° ± 0.2°, 24.622° ± 0.2°, 25.073° ± 0.2°, 25.362° ± 0.2°, 26.376° ± 0.2°, 27.038° ± 0.2°, 27.683° ± 0.2°, 28.279° ± 0.2°, 29.787° ± 0.2°, 30.379° ± 0.2°, 31.242° ± 0.2°, 31.967° ± 0.2°, 32.593° ± 0.2°, 32.844° ± 0.2°, 33.425° ± 0.2°, 35.629° ± 0.2°, 35.996° ± 0.2°, 37.988° ± 0.2°, and 38.737° ± 0.2°.

In one embodiment, the crystalline form F of the compound of formula I has X-ray powder diffraction pattern analysis data substantially as shown in Table 6:

**Table 6. X-ray powder diffraction pattern analysis data for the crystalline form F of the compound of formula I**

| Position [°2θ] ± 0.2° | d-spacing [Å] | Relative intensity [%] |
|---|---|---|
| 5.728 | 15.4169 | 100 |
| 8.026 | 11.0064 | 8.2 |
| 9.701 | 9.1101 | 96.4 |
| 10.421 | 8.4819 | 51.8 |
| 10.946 | 8.0763 | 19.1 |
| 11.333 | 7.8014 | 16.6 |
| 11.944 | 7.4034 | 20.5 |
| 12.546 | 7.0496 | 12.7 |
| 13.616 | 6.4981 | 25.5 |
| 14.847 | 5.962 | 19.2 |
| 15.761 | 5.6179 | 11.4 |
| 16.658 | 5.3176 | 60 |
| 17.358 | 5.1046 | 34.6 |
| 18.038 | 4.9136 | 92.3 |
| 19.17 | 4.626 | 22.7 |
| 19.851 | 4.4689 | 74 |
| 21.116 | 4.204 | 40.9 |
| 21.815 | 4.0708 | 24.3 |
| 22.382 | 3.9688 | 79.8 |
| 23.066 | 3.8526 | 20.5 |
| 23.454 | 3.7899 | 41.4 |
| 24.334 | 3.6547 | 23.3 |
| 24.622 | 3.6126 | 21.7 |
| 25.073 | 3.5486 | 17.1 |
| 25.362 | 3.5088 | 29.5 |
| 26.376 | 3.3762 | 36 |
| 27.038 | 3.2951 | 22.7 |
| 27.683 | 3.2197 | 14.8 |
| 28.279 | 3.1532 | 13.1 |
| 29.787 | 2.997 | 16.8 |
| 30.379 | 2.9398 | 15.2 |
| 31.242 | 2.8606 | 15.2 |
| 31.967 | 2.7973 | 11.7 |
| 32.593 | 2.745 | 13.9 |
| 32.844 | 2.7246 | 17.1 |
| 33.425 | 2.6786 | 15.2 |
| 35.629 | 2.5178 | 15.1 |
| 35.996 | 2.4929 | 12.1 |
| 37.988 | 2.3667 | 11 |
| 38.737 | 2.3226 | 13 |

In one embodiment, the crystalline form F of the compound of formula I has an X-ray powder diffraction pattern substantially as shown in FIG. 20.

In one embodiment, the crystalline form F of the compound of formula I has a thermogravimetric analysis (TGA) curve showing a weight loss of 0.7264% ± 0.2% during heating from 37.55 °C ± 3 °C to 150.41 °C ± 3 °C.

In one embodiment, the crystalline form F of the compound of formula I has a thermogravimetric analysis curve showing a weight loss of 0.7264% during heating from 37.55 °C to 150.41 °C.

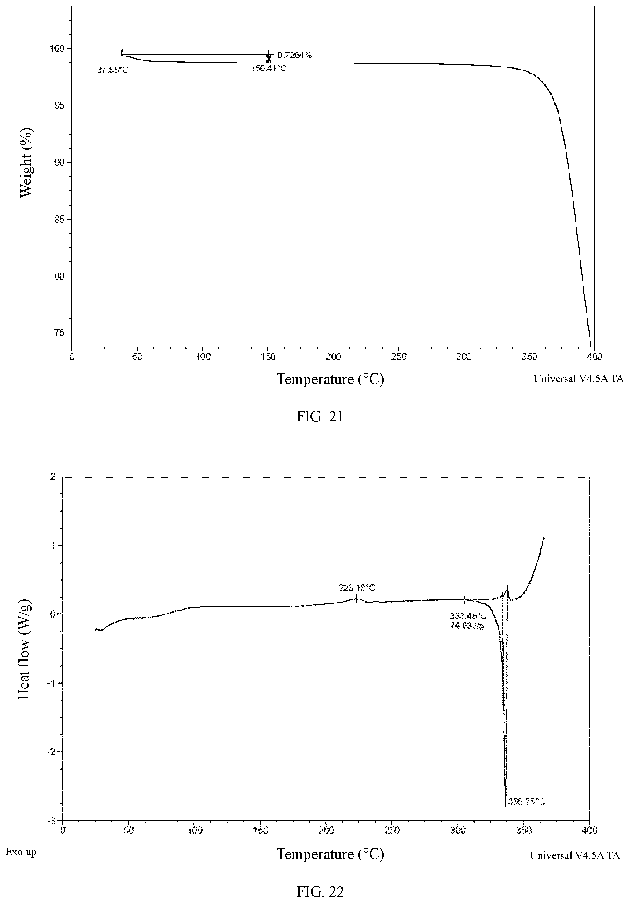

In one embodiment, the crystalline form F of the compound of formula I has a thermogravimetric analysis curve substantially as shown in FIG. 21.

In one embodiment, the crystalline form F of the compound of formula I has a differential scanning calorimetry (DSC) curve having an endothermic peak with an initial temperature of 333.46 °C ± 3 °C.

In one embodiment, the crystalline form F of the compound of formula I has a differential scanning calorimetry curve having an endothermic peak with an initial temperature of 333.46 °C.

In one embodiment, the crystalline form F of the compound of formula I has a differential scanning calorimetry curve having an endothermic peak with a peak temperature of 336.25 °C ± 3 °C.

In one embodiment, the crystalline form F of the compound of formula I has a differential scanning calorimetry curve having an endothermic peak with a peak temperature of 336.25 °C.

In one embodiment, the crystalline form F of the compound of formula I has a differential scanning calorimetry curve having an exothermic peak with a peak temperature of 223.19 °C ± 3 °C.

In one embodiment, the crystalline form F of the compound of formula I has a differential scanning calorimetry curve having an exothermic peak with a peak temperature of 223.19 °C.

In one embodiment, the crystalline form F of the compound of formula I has a differential scanning calorimetry curve substantially as shown in FIG. 22.

The present invention further provides a preparation method for the crystalline form F of the compound of formula I, which preferably comprises the following steps: stirring a crystalline form B of a compound of formula I in a mixed solvent of methanol and water in a volume ratio of (0.5-1.5):1 to precipitate a solid, and separating and drying the solid to obtain the crystalline form F of the compound of formula I, wherein the stirring is performed at a temperature of 20-30 °C; the stirring is preferably performed for a period of 1.5-2.5 days; and the crystalline form B of the compound of formula I and the mixed solvent are preferably in a mass-to-volume ratio of (50 mg-350 mg):1 mL.

In one embodiment, in the preparation method for the crystalline form F of the compound of formula I, the methanol and the water are preferably in a volume ratio of 1:1. The stirring is preferably performed at a temperature of 25 °C. The stirring is preferably performed for a period of 2 days. The crystalline form B of the compound of formula I and the methanol are preferably in a mass-to-volume ratio of 100 mg:1 mL or 300 mg:1 mL.

The present invention provides a crystalline form G of a compound of formula I, which has an X-ray powder diffraction (XRPD) pattern having at least three, at least four, at least five, at least six, at least seven, or at least eight characteristic peaks at the following 2Θ angles: 5.279° ± 0.2°, 10.926° ± 0.2°, 11.843° ± 0.2°, 16.187° ± 0.2°, 16.831° ± 0.2°, 20.453° ± 0.2°, 23.55° ± 0.2°, and 26.825° ± 0.2°;

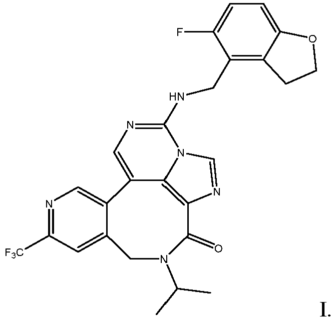

In one embodiment, the crystalline form G of the compound of formula I has an X-ray powder diffraction pattern having characteristic peaks at the following 2Θ angles: 5.279° ± 0.2°, 20.453° ± 0.2°, and 23.55° ± 0.2°.

In one embodiment, the crystalline form G of the compound of formula I has an X-ray powder diffraction pattern having characteristic peaks at the following 2Θ angles: 5.279° ± 0.2°, 11.843° ± 0.2°, 20.453° ± 0.2°, and 23.55° ± 0.2°.

In one embodiment, the crystalline form G of the compound of formula I has an X-ray powder diffraction pattern having characteristic peaks at the following 2Θ angles: 5.279° ± 0.2°, 11.843° ± 0.2°, 16.831° ± 0.2°, 20.453° ± 0.2°, and 23.55° ± 0.2°.

In one embodiment, the crystalline form G of the compound of formula I has an X-ray powder diffraction pattern having characteristic peaks at the following 2Θ angles: 5.279° ± 0.2°, 11.843° ± 0.2°, 16.187° ± 0.2°, 16.831° ± 0.2°, 20.453° ± 0.2°, and 23.55° ± 0.2°.

In one embodiment, the crystalline form G of the compound of formula I has an X-ray powder diffraction pattern having characteristic peaks at the following 2Θ angles: 5.279° ± 0.2°, 10.926° ± 0.2°, 11.843° ± 0.2°, 16.187° ± 0.2°, 16.831° ± 0.2°, 20.453° ± 0.2°, and 23.55° ± 0.2°.

In one embodiment, the crystalline form G of the compound of formula I has an X-ray powder diffraction pattern having characteristic peaks at the following 2Θ angles: 5.279° ± 0.2°, 10.926° ± 0.2°, 11.843° ± 0.2°, 16.187° ± 0.2°, 16.831° ± 0.2°, 20.453° ± 0.2°, 23.55° ± 0.2°, and 26.825° ± 0.2°.

In one embodiment, the crystalline form G of the compound of formula I has an X-ray powder diffraction pattern having characteristic peaks at the following 2Θ angles: 5.279° ± 0.2°, 6.388° ± 0.2°, 9.02° ± 0.2°, 10.926° ± 0.2°, 11.843° ± 0.2°, 12.68° ± 0.2°, 14.96° ± 0.2°, 15.758° ± 0.2°, 16.187° ± 0.2°, 16.831° ± 0.2°, 17.98° ± 0.2°, 18.352° ± 0.2°, 20.453° ± 0.2°, 20.881° ± 0.2°, 21.194° ± 0.2°, 22.732° ± 0.2°, 23.55° ± 0.2°, 25.208° ± 0.2°, 25.949° ± 0.2°, and 26.825° ± 0.2°.

In one embodiment, the crystalline form G of the compound of formula I has an X-ray powder diffraction pattern having characteristic peaks at the following 2Θ angles: 5.279° ± 0.2°, 6.388° ± 0.2°, 9.02° ± 0.2°, 10.926° ± 0.2°, 11.843° ± 0.2°, 12.68° ± 0.2°, 13.44° ± 0.2°, 14.162° ± 0.2°, 14.96° ± 0.2°, 15.758° ± 0.2°, 16.187° ± 0.2°, 16.831° ± 0.2°, 17.98° ± 0.2°, 18.352° ± 0.2°, 19.423° ± 0.2°, 20.453° ± 0.2°, 20.881° ± 0.2°, 21.194° ± 0.2°, 21.755° ± 0.2°, 22.732° ± 0.2°, 23.55° ± 0.2°, 24.217° ± 0.2°, 24.772° ± 0.2°, 25.208° ± 0.2°, 25.949° ± 0.2°, 26.825° ± 0.2°, 28.694° ± 0.2°, 29.143° ± 0.2°, 30.154° ± 0.2°, 30.774° ± 0.2°, 31.537° ± 0.2°, 32.318° ± 0.2°, and 33.834° ± 0.2°.

In one embodiment, the crystalline form G of the compound of formula I has an X-ray powder diffraction pattern having characteristic peaks at the following 2Θ angles: 5.279° ± 0.2°, 6.388° ± 0.2°, 7.147° ± 0.2°, 9.02° ± 0.2°, 10.926° ± 0.2°, 11.843° ± 0.2°, 12.68° ± 0.2°, 13.44° ± 0.2°, 14.162° ± 0.2°, 14.96° ± 0.2°, 15.758° ± 0.2°, 16.187° ± 0.2°, 16.831° ± 0.2°, 17.98° ± 0.2°, 18.352° ± 0.2°, 19.423° ± 0.2°, 20.453° ± 0.2°, 20.881° ± 0.2°, 21.194° ± 0.2°, 21.755° ± 0.2°, 22.732° ± 0.2°, 23.55° ± 0.2°, 24.217° ± 0.2°, 24.772° ± 0.2°, 25.208° ± 0.2°, 25.949° ± 0.2°, 26.825° ± 0.2°, 27.64° ± 0.2°, 28.694° ± 0.2°, 29.143° ± 0.2°, 30.154° ± 0.2°, 30.774° ± 0.2°, 31.537° ± 0.2°, 32.318° ± 0.2°, 33.834° ± 0.2°, 35.49° ± 0.2°, 36.347° ± 0.2°, 37.793° ± 0.2°, and 38.493° ± 0.2°.

In one embodiment, the crystalline form G of the compound of formula I has X-ray powder diffraction pattern analysis data substantially as shown in Table 7:

**Table 7. X-ray powder diffraction pattern analysis data for the crystalline form G of the compound of formula I**

| Position [°2θ] ± 0.2° | d-spacing [Å] | Relative intensity [%] |
|---|---|---|
| 5.279 | 16.725 | 100 |
| 6.388 | 13.8245 | 29.5 |
| 7.147 | 12.3582 | 7.1 |
| 9.02 | 9.796 | 23.3 |
| 10.926 | 8.0908 | 38.7 |
| 11.843 | 7.4665 | 56.2 |
| 12.68 | 6.9755 | 28.4 |
| 13.44 | 6.5825 | 15 |
| 14.162 | 6.2485 | 14.2 |
| 14.96 | 5.917 | 29.8 |
| 15.758 | 5.6193 | 31.4 |
| 16.187 | 5.4711 | 41.1 |
| 16.831 | 5.2633 | 46.7 |
| 17.98 | 4.9295 | 33.2 |
| 18.352 | 4.8304 | 22.2 |
| 19.423 | 4.5663 | 18 |
| 20.453 | 4.3386 | 60.2 |
| 20.881 | 4.2507 | 21 |
| 21.194 | 4.1887 | 34.3 |
| 21.755 | 4.0818 | 11.7 |
| 22.732 | 3.9085 | 22.3 |
| 23.55 | 3.7746 | 66.7 |
| 24.217 | 3.6722 | 19.7 |
| 24.772 | 3.5912 | 17.3 |
| 25.208 | 3.53 | 29.6 |
| 25.949 | 3.4309 | 35.8 |
| 26.825 | 3.3208 | 36.7 |
| 27.64 | 3.2246 | 8.8 |
| 28.694 | 3.1086 | 11.1 |
| 29.143 | 3.0617 | 16 |
| 30.154 | 2.9613 | 13.6 |
| 30.774 | 2.903 | 12.8 |
| 31.537 | 2.8345 | 13 |
| 32.318 | 2.7677 | 13.1 |
| 33.834 | 2.6471 | 13.1 |
| 35.49 | 2.5273 | 8.8 |
| 36.347 | 2.4697 | 10 |
| 37.793 | 2.3784 | 8.6 |
| 38.493 | 2.3368 | 8.6 |

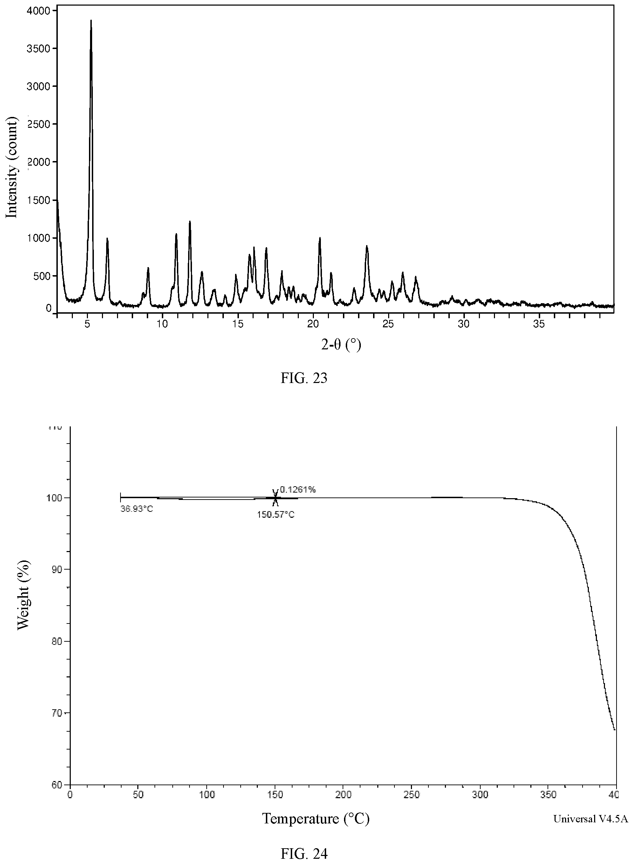

In one embodiment, the crystalline form G of the compound of formula I has an X-ray powder diffraction pattern substantially as shown in FIG. 23.

In one embodiment, the crystalline form G of the compound of formula I has a thermogravimetric analysis (TGA) curve showing a weight loss of 0.1261% ± 0.2% during heating from 36.93 °C ± 3 °C to 150.57 °C ± 3 °C.

In one embodiment, the crystalline form G of the compound of formula I has a thermogravimetric analysis curve showing a weight loss of 0.1261% during heating from 36.93 °C to 150.57 °C.

In one embodiment, the crystalline form G of the compound of formula I has a thermogravimetric analysis curve substantially as shown in FIG. 24.

In one embodiment, the crystalline form G of the compound of formula I has a differential scanning calorimetry (DSC) curve having an endothermic peak with an initial temperature of 334.21 °C ± 3 °C.

In one embodiment, the crystalline form G of the compound of formula I has a differential scanning calorimetry curve having an endothermic peak with an initial temperature of 334.21 °C.

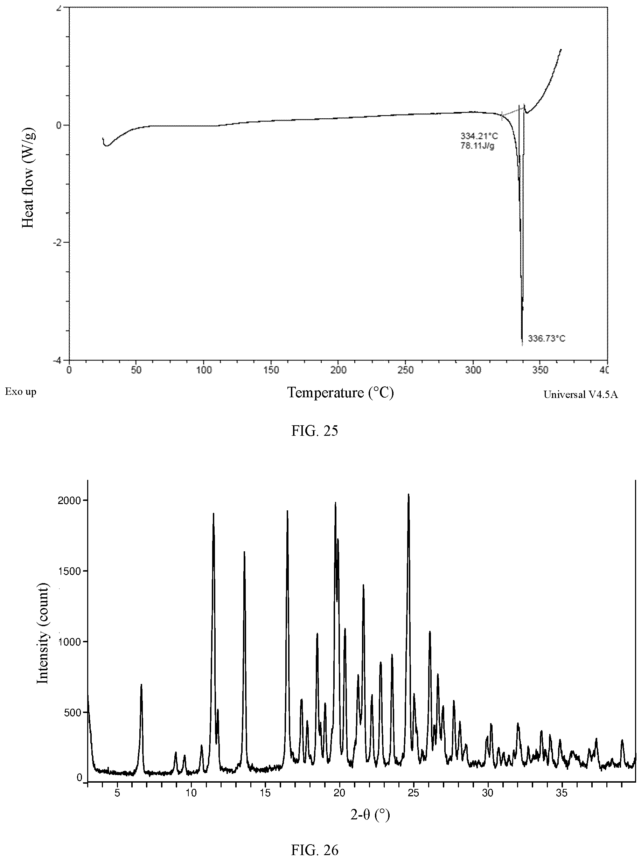

In one embodiment, the crystalline form G of the compound of formula I has a differential scanning calorimetry curve substantially as shown in FIG. 25.

The present invention further provides a preparation method for the crystalline form G of the compound of formula I, preferably any one of the following methods:
method I comprising the following steps: stirring a crystalline form A of a compound of formula I in toluene to precipitate a solid, and separating and drying the solid to obtain the crystalline form G of the compound of formula I, wherein the stirring is preferably performed at a temperature of 20-45 °C; the stirring is preferably performed for a period of 1.5-2.5 days; and the crystalline form A of the compound of formula I and the toluene are preferably in a mass-to-volume ratio of (100 mg-250 mg):1 mL;
method II comprising the following steps: stirring a crystalline form B of a compound of formula I in methyl tert-butyl ether to precipitate a solid, and separating and drying the solid to obtain the crystalline form G of the compound of formula I, wherein the stirring is performed at a temperature of 40-60 °C; the stirring is preferably performed for a period of 1.5-2.5 days; the crystalline form B of the compound of formula I and the methyl tert-butyl ether are preferably in a mass-to-volume ratio of (250 mg-350 mg):1 mL; and
method III comprising the following steps: stirring an amorphous form of a compound of formula I in a solvent to precipitate a solid, and separating and drying the solid to obtain the crystalline form G of the compound of formula I, wherein the solvent is water, heptane, toluene, or a mixed solvent of methanol and water in a volume ratio of (0.5-1.5):1; the stirring is preferably performed at a temperature of 20-60 °C; the stirring is preferably performed for a period of 1.5-2.5 days; the amorphous form of the compound of formula I and the solvent are preferably in a mass-to-volume ratio of (250 mg-350 mg):1 mL; wherein when the solvent is heptane or a mixed solvent, the stirring is performed at a temperature of 40-60 °C.

In one embodiment, in the method I, the stirring is preferably performed at a temperature of 25 °C or 40 °C. The stirring is preferably performed for a period of 2 days. The crystalline form A of the compound of formula I and the toluene are preferably in a mass-to-volume ratio of 150 mg:1 mL or 200 mg:1 mL.

In one embodiment, in the method II, the stirring is preferably performed at a temperature of 50 °C. The stirring is preferably performed for a period of 2 days. The crystalline form A of the compound of formula I and the toluene are preferably in a mass-to-volume ratio of 300 mg:1 mL.

In one embodiment, in the method III, the stirring is preferably performed at a temperature of 25 °C or 50 °C. The stirring is preferably performed for a period of 2 days. The amorphous form of the compound of formula I and the solvent are preferably in a mass-to-volume ratio of 300 mg:1 mL. The methanol and the water are preferably in a volume ratio of 1:1. When the solvent is heptane or a mixed solvent, the stirring is preferably performed at a temperature of 50 °C.

The present invention provides a crystalline form of an N-methyl-2-pyrrolidone solvate of a compound of formula I, which has an X-ray powder diffraction (XRPD) pattern having at least three, at least four, at least five, or at least six characteristic peaks at the following 2Θ angles: 11.493° ± 0.2°, 13.56° ± 0.2°, 16.481° ± 0.2°, 19.713° ± 0.2°, 21.586° ± 0.2°, and 24.643° ± 0.2°;

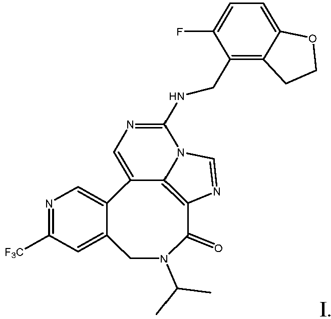

In one embodiment, the crystalline form of the N-methyl-2-pyrrolidone solvate of the compound of formula I has an X-ray powder diffraction pattern having characteristic peaks at the following 2Θ angles: 16.481° ± 0.2°, 19.713° ± 0.2°, and 24.643° ± 0.2°.

In one embodiment, the crystalline form of the N-methyl-2-pyrrolidone solvate of the compound of formula I has an X-ray powder diffraction pattern having characteristic peaks at the following 2Θ angles: 11.493° ± 0.2°, 16.481° ± 0.2°, 19.713° ± 0.2°, and 24.643° ± 0.2°.

In one embodiment, the crystalline form of the N-methyl-2-pyrrolidone solvate of the compound of formula I has an X-ray powder diffraction pattern having characteristic peaks at the following 2Θ angles: 11.493° ± 0.2°, 13.56° ± 0.2°, 16.481° ± 0.2°, 19.713° ± 0.2°, and 24.643° ± 0.2°.

In one embodiment, the crystalline form of the *N*-methyl-2-pyrrolidone solvate of the compound of formula I has an X-ray powder diffraction pattern having characteristic peaks at the following 2Θ angles: 11.493° ± 0.2°, 13.56° ± 0.2°, 16.481° ± 0.2°, 19.713° ± 0.2°, 21.586° ± 0.2°, and 24.643° ± 0.2°.

In one embodiment, the crystalline form of the N-methyl-2-pyrrolidone solvate of the compound of formula I has an X-ray powder diffraction pattern having characteristic peaks at the following 2Θ angles: 6.642° ± 0.2°, 11.493° ± 0.2°, 11.783° ± 0.2°, 13.56° ± 0.2°, 16.481° ± 0.2°, 17.45° ± 0.2°, 17.807° ± 0.2°, 18.486° ± 0.2°, 18.717° ± 0.2°, 19.013° ± 0.2°, 19.713° ± 0.2°, 20.355° ± 0.2°, 21.233° ± 0.2°, 21.586° ± 0.2°, 22.166° ± 0.2°, 22.752° ± 0.2°, 23.534° ± 0.2°, 24.643° ± 0.2°, 25.014° ± 0.2°, 26.083° ± 0.2°, 26.611° ± 0.2°, 26.976° ± 0.2°, 27.684° ± 0.2°, 28.092° ± 0.2°, 30.179° ± 0.2°, and 32.024° ± 0.2°.

In one embodiment, the crystalline form of the N-methyl-2-pyrrolidone solvate of the compound of formula I has an X-ray powder diffraction pattern having characteristic peaks at the following 2θ angles: 6.642° ± 0.2°, 8.957° ± 0.2°, 9.545° ± 0.2°, 10.679° ± 0.2°, 11.493° ± 0.2°, 11.783° ± 0.2°, 13.56° ± 0.2°, 16.481° ± 0.2°, 17.45° ± 0.2°, 17.807° ± 0.2°, 18.486° ± 0.2°, 18.717° ± 0.2°, 19.013° ± 0.2°, 19.713° ± 0.2°, 20.355° ± 0.2°, 21.233° ± 0.2°, 21.586° ± 0.2°, 22.166° ± 0.2°, 22.752° ± 0.2°, 23.534° ± 0.2°, 24.643° ± 0.2°, 25.014° ± 0.2°, 26.083° ± 0.2°, 26.359° ± 0.2°, 26.611° ± 0.2°, 26.976° ± 0.2°, 27.684° ± 0.2°, 28.092° ± 0.2°, 28.482° ± 0.2°, 29.955° ± 0.2°, 30.179° ± 0.2°, 30.702° ± 0.2°, 31.062° ± 0.2°, 31.749° ± 0.2°, 32.024° ± 0.2°, 32.71° ± 0.2°, 33.62° ± 0.2°, 33.877° ± 0.2°, 34.174° ± 0.2°, 35.648° ± 0.2°, 36.8° ± 0.2°, 37.301° ± 0.2°, 38.369° ± 0.2°, and 39.036° ± 0.2°.

In one embodiment, the crystalline form of the *N*-methyl-2-pyrrolidone solvate of the compound of formula I has X-ray powder diffraction pattern analysis data substantially as shown in Table 8:

**Table 8. X-ray powder diffraction pattern analysis data for the crystalline form of the N-methyl-2-pyrrolidone solvate of the compound of formula I**

| Position [°2θ] ± 0.2° | d-spacing [Å] | Relative intensity [%] |
|---|---|---|
| 6.642 | 13.2967 | 33.8 |
| 8.957 | 9.8646 | 10.3 |
| 9.545 | 9.2579 | 9.3 |
| 10.679 | 8.2776 | 12.8 |
| 11.493 | 7.693 | 93.3 |
| 11.783 | 7.5041 | 25.2 |
| 13.56 | 6.5245 | 79.9 |
| 16.481 | 5.3744 | 94.2 |
| 17.45 | 5.0778 | 28.6 |
| 17.807 | 4.9769 | 21.2 |
| 18.486 | 4.7956 | 51.5 |
| 18.717 | 4.7368 | 20.6 |
| 19.013 | 4.664 | 27.3 |
| 19.713 | 4.4998 | 97.1 |
| 20.355 | 4.3593 | 53.3 |
| 21.233 | 4.1809 | 37.2 |
| 21.586 | 4.1135 | 68.5 |
| 22.166 | 4.0071 | 30.3 |
| 22.752 | 3.9052 | 41.6 |
| 23.534 | 3.7771 | 44.2 |
| 24.643 | 3.6097 | 100 |
| 25.014 | 3.5569 | 30.7 |
| 26.083 | 3.4135 | 52.3 |
| 26.359 | 3.3784 | 19.7 |
| 26.611 | 3.347 | 37.5 |
| 26.976 | 3.3025 | 26.4 |
| 27.684 | 3.2196 | 28.3 |
| 28.092 | 3.1738 | 21.1 |
| 28.482 | 3.1312 | 13.1 |
| 29.955 | 2.9805 | 15.8 |
| 30.179 | 2.9589 | 20.2 |
| 30.702 | 2.9097 | 12 |
| 31.062 | 2.8768 | 10.2 |
| 31.749 | 2.8161 | 11 |
| 32.024 | 2.7925 | 20.4 |
| 32.71 | 2.7355 | 12.4 |
| 33.62 | 2.6635 | 17.8 |
| 33.877 | 2.6438 | 11.2 |
| 34.174 | 2.6216 | 16.4 |
| 35.648 | 2.5165 | 10.6 |
| 36.8 | 2.4403 | 11.5 |
| 37.301 | 2.4087 | 15.1 |
| 38.369 | 2.344 | 8.2 |
| 39.036 | 2.3055 | 14.6 |

In one embodiment, the crystalline form of the *N*-methyl-2-pyrrolidone solvate of the compound of formula I has an X-ray powder diffraction pattern substantially as shown in Table 26.

In one embodiment, the crystalline form of the *N*-methyl-2-pyrrolidone solvate of the compound of formula I has a thermogravimetric analysis (TGA) curve showing a weight loss of 15.56% ± 0.5% during heating from 114.32 °C ± 3 °C to 209.84 °C ± 3 °C.

In one embodiment, the crystalline form of the *N*-methyl-2-pyrrolidone solvate of the compound of formula I has a thermogravimetric analysis curve showing a weight loss of 15.56% during heating from 114.32 °C to 209.84 °C.

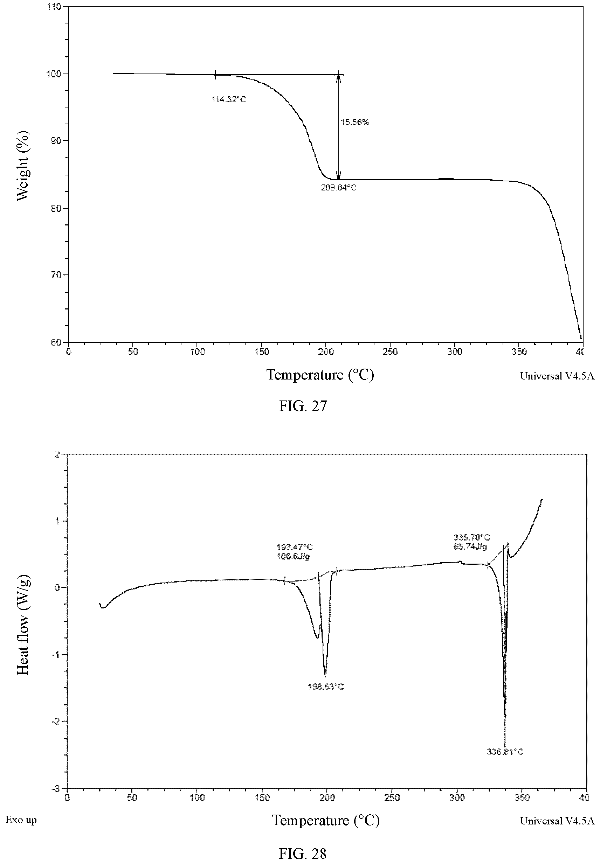

In one embodiment, the crystalline form of the *N*-methyl-2-pyrrolidone solvate of the compound of formula I has a thermogravimetric analysis curve substantially as shown in FIG. 27.

In one embodiment, the crystalline form of the *N*-methyl-2-pyrrolidone solvate of the compound of formula I has a differential scanning calorimetry (DSC) curve having endothermic peaks with initial temperatures of 193.47 °C ± 3 °C and 335.7 °C ± 3 °C, respectively.

In one embodiment, the crystalline form of the N-methyl-2-pyrrolidone solvate of the compound of formula I has a differential scanning calorimetry curve having endothermic peaks with initial temperatures of 193.47 °C and 335.7 °C, respectively.

In one embodiment, the crystalline form of the *N*-methyl-2-pyrrolidone solvate of the compound of formula I has a differential scanning calorimetry curve having endothermic peaks with peak temperatures of 198.63 °C ± 3 °C and 336.81 °C ± 3 °C, respectively.

In one embodiment, the crystalline form of the *N*-methyl-2-pyrrolidone solvate of the compound of formula I has a differential scanning calorimetry curve having endothermic peaks with peak temperatures of 198.63 °C and 336.81 °C, respectively.

In one embodiment, the crystalline form of the *N*-methyl-2-pyrrolidone solvate of the compound of formula I has a differential scanning calorimetry curve substantially as shown in FIG. 28.

In one embodiment, the crystalline form of the *N*-methyl-2-pyrrolidone solvate of the compound of formula I has a nuclear magnetic resonance hydrogen spectrum (¹H-NMR) showing the presence of *N*-methyl-2-pyrrolidone with a weight percentage of 15.3% ± 0.5%.

In one embodiment, the crystalline form of the *N*-methyl-2-pyrrolidone solvate of the compound of formula I has a nuclear magnetic resonance hydrogen spectrum showing the presence of *N*-methyl-2-pyrrolidone with a weight percentage of 15.3% and with a molar ratio of 1:0.96 to the compound of formula I.

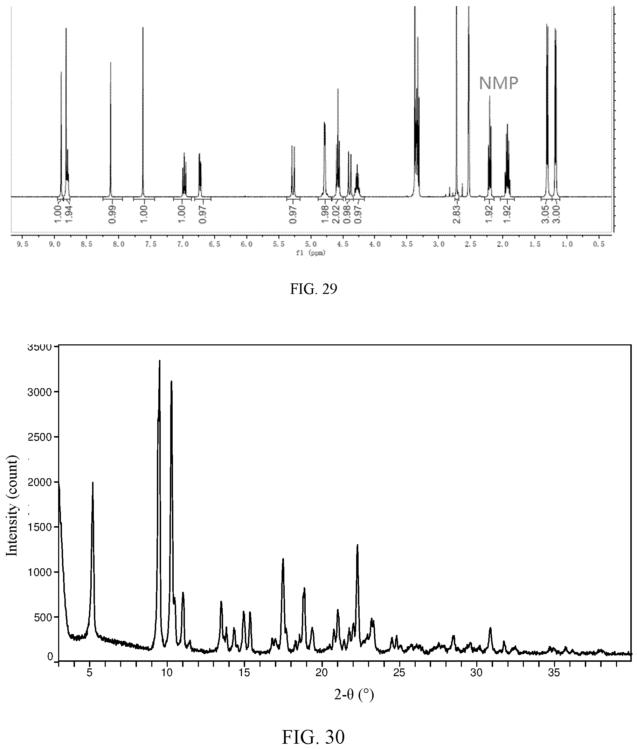

In one embodiment, the crystalline form of the *N*-methyl-2-pyrrolidone solvate of the compound of formula I has a nuclear magnetic resonance hydrogen spectrum as shown in FIG. 29.

The present invention further provides a preparation method for the crystalline form of the *N*-methyl-2-pyrrolidone solvate of the compound of formula I, which preferably comprises the following steps: stirring an amorphous form of a compound of formula I in *N*-methyl-2-pyrrolidone to precipitate a solid, and separating and drying the solid to obtain the crystalline form of the *N*-methyl-2-pyrrolidone solvate of the compound of formula I, wherein the stirring is preferably performed at a temperature of 20-60 °C; the stirring is preferably performed for a period of 1.5-2.5 days; the amorphous form of the compound of formula I and the N-methyl-2-pyrrolidone are preferably in a mass-to-volume ratio of (200 mg-350 mg):1 mL.

In one embodiment, in the preparation method for the crystalline form of the N-methyl-2-pyrrolidone solvate of the compound of formula I, the stirring is preferably performed at a temperature of 25 °C or 50 °C. The stirring is preferably performed for a period of 2 days. The amorphous form of the compound of formula I and the N-methyl-2-pyrrolidone are preferably in a mass-to-volume ratio of 250 mg:1 mL or 300 mg:1 mL.

The present invention provides a crystalline form of a DMF solvate of a compound of formula I, which has an X-ray powder diffraction (XRPD) pattern having at least three, at least four, or at least five characteristic peaks at the following 2Θ angles: 5.2° ± 0.2°, 9.503° ± 0.2°, 10.267° ± 0.2°, 17.491° ± 0.2°, and 22.283° ± 0.2°;

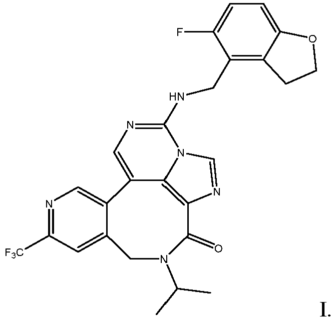

In one embodiment, the crystalline form of the DMF solvate of the compound of formula I has an X-ray powder diffraction pattern having characteristic peaks at the following 2Θ angles: 5.2° ± 0.2°, 9.503° ± 0.2°, and 10.267° ± 0.2°.

In one embodiment, the crystalline form of the DMF solvate of the compound of formula I has an X-ray powder diffraction pattern having characteristic peaks at the following 2Θ angles: 5.2° ± 0.2°, 9.503° ± 0.2°, 10.267° ± 0.2°, and 22.283° ± 0.2°.

In one embodiment, the crystalline form of the DMF solvate of the compound of formula I has an X-ray powder diffraction pattern having characteristic peaks at the following 2Θ angles: 5.2° ± 0.2°, 9.503° ± 0.2°, 10.267° ± 0.2°, 11.008° ± 0.2°, 13.5° ± 0.2°, 13.831° ± 0.2°, 14.32° ± 0.2°, 14.943° ± 0.2°, 15.353° ± 0.2°, 17.491° ± 0.2°, 18.856° ± 0.2°, 19.363° ± 0.2°, 20.75° ± 0.2°, 21.018° ± 0.2°, 21.741° ± 0.2°, 22.03° ± 0.2°, 22.283° ± 0.2°, 23.317° ± 0.2°, and 30.874° ± 0.2°.

In one embodiment, the crystalline form of the DMF solvate of the compound of formula I has an X-ray powder diffraction pattern having characteristic peaks at the following 2Θ angles: 5.2° ± 0.2°, 9.503° ± 0.2°, 10.267° ± 0.2°, 11.008° ± 0.2°, 13.5° ± 0.2°, 13.831° ± 0.2°, 14.32° ± 0.2°, 14.943° ± 0.2°, 15.353° ± 0.2°, 16.778° ± 0.2°, 16.986° ± 0.2°, 17.491° ± 0.2°, 18.547° ± 0.2°, 18.856° ± 0.2°, 19.363° ± 0.2°, 20.489° ± 0.2°, 20.75° ± 0.2°, 21.018° ± 0.2°, 21.424° ± 0.2°, 21.741° ± 0.2°, 22.03° ± 0.2°, 22.283° ± 0.2°, 22.926° ± 0.2°, 23.317° ± 0.2°, 24.507° ± 0.2°, 24.814° ± 0.2°, 25.068° ± 0.2°, 25.771° ± 0.2°, 26.1° ± 0.2°, 26.33° ± 0.2°, 27.544° ± 0.2°, 27.835° ± 0.2°, 28.532° ± 0.2°, 28.784° ± 0.2°, 29.572° ± 0.2°, 30.194° ± 0.2°, 30.874° ± 0.2°, 31.738° ± 0.2°, 32.49° ± 0.2°, 34.71° ± 0.2°, 34.933° ± 0.2°, 35.709° ± 0.2°, and 36.156° ± 0.2°.

In one embodiment, the crystalline form of the DMF solvate of the compound of formula I has X-ray powder diffraction pattern analysis data substantially as shown in Table 9:

**Table 9. X-ray powder diffraction pattern analytical data for the crystalline form of the DMF solvate of the compound of formula I**

| Position [°2θ] ± 0.2° | d-spacing [Å] | Relative intensity [%] |
|---|---|---|
| 5.2 | 16.9811 | 59.5 |
| 9.503 | 9.2995 | 100 |
| 10.267 | 8.609 | 93.2 |
| 11.008 | 8.0311 | 22.9 |
| 13.5 | 6.5534 | 19.9 |
| 13.831 | 6.3975 | 11.3 |
| 14.32 | 6.1802 | 11.1 |
| 14.943 | 5.9238 | 16.5 |
| 15.353 | 5.7665 | 16.4 |
| 16.778 | 5.2799 | 7.6 |
| 16.986 | 5.2157 | 7.5 |
| 17.491 | 5.0661 | 34.1 |
| 18.547 | 4.7799 | 8.9 |
| 18.856 | 4.7022 | 24.4 |
| 19.363 | 4.5804 | 11.2 |
| 20.489 | 4.3311 | 5.6 |
| 20.75 | 4.2772 | 10.6 |
| 21.018 | 4.2232 | 17.1 |
| 21.424 | 4.1441 | 7.2 |
| 21.741 | 4.0844 | 11.1 |
| 22.03 | 4.0315 | 12.6 |
| 22.283 | 3.9863 | 38.8 |
| 22.926 | 3.8759 | 8.9 |
| 23.317 | 3.8118 | 13.4 |
| 24.507 | 3.6294 | 7.8 |
| 24.814 | 3.5851 | 8.5 |
| 25.068 | 3.5494 | 5.3 |
| 25.771 | 3.4541 | 5.6 |
| 26.1 | 3.4113 | 5.7 |
| 26.33 | 3.3821 | 5.3 |
| 27.544 | 3.2357 | 6.1 |
| 27.835 | 3.2025 | 5.1 |
| 28.532 | 3.1259 | 8.4 |
| 28.784 | 3.0991 | 4.6 |
| 29.572 | 3.0183 | 6.2 |
| 30.194 | 2.9574 | 5.2 |
| 30.874 | 2.8939 | 11.1 |
| 31.738 | 2.817 | 6.7 |
| 32.49 | 2.7535 | 5 |
| 34.71 | 2.5823 | 4.8 |
| 34.933 | 2.5663 | 4.4 |
| 35.709 | 2.5123 | 4.8 |
| 36.156 | 2.4823 | 3.8 |

In one embodiment, the crystalline form of the DMF solvate of the compound of formula I has an X-ray powder diffraction pattern substantially as shown in FIG. 30.

In one embodiment, the crystalline form of the DMF solvate of the compound of formula I has a thermogravimetric analysis (TGA) curve showing a weight loss of 3.381% ± 0.5% in DMF during heating from 38.31 °C ± 3 °C to 196.62 °C ± 3 °C.

In one embodiment, the crystalline form of the DMF solvate of the compound of formula I has a thermogravimetric analysis curve showing a weight loss of 3.381% in DMF during heating from 38.31 °C to 196.62 °C.

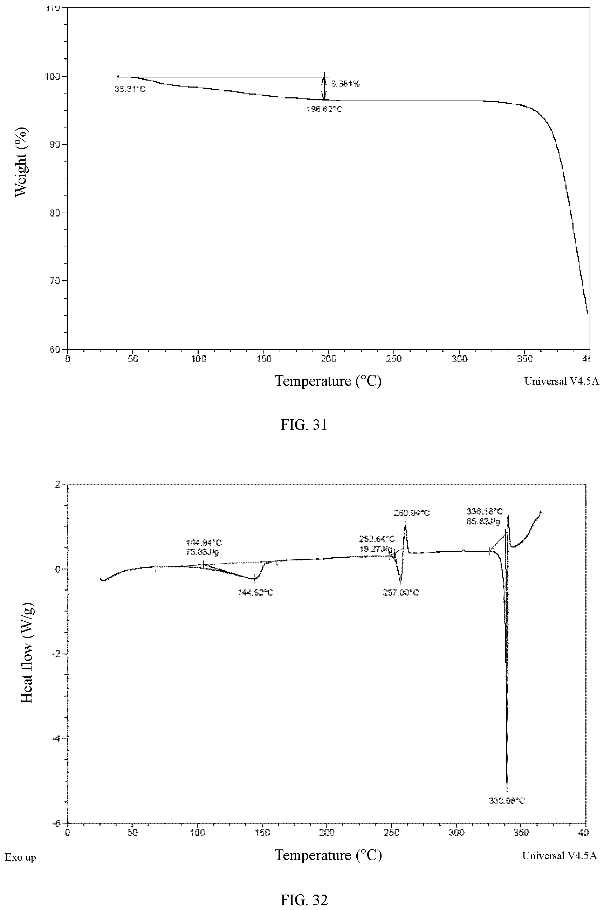

In one embodiment, the DMF solvate of the compound of formula I has a thermogravimetric analysis curve substantially as shown in FIG. 31.

In one embodiment, the crystalline form of the DMF solvate of the compound of formula I has a differential scanning calorimetry (DSC) curve having endothermic peaks with initial temperatures of 104.94 °C ± 3 °C, 252.64 °C ± 3 °C, and 338.18 °C ± 3 °C, respectively.

In one embodiment, the crystalline form of the DMF solvate of the compound of formula I has a differential scanning calorimetry curve having endothermic peaks with initial temperatures of 104.94 °C, 252.64 °C, and 338.18 °C, respectively.

In one embodiment, the crystalline form of the DMF solvate of the compound of formula I has a differential scanning calorimetry curve having endothermic peaks with peak temperatures of 114.52 °C ± 3 °C, 257 °C ± 3 °C, and 338.98 °C ± 3 °C, respectively.

In one embodiment, the crystalline form of the DMF solvate of the compound of formula I has a differential scanning calorimetry curve having endothermic peaks with peak temperatures of 114.52 °C, 257 °C, and 338.98 °C, respectively.

In one embodiment, the crystalline form of the DMF solvate of the compound of formula I has a differential scanning calorimetry curve having an exothermic peak with a peak temperature of 260.94 °C ± 3 °C.

In one embodiment, the crystalline form of the DMF solvate of the compound of formula I has a differential scanning calorimetry curve having an exothermic peak with a peak temperature of 260.94 °C.

In one embodiment, the crystalline form of the DMF solvate of the compound of formula I has a differential scanning calorimetry curve substantially as shown in FIG. 32.

In one embodiment, the crystalline form of the DMF solvate of the compound of formula I has a nuclear magnetic resonance hydrogen spectrum (¹H-NMR) showing the presence of toluene with a weight percentage of 10.3% ± 0.5%.

In one embodiment, the crystalline form of the DMF solvate of the compound of formula I has a nuclear magnetic resonance hydrogen spectrum showing the presence of DMF with a weight percentage of 10.3% and with a molar ratio of 1:0.83 to the compound of formula I.

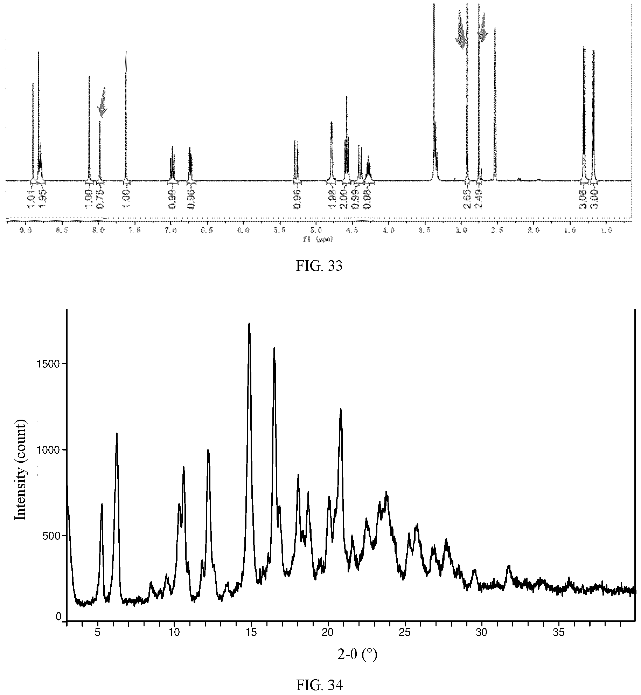

In one embodiment, the crystalline form of the DMF solvate of the compound of formula I has a nuclear magnetic resonance hydrogen spectrum as shown in FIG. 33.

The present invention further provides a preparation method for the crystalline form of the DMF solvate of the compound of formula I, which preferably comprises the following steps: stirring an amorphous form of a compound of formula I in DMF to precipitate a solid, and separating and drying the solid to obtain the crystalline form of the DMF solvate of the compound of formula I, wherein the stirring is preferably performed at a temperature of 20-60 °C; the stirring is preferably performed for a period of 1.5-2.5 days; and the amorphous form of the compound of formula I and the DMF are preferably in a mass-to-volume ratio of (200 mg-350 mg):1 mL.

In one embodiment, in the preparation method for the crystalline form of the DMF solvate of the compound of formula I, the stirring is preferably performed at a temperature of 25 °C or 50 °C. The stirring is preferably performed for a period of 2 days. The amorphous form of the compound of formula I and the DMF are preferably in a mass-to-volume ratio of 250 mg:1 mL or 300 mg:1 mL.

The present invention provides a crystalline form K of a compound of formula I, which has an X-ray powder diffraction (XRPD) pattern having at least three, at least four, at least five, or at least six characteristic peaks at the following 2Θ angles: 6.233° ± 0.2°, 10.598° ± 0.2°, 12.177° ± 0.2°, 14.844° ± 0.2°, 16.48° ± 0.2°, and 20.823° ± 0.2°;

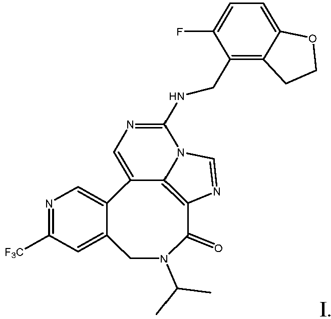

In one embodiment, the crystalline form K of the compound of formula I has an X-ray powder diffraction pattern having characteristic peaks at the following 2Θ angles: 14.844° ± 0.2°, 16.48° ± 0.2°, and 20.823° ± 0.2°.

In one embodiment, the crystalline form K of the compound of formula I has an X-ray powder diffraction pattern having characteristic peaks at the following 2Θ angles: 6.233° ± 0.2°, 14.844° ± 0.2°, 16.48° ± 0.2°, and 20.823° ± 0.2°.

In one embodiment, the crystalline form K of the compound of formula I has an X-ray powder diffraction pattern having characteristic peaks at the following 2Θ angles: 6.233° ± 0.2°, 12.177° ± 0.2°, 14.844° ± 0.2°, 16.48° ± 0.2°, and 20.823° ± 0.2°.

In one embodiment, the crystalline form K of the compound of formula I has an X-ray powder diffraction pattern having characteristic peaks at the following 2Θ angles: 6.233° ± 0.2°, 10.598° ± 0.2°, 12.177° ± 0.2°, 14.844° ± 0.2°, 16.48° ± 0.2°, and 20.823° ± 0.2°.

In one embodiment, the crystalline form K of the compound of formula I has an X-ray powder diffraction pattern having characteristic peaks at the following 2Θ angles: 5.275° ± 0.2°, 6.233° ± 0.2°, 8.473° ± 0.2°, 9.485° ± 0.2°, 10.305° ± 0.2°, 10.907° ± 0.2°, 11.804° ± 0.2°, 12.117° ± 0.2°, 12.601° ± 0.2°, 13.477° ± 0.2°, 14.844° ± 0.2°, 16.48° ± 0.2°, 16.831° ± 0.2°, 18.038° ± 0.2°, 18.681° ± 0.2°, 20.062° ± 0.2°, 20.083° ± 0.2°, 21.548° ± 0.2°, 22.461° ± 0.2°, 23.298° ± 0.2°, 23.766° ± 0.2°, 25.244° ± 0.2°, 25.791° ± 0.2°, 26.886° ± 0.2°, 27.665° ± 0.2°, 28.012° ± 0.2°, 29.513° ± 0.2°, 31.789° ± 0.2°, 33.973° ± 0.2°, and 35.645° ± 0.2°.

In one embodiment, the crystalline form K of the compound of formula I has X-ray powder diffraction pattern analysis data substantially as shown in Table 10:

**Table 10. X-ray powder diffraction pattern analysis data for the crystalline form K of the compound of formula I**

| Position [°2θ] ± 0.2° | d-spacing [Å] | Relative intensity [%] |
|---|---|---|
| 5.275 | 16.7397 | 39.2 |
| 6.233 | 14.1675 | 63.1 |
| 8.473 | 10.4265 | 13.1 |
| 9.485 | 9.3168 | 15.7 |
| 10.305 | 8.5771 | 39.4 |
| 10.598 | 8.3408 | 51.8 |
| 10.907 | 8.1051 | 18 |
| 11.804 | 7.4913 | 20.4 |
| 12.177 | 7.2624 | 57.4 |
| 12.601 | 7.0187 | 18.8 |
| 13.477 | 6.5648 | 13 |
| 14.844 | 5.9628 | 100 |
| 16.48 | 5.3745 | 91.7 |
| 16.831 | 5.2634 | 38.5 |
| 18.038 | 4.9137 | 49 |
| 18.681 | 4.7459 | 43.3 |
| 20.062 | 4.4223 | 41.8 |
| 20.823 | 4.2624 | 71.2 |
| 21.548 | 4.1205 | 28.7 |
| 22.461 | 3.9551 | 34.5 |
| 23.298 | 3.8148 | 39 |
| 23.766 | 3.7407 | 43.3 |
| 25.244 | 3.525 | 29.4 |
| 25.791 | 3.4515 | 32.5 |
| 26.886 | 3.3134 | 24.2 |
| 27.665 | 3.2218 | 27.7 |
| 28.012 | 3.1826 | 20.8 |
| 29.513 | 3.0241 | 17.3 |
| 31.789 | 2.8126 | 18.6 |
| 33.973 | 2.6366 | 13.6 |
| 35.645 | 2.5167 | 14.6 |

In one embodiment, the crystalline form K of the compound of formula I has an X-ray powder diffraction pattern substantially as shown in FIG. 34.

In one embodiment, the crystalline form K of the compound of formula I has a differential scanning calorimetry (DSC) curve having an endothermic peak with an initial temperature of 333.08 °C ± 3 °C.

In one embodiment, the crystalline form K of the compound of formula I has a differential scanning calorimetry curve having an endothermic peak with an initial temperature of 333.08 °C.

In one embodiment, the crystalline form K of the compound of formula I has a differential scanning calorimetry curve having an endothermic peak with a final temperature of 339.22 °C ± 3 °C.

In one embodiment, the crystalline form K of the compound of formula I has a differential scanning calorimetry curve having an endothermic peak with a final temperature of 339.22 °C.

In one embodiment, the crystalline form K of the compound of formula I has a differential scanning calorimetry curve having an endothermic peak with a peak temperature of 335.88 °C ± 3 °C.

In one embodiment, the crystalline form K of the compound of formula I has a differential scanning calorimetry curve having an endothermic peak with a peak temperature of 335.88 °C.

In one embodiment, the crystalline form K of the compound of formula I has a differential scanning calorimetry curve having an exothermic peak with a peak temperature of 303.12 °C ± 3 °C.

In one embodiment, the crystalline form K of the compound of formula I has a differential scanning calorimetry curve having an exothermic peak with a peak temperature of 303.12 °C.

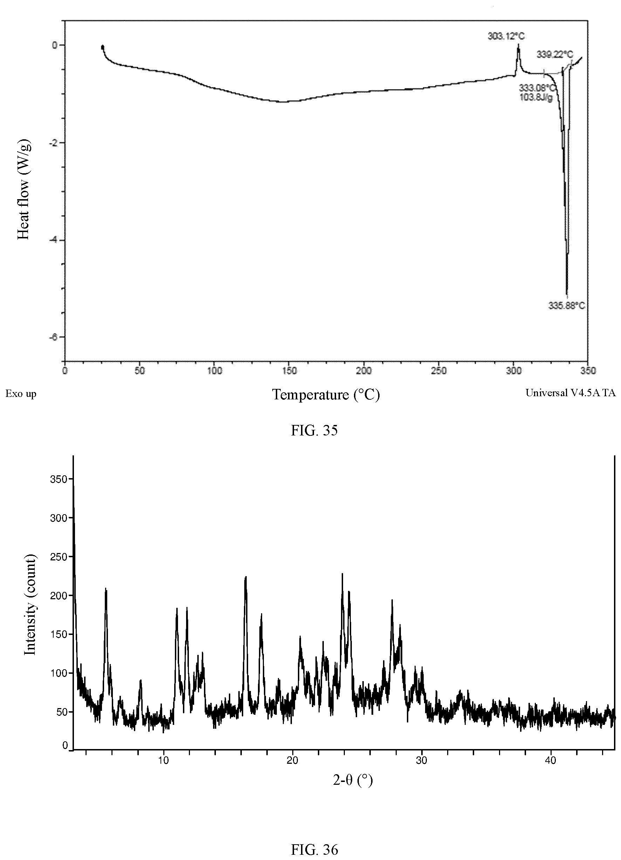

In one embodiment, the crystalline form K of the compound of formula I has a differential scanning calorimetry curve substantially as shown in FIG. 35.

The present invention further provides a preparation method for the crystalline form K of the compound of formula I, which preferably comprises the following steps: keeping a crystalline form B of a compound of formula I at a temperature of 250-300 °C for 1-5 min to obtain the crystalline form K of the compound of formula I.

In an embodiment, in the preparation method for the crystalline form K of the compound of formula I, the crystalline form B of the compound of formula I is preferably kept at a temperature of 280 °C for 2 min.

The present invention provides a crystalline form A of a hydrochloride salt of a compound of formula I, which has an X-ray powder diffraction (XRPD) pattern having at least three, at least four, at least five, at least six, at least seven, at least eight characteristic peaks at the following 2Θ angles: 5.527° ± 0.2°, 11.026° ± 0.2°, 11.805° ± 0.2°, 16.39° ± 0.2°, 17.572° ± 0.2°, 23.853° ± 0.2°, 24.398° ± 0.2°, and 27.683° ± 0.2°;

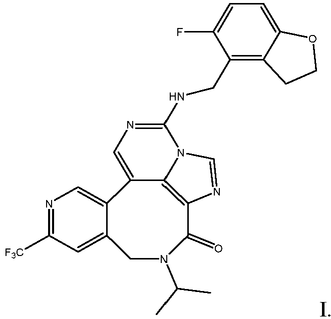

In one embodiment, the crystalline form A of the hydrochloride salt of the compound of formula I has an X-ray powder diffraction pattern having characteristic peaks at the following 2Θ angles: 5.527° ± 0.2°, 16.39° ± 0.2°, and 23.853° ± 0.2°.

In one embodiment, the crystalline form A of the hydrochloride salt of the compound of formula I has an X-ray powder diffraction pattern having characteristic peaks at the following 2Θ angles: 5.527° ± 0.2°, 16.39° ± 0.2°, 23.853° ± 0.2°, and 24.398° ± 0.2°.

In one embodiment, the crystalline form A of the hydrochloride salt of the compound of formula I has an X-ray powder diffraction pattern having characteristic peaks at the following 2Θ angles: 5.527° ± 0.2°, 16.39° ± 0.2°, 23.853° ± 0.2°, 24.398° ± 0.2°, and 27.683° ± 0.2°.

In one embodiment, the crystalline form A of the hydrochloride salt of the compound of formula I has an X-ray powder diffraction pattern having characteristic peaks at the following 2Θ angles: 5.527° ± 0.2°, 11.805° ± 0.2°, 16.39° ± 0.2°, 23.853° ± 0.2°, 24.398° ± 0.2°, and 27.683° ± 0.2°.

In one embodiment, the crystalline form A of the hydrochloride salt of the compound of formula I has an X-ray powder diffraction pattern having characteristic peaks at the following 2Θ angles: 5.527° ± 0.2°, 11.026° ± 0.2°, 11.805° ± 0.2°, 16.39° ± 0.2°, 23.853° ± 0.2°, 24.398° ± 0.2°, and 27.683° ± 0.2°.

In one embodiment, the crystalline form A of the hydrochloride salt of the compound of formula I has an X-ray powder diffraction pattern having characteristic peaks at the following 2Θ angles: 5.527° ± 0.2°, 11.026° ± 0.2°, 11.805° ± 0.2°, 16.39° ± 0.2°, 17.572° ± 0.2°, 23.853° ± 0.2°, 24.398° ± 0.2°, and 27.683° ± 0.2°.

In one embodiment, the crystalline form A of the hydrochloride salt of the compound of formula I has an X-ray powder diffraction pattern having characteristic peaks at the following 2Θ angles: 5.527° ± 0.2°, 11.026° ± 0.2°, 11.805° ± 0.2°, 12.655° ± 0.2°, 13.027° ± 0.2°, 16.39° ± 0.2°, 17.572° ± 0.2°, 20.57° ± 0.2°, 21.819° ± 0.2°, 22.358° ± 0.2°, 22.685° ± 0.2°, 23.853° ± 0.2°, 24.398° ± 0.2°, 27.037° ± 0.2°, 27.683° ± 0.2°, and 28.304° ± 0.2°.

In one embodiment, the crystalline form A of the hydrochloride salt of the compound of formula I has an X-ray powder diffraction pattern having characteristic peaks at the following 2Θ angles: 5.527° ± 0.2°, 5.911° ± 0.2°, 8.228° ± 0.2°, 11.026° ± 0.2°, 11.805° ± 0.2°, 12.655° ± 0.2°, 13.027° ± 0.2°, 16.39° ± 0.2°, 17.572° ± 0.2°, 18.876° ± 0.2°, 20.57° ± 0.2°, 21.819° ± 0.2°, 22.358° ± 0.2°, 22.685° ± 0.2°, 23.284° ± 0.2°, 23.853° ± 0.2°, 24.398° ± 0.2°, 27.037° ± 0.2°, 27.683° ± 0.2°, 28.304° ± 0.2°, 29.471° ± 0.2°, 30.051° ± 0.2°, 33.574° ± 0.2°, 36.726° ± 0.2°, and 38.297° ± 0.2°.

In one embodiment, the crystalline form A of the hydrochloride salt of the compound of formula I has X-ray powder diffraction pattern analysis data substantially as shown in Table 11:

**Table 11. X-ray powder diffraction pattern analysis data for the crystalline form A of the hydrochloride salt of the compound of formula I**

| Position [°2θ] ± 0.2° | d-spacing [Å] | Relative intensity [%] |
|---|---|---|
| 5.527 | 15.976 | 91.7 |
| 5.911 | 14.9386 | 43.9 |
| 8.228 | 10.7364 | 39.5 |
| 11.026 | 8.0181 | 80.3 |
| 11.805 | 7.4902 | 80.7 |
| 12.655 | 6.9891 | 53.5 |
| 13.027 | 6.7903 | 52.2 |
| 16.39 | 5.4037 | 98.2 |
| 17.572 | 5.0428 | 77.2 |
| 18.876 | 4.6974 | 40.4 |
| 20.57 | 4.3142 | 64.5 |
| 21.819 | 4.07 | 52.6 |
| 22.358 | 3.9731 | 61.4 |
| 22.685 | 3.9165 | 51.8 |
| 23.284 | 3.8171 | 49.6 |
| 23.853 | 3.7273 | 100 |
| 24.398 | 3.6453 | 89.5 |
| 27.037 | 3.2952 | 50.4 |
| 27.683 | 3.2198 | 85.1 |
| 28.304 | 3.1505 | 71.1 |
| 29.471 | 3.0283 | 47.4 |
| 30.051 | 2.9712 | 43.9 |
| 33.574 | 2.667 | 33.8 |
| 36.726 | 2.445 | 29.4 |
| 38.297 | 2.3483 | 26.3 |

In one embodiment, the crystalline form A of the hydrochloride salt of the compound of formula I has an X-ray powder diffraction pattern substantially as shown in FIG. 36.

In one embodiment, the crystalline form A of the hydrochloride salt of the compound of formula I has a thermogravimetric analysis (TGA) curve showing a weight loss of 1.033% ± 0.5% during heating from 25.19 °C ± 3 °C to 108.66 °C ± 3 °C; and a weight loss of 6.683% ± 0.5% during heating from 108.66 °C ± 3 °C from 209.57 °C ± 3 °C.

In one embodiment, the crystalline form A of the hydrochloride salt of the compound of formula I has a thermogravimetric analysis curve showing a weight loss of 1.033% during heating from 25.19 °C to 108.66 °C; and a weight loss of 6.683% during heating from 108.66 °C to 209.57 °C.

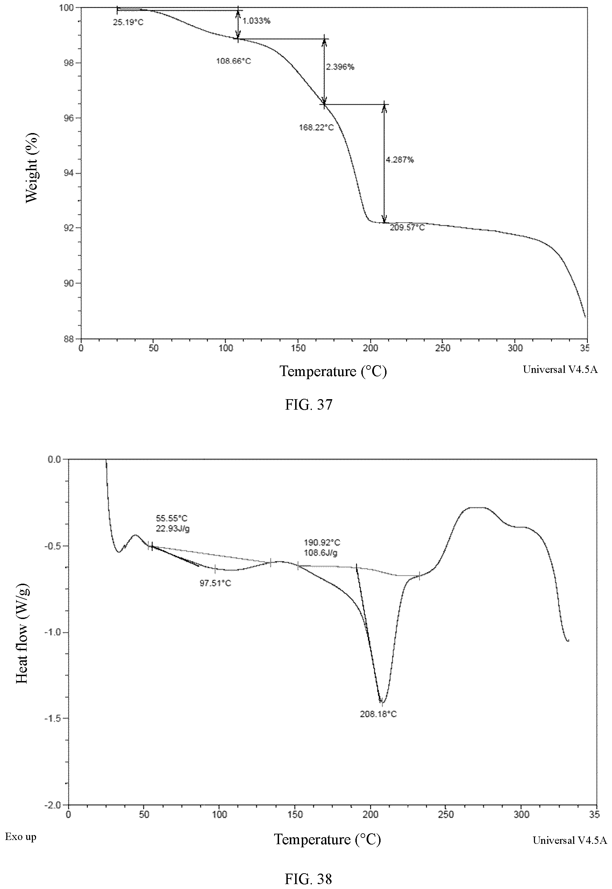

In one embodiment, the crystalline form A of the hydrochloride salt of the compound of formula I has a thermogravimetric analysis curve substantially as shown in FIG. 37.

In one embodiment, the crystalline form A of the hydrochloride salt of the compound of formula I has a differential scanning calorimetry (DSC) curve having endothermic peaks with initial temperatures of 55.55 °C ± 3 °C and 190.92 °C ± 3 °C, respectively.

In one embodiment, the crystalline form A of the hydrochloride salt of the compound of formula I has a differential scanning calorimetry curve having endothermic peaks with initial temperatures of 55.55 °C and 190.92 °C, respectively.

In one embodiment, the crystalline form A of the hydrochloride salt of the compound of formula I has a differential scanning calorimetry curve having endothermic peaks with peak temperatures of 97.51 °C ± 3 °C and 208.18 °C ± 3 °C, respectively.

In one embodiment, the crystalline form A of the hydrochloride salt of the compound of formula I has a differential scanning calorimetry curve having endothermic peaks with peak temperatures of 97.51 °C and 208.18 °C, respectively.

In one embodiment, the crystalline form A of the hydrochloride salt of the compound of formula I has a differential scanning calorimetry curve substantially as shown in FIG. 38.

In one embodiment, the crystalline form A of the hydrochloride salt of the compound of formula I has a dynamic vapor sorption (DVS) curve showing a hygroscopic weight gain of 0.31% ± 0.2% at 25 °C and 80% RH.

In one embodiment, the crystalline form A of the hydrochloride salt of the compound of formula I has a dynamic vapor sorption curve showing a hygroscopic weight gain of 0.31% at 25 °C and 80% RH.

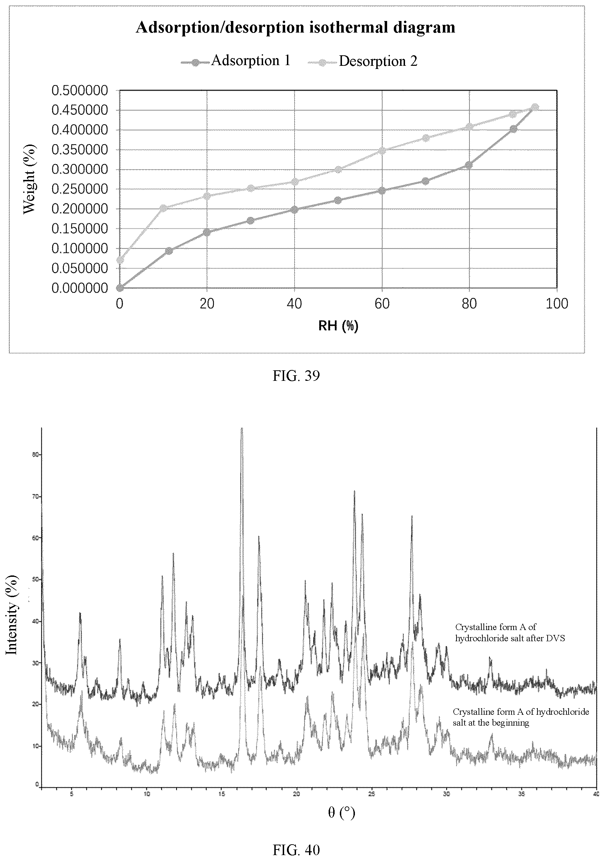

In one embodiment, the crystalline form A of the hydrochloride salt of the compound of formula I has a dynamic vapor sorption curve as shown in FIG. 39.

The present invention further provides a preparation method for the crystalline form A of the hydrochloride salt of the compound of formula I, which preferably comprises the following steps: mixing and stirring a compound of formula I, hydrochloric acid, and tetrahydrofuran to precipitate a solid, and separating and drying the solid to obtain the crystalline form A of the hydrochloride salt of the compound of formula I, wherein the stirring is performed at a temperature of 20-45 °C; the stirring is preferably performed for a period of 0.2-0.8 h; the compound of formula I and the hydrochloric acid are preferably in a molar ratio of (1-1.5): 1; and the compound of formula I and the tetrahydrofuran are preferably in a mass-to-volume ratio of (20 mg-30 mg):1 mL.

In one embodiment, in the preparation method for the crystalline form A of the hydrochloride salt of the compound of formula I, the compound of formula I is preferably a crystalline form A of a compound of formula I. The stirring is preferably performed at a temperature of 25 °C or 40 °C. The stirring is preferably performed for a period of 0.5 h. The compound of the formula I and the hydrochloric acid are preferably in a molar ratio of 1.1:1. The compound of formula I and the tetrahydrofuran are preferably in a mass-to-volume ratio of 23.85 mg:1 mL.

The present invention provides a crystalline form of an ethanol solvate of a hydrochloride salt of a compound of formula I, which has an X-ray powder diffraction (XRPD) pattern having at least three, at least four, at least five, or at least six characteristic peaks at the following 2Θ angles: 6.24° ± 0.2°, 14.43° ± 0.2°, 15.744° ± 0.2°, 18.391° ± 0.2°, 24.01° ± 0.2°, and 26.261° ± 0.2°;

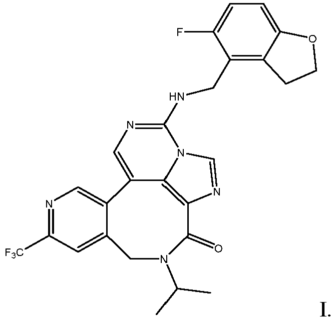

In one embodiment, the crystalline form of the ethanol solvate of the hydrochloride salt of the compound of formula I has an X-ray powder diffraction pattern having characteristic peaks at the following 2Θ angles: 6.24° ± 0.2°, 15.744° ± 0.2°, and 26.261° ± 0.2°.

In one embodiment, the crystalline form of the ethanol solvate of the hydrochloride salt of the compound of formula I has an X-ray powder diffraction pattern having characteristic peaks at the following 2Θ angles: 6.24° ± 0.2°, 15.744° ± 0.2°, 18.391° ± 0.2°, and 26.261° ± 0.2°.

In one embodiment, the crystalline form of the ethanol solvate of the hydrochloride salt of the compound of formula I has an X-ray powder diffraction pattern having characteristic peaks at the following 2Θ angles: 6.24° ± 0.2°, 14.43° ± 0.2°, 15.744° ± 0.2°, 18.391° ± 0.2°, and 26.261° ± 0.2°.

In one embodiment, the crystalline form of the ethanol solvate of the hydrochloride salt of the compound of formula I has an X-ray powder diffraction pattern having characteristic peaks at the following 2Θ angles: 6.24° ± 0.2°, 14.43° ± 0.2°, 15.744° ± 0.2°, 18.391° ± 0.2°, 24.01° ± 0.2°, and 26.261° ± 0.2°.

In one embodiment, the crystalline form of the ethanol solvate of the hydrochloride salt of the compound of formula I has an X-ray powder diffraction pattern having characteristic peaks at the following 2Θ angles: 6.24° ± 0.2°, 6.673° ± 0.2°, 8.442° ± 0.2°, 13.706° ± 0.2°, 14.43° ± 0.2°, 15.744° ± 0.2°, 15.958° ± 0.2°, 17.924° ± 0.2°, 18.391° ± 0.2°, 18.937° ± 0.2°, 20.84° ± 0.2°, 21.458° ± 0.2°, 24.01° ± 0.2°, 24.708° ± 0.2°, 25.546° ± 0.2°, and 26.261° ± 0.2°.

In one embodiment, the crystalline form of the ethanol solvate of the hydrochloride salt of the compound of formula I has an X-ray powder diffraction pattern having characteristic peaks at the following 2Θ angles: 6.24° ± 0.2°, 6.673° ± 0.2°, 7.293° ± 0.2°, 8.442° ± 0.2°, 8.89° ± 0.2°, 10.326° ± 0.2°, 13.706° ± 0.2°, 14.43° ± 0.2°, 15.744° ± 0.2°, 15.958° ± 0.2°, 17.924° ± 0.2°, 18.391° ± 0.2°, 18.937° ± 0.2°, 19.246° ± 0.2°, 20.84° ± 0.2°, 21.458° ± 0.2°, 22.746° ± 0.2°, 24.01° ± 0.2°, 24.708° ± 0.2°, 25.546° ± 0.2°, 26.261° ± 0.2°, 27.08° ± 0.2°, 31.007° ± 0.2°, 31.298° ± 0.2°, 31.917° ± 0.2°, and 33.555° ± 0.2°.

In one embodiment, the crystalline form of the ethanol solvate of the hydrochloride salt of the compound of formula I has X-ray powder diffraction pattern analysis data substantially as shown in Table 12:

**Table 12. X-ray powder diffraction pattern analytical data for the crystalline form of the ethanol solvate of the hydrochloride salt of the compound of formula I**

| Position [°2θ] ± 0.2° | d-spacing [Å] | Relative intensity [%] |
|---|---|---|
| 6.24 | 14.1518 | 100 |
| 6.673 | 13.2348 | 67.1 |
| 7.293 | 12.1105 | 24.4 |
| 8.442 | 10.4658 | 60.4 |
| 8.89 | 9.9392 | 34.8 |
| 10.326 | 8.5597 | 34.2 |
| 13.706 | 6.4553 | 54.4 |
| 14.43 | 6.1332 | 75 |
| 15.744 | 5.6242 | 95.3 |
| 15.958 | 5.5491 | 55.4 |
| 17.924 | 4.9447 | 64.6 |
| 18.391 | 4.8201 | 81.3 |
| 18.937 | 4.6823 | 61.1 |
| 19.246 | 4.6079 | 48.7 |
| 20.84 | 4.2589 | 66.8 |
| 21.458 | 4.1377 | 62.7 |
| 22.746 | 3.9063 | 49.7 |
| 24.01 | 3.7034 | 73.7 |
| 24.708 | 3.6002 | 65.2 |
| 25.546 | 3.484 | 55.1 |
| 26.261 | 3.3908 | 85.8 |
| 27.08 | 3.2901 | 46.2 |
| 31.007 | 2.8817 | 41.1 |
| 31.298 | 2.8556 | 39.2 |
| 31.917 | 2.8016 | 32.6 |
| 33.555 | 2.6685 | 25.9 |

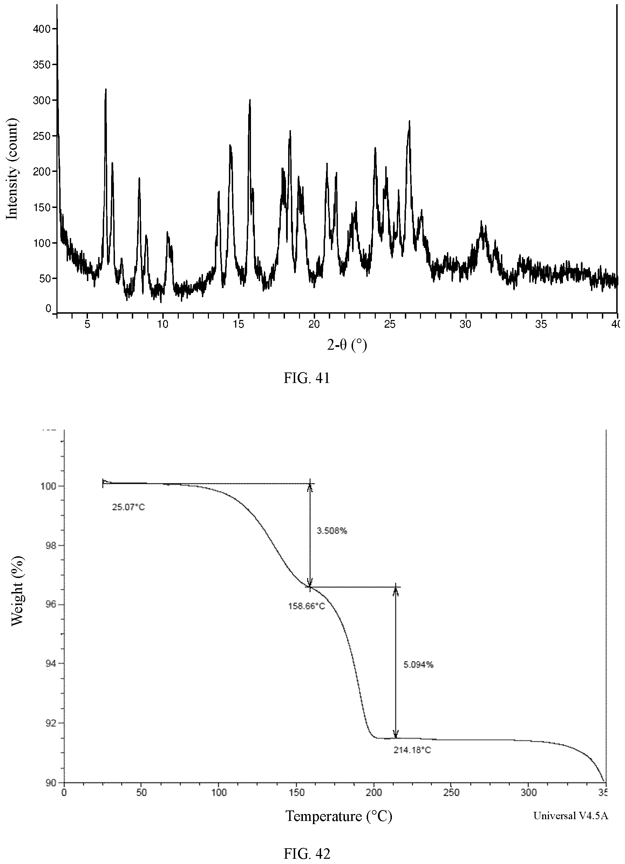

In one embodiment, the crystalline form of the ethanol solvate of the hydrochloride salt of the compound of formula I has an X-ray powder diffraction pattern substantially as shown in FIG. 41.

In one embodiment, the crystalline form of the ethanol solvate of the hydrochloride salt of the compound of formula I has a thermogravimetric analysis (TGA) curve showing a weight loss of 3.508% ± 0.5% during heating from 25.07 °C ± 3 °C to 158.66 °C ± 3 °C; and a weight loss of 5.094% ± 0.5% during heating from 158.66 °C ± 3 °C from 214.18 °C ± 3 °C.

In one embodiment, the crystalline form of the ethanol solvate of the hydrochloride salt of the compound of formula I has a thermogravimetric analysis curve showing a weight loss of 3.508% during heating from 25.07 °C to 158.66 °C; and a weight loss of 5.094% during heating from 158.66 °C to 214.18 °C.

In one embodiment, the crystalline form of the ethanol solvate of the hydrochloride salt of the compound of formula I has a thermogravimetric analysis curve substantially as shown in FIG. 42.

In one embodiment, the crystalline form of the ethanol solvate of the hydrochloride salt of the compound of formula I has a differential scanning calorimetry (DSC) curve having endothermic peaks with initial temperatures of 106 °C ± 3 °C and 189.22 °C ± 3 °C, respectively.

In one embodiment, the crystalline form of the ethanol solvate of the hydrochloride salt of the compound of formula I has a differential scanning calorimetry curve having endothermic peaks with initial temperatures of 106 °C and 189.22 °C, respectively.

In one embodiment, the crystalline form of the ethanol solvate of the hydrochloride salt of the compound of formula I has a differential scanning calorimetry curve having endothermic peaks with peak temperatures of 146.54 °C ± 3 °C and 205.32 °C ± 3 °C, respectively.

In one embodiment, the crystalline form of the ethanol solvate of the hydrochloride salt of the compound of formula I has a differential scanning calorimetry curve having endothermic peaks with peak temperatures of 146.54 °C and 205.32 °C, respectively.

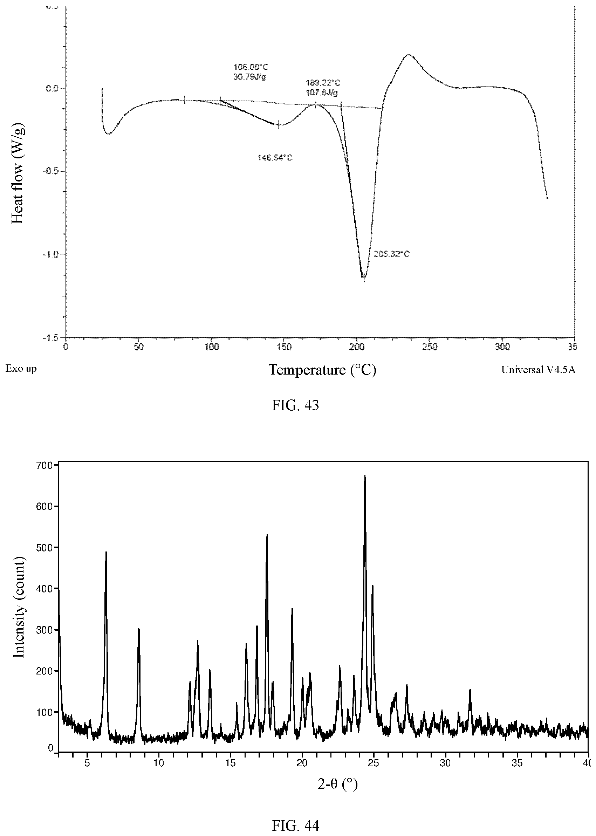

In one embodiment, the crystalline form of the ethanol solvate of the hydrochloride salt of the compound of formula I has a differential scanning calorimetry curve substantially as shown in FIG. 43.

The present invention further provides a preparation method for the crystalline form of the ethanol solvate of the hydrochloride salt of the compound of formula I, which preferably comprises the following steps: stirring a crystalline form A of a hydrochloride salt of a compound of formula I in ethanol to precipitate a solid, and separating and drying the solid to obtain the crystalline form of the ethanol solvate of the hydrochloride salt of the compound of formula I, wherein the stirring is preferably performed at a temperature of 25-45 °C; the stirring is preferably performed for a period of 2.5-4.5 days; and the crystalline form A of the hydrochloride salt of the compound of formula I and the ethanol are preferably in a mass-to-volume ratio of (50 mg-200 mg):1 mL.

In one embodiment, in the preparation method for the crystalline form of the ethanol solvate of the hydrochloride salt of the compound of formula I, the stirring is preferably performed at a temperature of 30 °C or 40 °C. The stirring is preferably performed for a period of 3 or 4 days. The crystalline form A of the ethanol solvate of the hydrochloride salt of the compound of formula I and the ethanol are preferably in a mass-to-volume ratio of 100 mg:1 mL or 150 mg:1 mL.

The present invention provides a crystalline form of an isopropanol solvate of a hydrochloride salt of a compound of formula I, which has an X-ray powder diffraction (XRPD) pattern having at least three, at least four, or at least five characteristic peaks at the following 2Θ angles: 6.301° ± 0.2°, 17.554° ± 0.2°, 19.305° ± 0.2°, 24.378° ± 0.2° and 24.903° ± 0.2°.

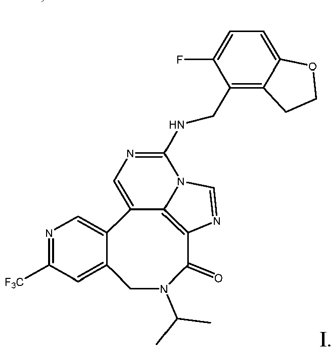

In one embodiment, the crystalline form of the isopropanol solvate of the hydrochloride salt of the compound of formula I has an X-ray powder diffraction pattern having characteristic peaks at the following 2Θ angles: 6.301° ± 0.2°, 17.554° ± 0.2°, and 24.378° ± 0.2°.

In one embodiment, the crystalline form of the isopropanol solvate of the hydrochloride salt of the compound of formula I has an X-ray powder diffraction pattern having characteristic peaks at the following 2Θ angles: 6.301° ± 0.2°, 17.554° ± 0.2°, 19.305° ± 0.2°, and 24.378° ± 0.2°.

In one embodiment, the crystalline form of the isopropanol solvate of the hydrochloride salt of the compound of formula I has an X-ray powder diffraction pattern having characteristic peaks at the following 2Θ angles: 6.301° ± 0.2°, 17.554° ± 0.2°, 19.305° ± 0.2°, 24.378° ± 0.2°, and 24.903° ± 0.2°.

In one embodiment, the crystalline form of the isopropanol solvate of the hydrochloride salt of the compound of formula I has an X-ray powder diffraction pattern having characteristic peaks at the following 2Θ angles: 6.301° ± 0.2°, 8.58° ± 0.2°, 12.172° ± 0.2°, 12.717° ± 0.2°, 13.536° ± 0.2°, 16.098° ± 0.2°, 16.837° ± 0.2°, 17.554° ± 0.2°, 17.964° ± 0.2°, 19.305° ± 0.2°, 20.007° ± 0.2°, 20.355° ± 0.2°, 20.513° ± 0.2°, 22.611° ± 0.2°, 23.606° ± 0.2°, 24.378° ± 0.2°, 24.903° ± 0.2°, 26.57° ± 0.2°, 27.293° ± 0.2°, and 31.706° ± 0.2°.

In one embodiment, the crystalline form of the isopropanol solvate of the hydrochloride salt of the compound of formula I has an X-ray powder diffraction pattern having characteristic peaks at the following 2Θ angles: 5.212° ± 0.2°, 6.301° ± 0.2°, 8.58° ± 0.2°, 12.172° ± 0.2°, 12.717° ± 0.2°, 13.536° ± 0.2°, 15.423° ± 0.2°, 16.098° ± 0.2°, 16.837° ± 0.2°, 17.554° ± 0.2°, 17.964° ± 0.2°, 18.737° ± 0.2°, 19.057° ± 0.2°, 19.305° ± 0.2°, 20.007° ± 0.2°, 20.355° ± 0.2°, 20.513° ± 0.2°, 22.437° ± 0.2°, 22.611° ± 0.2°, 23.191° ± 0.2°, 23.606° ± 0.2°, 24.378° ± 0.2°, 24.903° ± 0.2°, 26.323° ± 0.2°, 26.57° ± 0.2°, 27.293° ± 0.2°, 27.665° ± 0.2°, 28.499° ± 0.2°, 29.16° ± 0.2°, 29.739° ± 0.2°, 30.889° ± 0.2°, 31.706° ± 0.2°, 32.957° ± 0.2°, 33.603° ± 0.2°, 34.927° ± 0.2°, 37.052° ± 0.2°, 37.901° ± 0.2°, and 38.625° ± 0.2°.

In one embodiment, the crystalline form of the isopropanol solvate of the hydrochloride salt of the compound of formula I has X-ray powder diffraction pattern analysis data substantially as shown in Table 13:

**Table 13. X-ray powder diffraction pattern analytical data for the crystalline form of the isopropanol solvate of the hydrochloride salt of the compound of formula I**

| Position [°2θ] ± 0.2° | d-spacing [Å] | Relative intensity [%] |
|---|---|---|
| 5.212 | 16.9422 | 11.8 |
| 6.301 | 14.0148 | 72.3 |
| 8.58 | 10.2975 | 44.7 |
| 12.172 | 7.2655 | 25.6 |
| 12.717 | 6.9554 | 40.4 |
| 13.536 | 6.5362 | 29.7 |
| 15.423 | 5.7403 | 17.9 |
| 16.098 | 5.5012 | 39.2 |
| 16.837 | 5.2614 | 45.6 |
| 17.554 | 5.0481 | 78.7 |
| 17.964 | 4.9338 | 25.6 |
| 18.737 | 4.7319 | 10.9 |
| 19.057 | 4.6533 | 12.7 |
| 19.305 | 4.5939 | 51.8 |
| 20.007 | 4.4343 | 27.1 |
| 20.355 | 4.3593 | 22.9 |
| 20.513 | 4.326 | 28.7 |
| 22.437 | 3.9593 | 18.8 |
| 22.611 | 3.9291 | 31.4 |
| 23.191 | 3.8322 | 15.1 |
| 23.606 | 3.7658 | 27.7 |
| 24.378 | 3.6482 | 100 |
| 24.903 | 3.5725 | 60.2 |
| 26.323 | 3.383 | 18.2 |
| 26.57 | 3.352 | 21.4 |
| 27.293 | 3.2648 | 24.4 |
| 27.665 | 3.2218 | 13.9 |
| 28.499 | 3.1293 | 14.6 |
| 29.16 | 3.0599 | 14.3 |
| 29.739 | 3.0016 | 15.2 |
| 30.889 | 2.8925 | 14.5 |
| 31.706 | 2.8197 | 22.6 |
| 32.957 | 2.7156 | 14.2 |
| 33.603 | 2.6648 | 11.8 |
| 34.927 | 2.5668 | 11.5 |
| 37.052 | 2.4243 | 12.3 |
| 37.901 | 2.3719 | 10.2 |
| 38.625 | 2.3291 | 10.4 |

In one embodiment, the crystalline form of the isopropanol solvate of the hydrochloride salt of the compound of formula I has an X-ray powder diffraction pattern substantially as shown in FIG. 44.

In one embodiment, the crystalline form of the isopropanol solvate of the hydrochloride salt of the compound of formula I has a thermogravimetric analysis (TGA) curve showing a weight loss of 14.79% ± 0.5% during heating from 28.2 °C ± 3 °C to 205.15 °C ± 3 °C.

In one embodiment, the crystalline form of the isopropanol solvate of the hydrochloride salt of the compound of formula I has a thermogravimetric analysis curve showing a weight loss of 14.79% during heating from 28.2 °C to 205.15 °C.

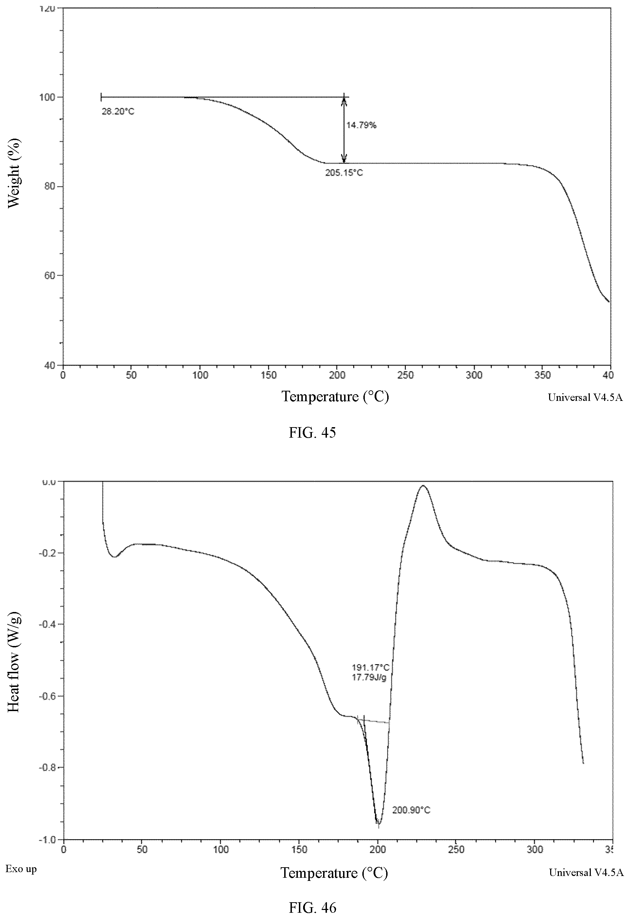

In one embodiment, the crystalline form of the isopropanol solvate of the hydrochloride salt of the compound of formula I has a thermogravimetric analysis curve substantially as shown in FIG. 45.

In one embodiment, the crystalline form of the isopropanol solvate of the hydrochloride salt of the compound of formula I has a differential scanning calorimetry (DSC) curve having an endothermic peak with an initial temperature of 191.17 ± 3 °C, respectively.

In one embodiment, the crystalline form of the isopropanol solvate of the hydrochloride salt of the compound of formula I has a differential scanning calorimetry curve having an endothermic peak with an initial temperature of 191.17 °C.

In one embodiment, the crystalline form of the isopropanol solvate of the hydrochloride salt of the compound of formula I has a differential scanning calorimetry curve having an endothermic peak with a peak temperature of 200.9 °C ± 3 °C.

In one embodiment, the crystalline form of the isopropanol solvate of the hydrochloride salt of the compound of formula I has a differential scanning calorimetry curve having an endothermic peak with a peak temperature of 200.9 °C.

In one embodiment, the crystalline form of the isopropanol solvate of the hydrochloride salt of the compound of formula I has a differential scanning calorimetry curve substantially as shown in FIG. 46.

The present invention further provides a preparation method for the crystalline form of the isopropanol solvate of the hydrochloride salt of the compound of formula I, which preferably comprises the following steps: stirring a crystalline form A of a hydrochloride salt of a compound of formula I in isopropanol to precipitate a solid, and separating and drying the solid to obtain the crystalline form of the isopropanol solvate of the hydrochloride salt of the compound of formula I, wherein the stirring is preferably performed at a temperature of 25-45 °C; the stirring is preferably performed for a period of 2.5-4.5 days; and the crystalline form A of the hydrochloride salt of the compound of formula I and the isopropanol are preferably in a mass-to-volume ratio of (100 mg-200 mg):1 mL.

In one embodiment, in the preparation method for the crystalline form of the isopropanol solvate of the hydrochloride salt of the compound of formula I, the stirring is preferably performed at a temperature of 30°C or 40 °C. The stirring is preferably performed for a period of 3 or 4 days. The crystalline form A of the isopropanol solvate of the hydrochloride salt of the compound of formula I and the ethanol are preferably in a mass-to-volume ratio of 133.33 mg: 1 mL or 150 mg: 1 mL.

The present invention provides a crystalline form A of a sulfate salt of a compound of formula I, which has an X-ray powder diffraction (XRPD) pattern having at least three characteristic peaks at the following 2θ angles: 7.645° ± 0.2°, 15.146° ± 0.2°, and 17.17° ± 0.2°;

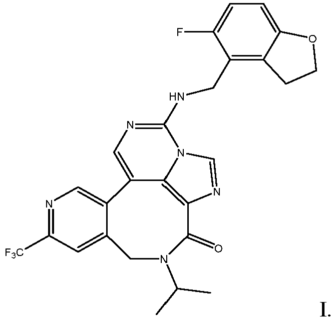

In one embodiment, the crystalline form A of the sulfate salt of the compound of formula I has an X-ray powder diffraction pattern having characteristic peaks at the following 2θ angles: 5.468° ± 0.2°, 7.645° ± 0.2°, 15.146° ± 0.2°, 17.17° ± 0.2°, 19.25° ± 0.2°, 19.813° ± 0.2°, and 24.106° ± 0.2°.

In one embodiment, the crystalline form A of the sulfate salt of the compound of formula I has an X-ray powder diffraction pattern having characteristic peaks at the following 2θ angles: 5.119° ± 0.2°, 5.468° ± 0.2°, 7.645° ± 0.2°, 8.424° ± 0.2°, 11.303° ± 0.2°, 15.146° ± 0.2°, 16.289° ± 0.2°, 16.701° ± 0.2°, 17.17° ± 0.2°, 18.176° ± 0.2°, 19.25° ± 0.2°, 19.813° ± 0.2°, 20.826° ± 0.2°, 21.138° ± 0.2°, 21.522° ± 0.2°, 22.476° ± 0.2°, 23.152° ± 0.2°, 24.106° ± 0.2°, 24.456° ± 0.2°, 25.351° ± 0.2°, 26.613° ± 0.2°, 27.41° ± 0.2°, 27.816° ± 0.2°, 28.324° ± 0.2°, and 29.084° ± 0.2°.

In one embodiment, the crystalline form A of the sulfate salt of the compound of formula I has X-ray powder diffraction pattern analysis data substantially as shown in Table 14:

**Table 14. X-ray powder diffraction pattern analysis data for the crystalline form A of the sulfate salt of the compound of formula I**

| Position [°2θ] ± 0.2° | d-spacing [Å] | Relative intensity [%] |
|---|---|---|
| 5.119 | 17.2503 | 5.8 |
| 5.468 | 16.1496 | 11.7 |
| 7.645 | 11.5541 | 100 |
| 8.424 | 10.4877 | 9.2 |
| 11.303 | 7.8219 | 2.9 |
| 15.146 | 5.845 | 46.3 |
| 16.289 | 5.437 | 9.5 |
| 16.701 | 5.3039 | 7 |
| 17.17 | 5.1602 | 17.8 |
| 18.176 | 4.8767 | 3.9 |
| 19.25 | 4.6069 | 15.7 |
| 19.813 | 4.4774 | 10.4 |
| 20.826 | 4.2617 | 5.1 |
| 21.138 | 4.1995 | 4.7 |
| 21.522 | 4.1255 | 5.1 |
| 22.476 | 3.9525 | 8.4 |
| 23.152 | 3.8385 | 6.8 |
| 24.106 | 3.6888 | 10.8 |
| 24.456 | 3.6368 | 8.9 |
| 25.351 | 3.5104 | 9.3 |
| 26.613 | 3.3467 | 4 |
| 27.41 | 3.2512 | 7.4 |
| 27.816 | 3.2047 | 6.2 |
| 28.324 | 3.1484 | 4.7 |
| 29.084 | 3.0677 | 3.9 |

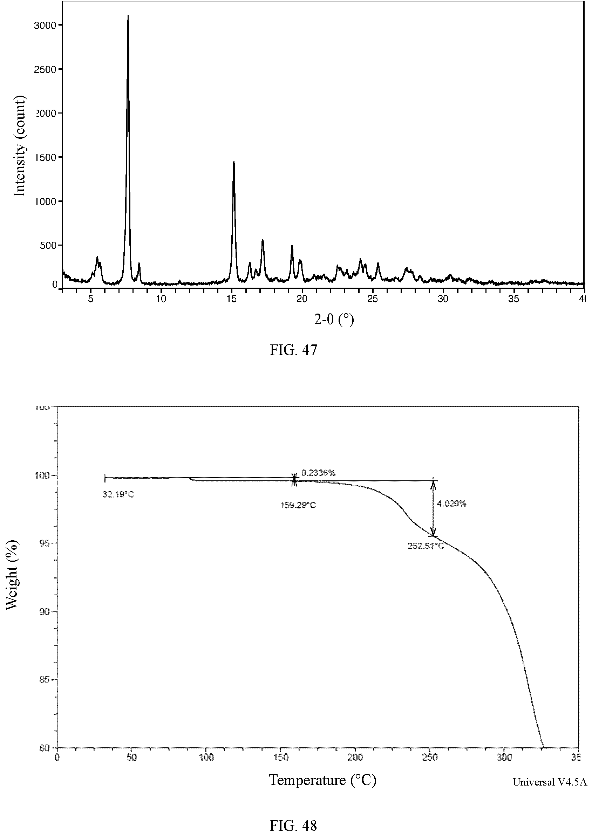

In one embodiment, the crystalline form A of the sulfate salt of the compound of formula I has an X-ray powder diffraction pattern substantially as shown in FIG. 47.

In one embodiment, the crystalline form A of the sulfate salt of the compound of formula I has a thermogravimetric analysis (TGA) curve showing a weight loss of 0.2336% ± 0.2% during heating from 32.19 °C ± 3 °C to 159.29 °C ± 3 °C; and a weight loss of 4.029 % ± 0.5% during heating from 159.29 °C ± 3 °C to 252.51 °C ± 3 °C.

In one embodiment, the crystalline form A of the sulfate salt of the compound of formula I has a thermogravimetric analysis curve showing a weight loss of 0.2336% during heating from 32.19 °C to 159.29 °C; and a weight loss of 4.029% during heating from 159.29 °C to 252.51 °C.

In one embodiment, the crystalline form A of the sulfate salt of the compound of formula I has a thermogravimetric analysis curve substantially as shown in FIG. 48.

In one embodiment, the crystalline form A of the sulfate salt of the compound of formula I has a differential scanning calorimetry (DSC) curve having an endothermic peak with an initial temperature of 210.46 °C ± 3°C.

In one embodiment, the crystalline form A of the sulfate salt of the compound of formula I has a differential scanning calorimetry curve having an endothermic peak with an initial temperature of 210.46 °C.

In one embodiment, the crystalline form A of the sulfate salt of the compound of formula I has a differential scanning calorimetry curve having an endothermic peak with a peak temperature of 224.37 °C ± 3 °C.

In one embodiment, the crystalline form A of the sulfate salt of the compound of formula I has a differential scanning calorimetry curve having an endothermic peak with a peak temperature of 224.37 °C.

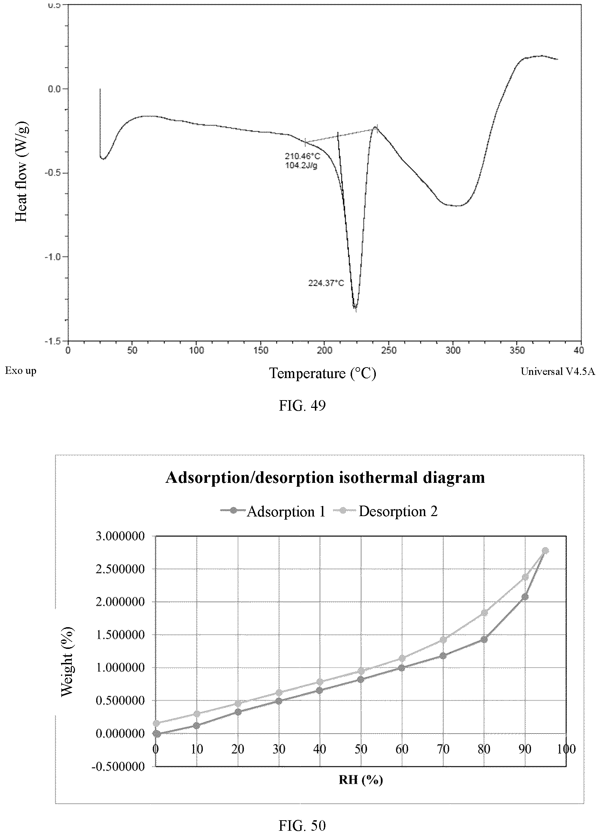

In one embodiment, the crystalline form A of the sulfate salt of the compound of formula I has a differential scanning calorimetry curve substantially as shown in FIG. 49.

In one embodiment, the crystalline form A of the sulfate salt of the compound of formula I has a dynamic vapor sorption (DVS) curve showing a hygroscopic weight gain of 1.43% ± 0.5% at 25 °C and 80% RH.

In one embodiment, the crystalline form A of the sulfate salt of the compound of formula I has a dynamic vapor sorption curve showing a hygroscopic weight gain of 1.43% at 25 °C and 80% RH.

In one embodiment, the crystalline form A of the sulfate salt of the compound of formula I has a dynamic vapor sorption curve as shown in FIG. 50.

The present invention further provides a preparation method for the crystalline form A of the sulfate salt of the compound of formula I, which preferably comprises the following steps: mixing and stirring a compound of formula I, sulfuric acid, and tetrahydrofuran, adding n-heptane to precipitate a solid, and separating and drying the solid to obtain the crystalline form A of the sulfate salt of the compound of formula I, wherein the stirring is preferably performed at a temperature of 20-30 °C; the stirring is preferably performed for a period of 0.2-0.8 days; the compound of formula I and the sulfuric acid are preferably in a molar ratio of (1-1.5):1; the compound of formula I and the tetrahydrofuran are preferably in a mass-to-volume ratio of (40 mg-50 mg):1 mL; and the compound of formula I and the n-heptane are in a mass-to-volume ratio of (70 mg-90 mg):1 mL. In one embodiment, in the preparation method for the crystalline form A of the sulfate salt of the compound of formula I, the compound of formula I is preferably a crystalline form A of a compound of formula I. The stirring is preferably performed at a temperature of 25 °C. The stirring is preferably performed for a period of 0.5 day; and the compound of formula I and the sulfuric acid are preferably in a molar ratio of 1.1:1. The compound of formula I and the tetrahydrofuran are preferably in a mass-to-volume ratio of 44.71 mg: 1 mL. The compound of formula I and the n-heptane are preferably in a mass-to-volume ratio of 82.33 mg: 1 mL. The present invention provides a crystalline form B of a sulfate salt of a compound of formula I, which has an X-ray powder diffraction (XRPD) pattern having at least three or at least four characteristic peaks at the following 2Θ angles: 5.896° ± 0.2°, 17.498° ± 0.2°, 20.314° ± 0.2°, and 22.806° ± 0.2°;

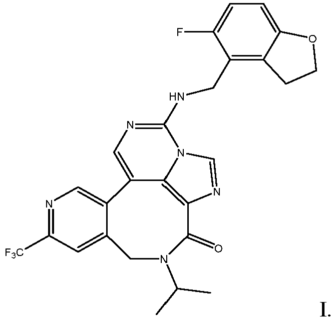

In one embodiment, the crystalline form B of the sulfate salt of the compound of formula I has an X-ray powder diffraction pattern having characteristic peaks at the following 2Θ angles: 5.896° ± 0.2°, 17.498° ± 0.2°, and 20.314° ± 0.2°.

In one embodiment, the crystalline form B of the sulfate salt of the compound of formula I has an X-ray powder diffraction pattern having characteristic peaks at the following 2Θ angles: 5.896° ± 0.2°, 17.498° ± 0.2°, 20.314° ± 0.2°, and 22.806° ± 0.2°.

In one embodiment, the crystalline form B of the sulfate salt of the compound of formula I has an X-ray powder diffraction pattern having characteristic peaks at the following 2θ angles: 5.896° ± 0.2°, 7.174° ± 0.2°, 16.585° ± 0.2°, 17.498° ± 0.2°, 18.294° ± 0.2°, 20.314° ± 0.2°, and 22.806° ± 0.2°.

In one embodiment, the crystalline form B of the sulfate salt of the compound of formula I has an X-ray powder diffraction pattern having characteristic peaks at the following 2θ angles: 5.896° ± 0.2°, 7.174° ± 0.2°, 7.589° ± 0.2°, 11.686° ± 0.2°, 12.54° ± 0.2°, 13.282° ± 0.2°, 14.199° ± 0.2°, 15.517° ± 0.2°, 15.773° ± 0.2°, 16.205° ± 0.2°, 16.585° ± 0.2°, 17.498° ± 0.2°, 18.294° ± 0.2°, 19.283° ± 0.2°, 19.598° ± 0.2°, 20.314° ± 0.2°, 21.271° ± 0.2°, 21.558° ± 0.2°, 22.105° ± 0.2°, 22.806° ± 0.2°, 23.351° ± 0.2°, 24.05° ± 0.2°, 25.135° ± 0.2°, 25.368° ± 0.2°, 25.892° ± 0.2°, 26.475° ± 0.2°, 26.764° ± 0.2°, 27.837° ± 0.2°, 28.069° ± 0.2°, 29.259° ± 0.2°, 29.956° ± 0.2°, 30.399° ± 0.2°, 30.807° ± 0.2°, 31.687° ± 0.2°, 31.874° ± 0.2°, 32.464° ± 0.2°, 33.591° ± 0.2°, 35.259° ± 0.2°, 36.912° ± 0.2°, 37.375° ± 0.2°, and 38.703° ± 0.2°.

In one embodiment, the crystalline form B of the sulfate salt of the compound of formula I has X-ray powder diffraction pattern analysis data substantially as shown in Table 15:

**Table 15. X-ray powder diffraction pattern analysis data for the crystalline form B of the sulfate salt of the compound of formula I**

| Position [°2θ] ± 0.2° | d-spacing [Å] | Relative intensity [%] |
|---|---|---|
| 5.896 | 14.9771 | 100 |
| 7.174 | 12.3124 | 11.5 |
| 7.589 | 11.6402 | 5.8 |
| 11.686 | 7.5661 | 6 |
| 12.54 | 7.053 | 2.9 |
| 13.282 | 6.6606 | 2.9 |
| 14.199 | 6.2324 | 5.2 |
| 15.517 | 5.706 | 5.6 |
| 15.773 | 5.6138 | 4.8 |
| 16.205 | 5.4651 | 4.3 |
| 16.585 | 5.3406 | 10.4 |
| 17.498 | 5.0641 | 24 |
| 18.294 | 4.8455 | 11.2 |
| 19.283 | 4.5992 | 6.9 |
| 19.598 | 4.526 | 7.5 |
| 20.317 | 4.3674 | 15.2 |
| 21.271 | 4.1736 | 4.5 |
| 21.558 | 4.1186 | 5.2 |
| 22.105 | 4.018 | 4.7 |
| 22.806 | 3.8961 | 15 |
| 23.351 | 3.8064 | 5.6 |
| 24.05 | 3.6973 | 6.4 |
| 25.135 | 3.5401 | 9.3 |
| 25.368 | 3.5081 | 5.9 |
| 25.892 | 3.4382 | 5.7 |
| 26.475 | 3.3638 | 7.5 |
| 26.764 | 3.3282 | 4.7 |
| 27.837 | 3.2022 | 5.3 |
| 28.069 | 3.1763 | 4.8 |
| 29.259 | 3.0498 | 4.1 |
| 29.956 | 2.9804 | 4.3 |
| 30.399 | 2.938 | 5.4 |
| 30.807 | 2.9 | 3.6 |
| 31.687 | 2.8214 | 3.4 |
| 31.874 | 2.8053 | 3.2 |
| 32.464 | 2.7557 | 3.5 |
| 33.591 | 2.6657 | 3.5 |
| 35.259 | 2.5433 | 3.2 |
| 36.912 | 2.4331 | 3 |
| 37.375 | 2.4041 | 2.7 |
| 38.703 | 2.3246 | 3 |

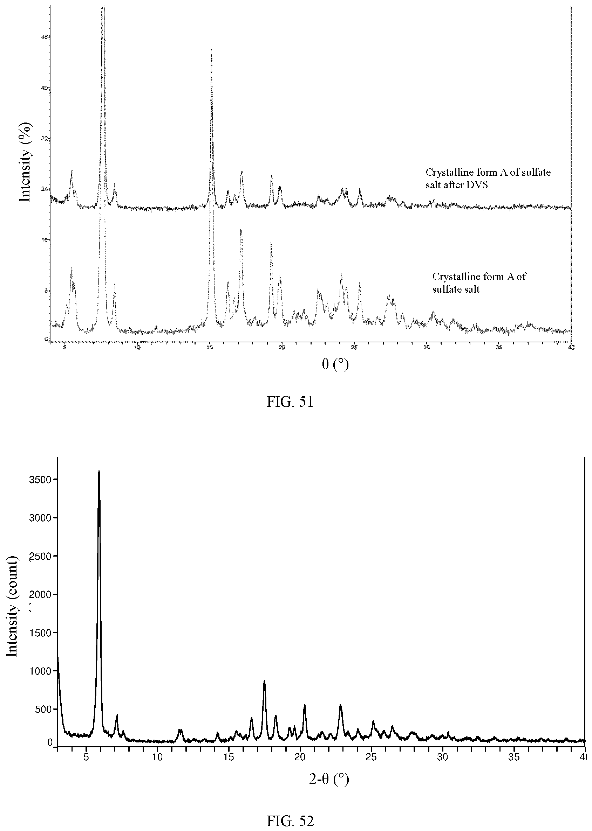

In one embodiment, the crystalline form B of the sulfate salt of the compound of formula I has an X-ray powder diffraction pattern substantially as shown in FIG. 52.

In one embodiment, the crystalline form B of the sulfate salt of the compound of formula I has a thermogravimetric analysis (TGA) curve showing a weight loss of 2.144% ± 0.5% during heating from 33.57 °C ± 3 °C to 120.18 °C ± 3 °C; and a weight loss of 6.564% ± 0.5% during heating from 120.18 °C ± 3 °C to 209.36 °C ± 3 °C.

In one embodiment, the crystalline form B of the sulfate salt of the compound of formula I has a thermogravimetric analysis curve showing a weight loss of 2.144% during heating from 33.57 °C to 120.18 °C; and a weight loss of 6.564% during heating from 120.18 °C to 209.36 °C.

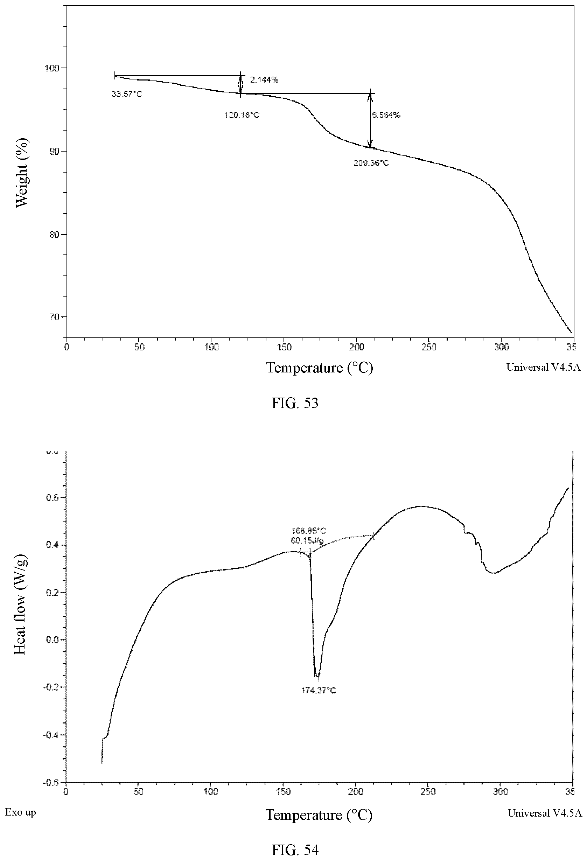

In one embodiment, the crystalline form B of the sulfate salt of the compound of formula I has a thermogravimetric analysis curve substantially as shown in FIG. 53.

In one embodiment, the crystalline form B of the sulfate salt of the compound of formula I has a differential scanning calorimetry (DSC) curve having an endothermic peak with an initial temperature of 168.85 °C ± 3 °C.

In one embodiment, the crystalline form B of the sulfate salt of the compound of formula I has a differential scanning calorimetry curve having an endothermic peak with an initial temperature of 168.85 °C.

In one embodiment, the crystalline form B of the sulfate salt of the compound of formula I has a differential scanning calorimetry curve having an endothermic peak with a peak temperature of 174.37 °C ± 3 °C.

In one embodiment, the crystalline form B of the sulfate salt of the compound of formula I has a differential scanning calorimetry curve having an endothermic peak with a peak temperature of 174.37 °C.

In one embodiment, the crystalline form B of the sulfate salt of the compound of formula I has a differential scanning calorimetry curve substantially as shown in FIG. 54.

The present invention further provides a preparation method for the crystalline form B of the sulfate salt of the compound of formula I, which preferably comprises the following steps: mixing and stirring a compound of formula I, sulfuric acid, and tetrahydrofuran to precipitate a solid, and separating and drying the solid to obtain the crystalline form B of the sulfate salt of the compound of formula I, wherein the stirring is performed at a temperature of 20-30 °C; the stirring is preferably performed for a period of 3-5 h; the compound of formula I and the sulfuric acid are in a molar ratio of (1-1.5): 1; and the compound of formula I and the tetrahydrofuran are in a mass-to-volume ratio of (30 mg-70 mg):1 mL.

In one embodiment, in the preparation method for the crystalline form B of the sulfate salt of the compound of formula I, the compound of formula I is preferably a crystalline form A of a compound of formula I. The stirring is preferably performed at a temperature of 25 °C. The stirring is preferably performed for a period of 4 h. The compound of the formula I and the sulfuric acid are preferably in a molar ratio of 1.2:1. The compound of formula I and the tetrahydrofuran are preferably in a mass-to-volume ratio of 43.14 mg: 1 mL.

The present invention provides a crystalline form C of a sulfate salt of a compound of formula I, which has an X-ray powder diffraction (XRPD) pattern having at least three characteristic peaks at the following 2θ angles: 5.818° ± 0.2°, 6.576° ± 0.2°, and 15.904° ± 0.2°;

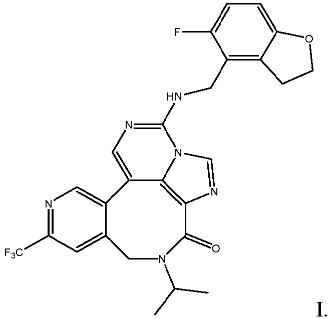

In one embodiment, the crystalline form C of the sulfate salt of the compound of formula I has an X-ray powder diffraction pattern having characteristic peaks at the following 2θ angles: 5.818° ± 0.2°, 6.576° ± 0.2°, 15.904° ± 0.2°, 16.292° ± 0.2°, and 22.994° ± 0.2°.

In one embodiment, the crystalline form C of the sulfate salt of the compound of formula I has an X-ray powder diffraction pattern having characteristic peaks at the following 2Θ angles: 5.818° ± 0.2°, 6.576° ± 0.2°, 8.996° ± 0.2°, 9.976° ± 0.2°, 10.578° ± 0.2°, 11.55° ± 0.2°, 15.239° ± 0.2°, 15.904° ± 0.2°, 16.292° ± 0.2°, 16.55° ± 0.2°, 17.599° ± 0.2°, 18.818° ± 0.2°, 20.641° ± 0.2°, 21.538° ± 0.2°, 22.14° ± 0.2°, 22.994° ± 0.2°, 23.815° ± 0.2°, 24.237° ± 0.2°, 24.468° ± 0.2°, 26.105° ± 0.2°, 29.566° ± 0.2°, and 30.089° ± 0.2°.

In one embodiment, the crystalline form C of the sulfate salt of the compound of formula I has X-ray powder diffraction pattern analysis data substantially as shown in Table 16:

**Table 16. X-ray powder diffraction pattern analysis data for the crystalline form C of the sulfate salt of the compound of formula I**

| Position [°2θ] ± 0.2° | d-spacing [Å] | Relative intensity [%] |
|---|---|---|
| 5.818 | 15.1778 | 69.3 |
| 6.576 | 13.4306 | 100 |
| 8.996 | 9.8224 | 3.7 |
| 9.976 | 8.8591 | 6.9 |
| 10.578 | 8.3563 | 4.3 |
| 11.55 | 7.6555 | 5.9 |
| 15.239 | 5.8093 | 5.3 |
| 15.904 | 5.5677 | 21.9 |
| 16.292 | 5.4362 | 10.4 |
| 16.55 | 5.3519 | 9 |
| 17.599 | 5.0351 | 6.1 |
| 18.818 | 4.7117 | 8.3 |
| 20.641 | 4.2996 | 5.8 |
| 21.538 | 4.1224 | 7 |
| 22.14 | 4.0118 | 7.6 |
| 22.994 | 3.8646 | 17.1 |
| 23.815 | 3.7332 | 6.8 |
| 24.237 | 3.6692 | 7.9 |
| 24.468 | 3.635 | 7.4 |
| 26.105 | 3.4107 | 4.9 |
| 29.566 | 3.0188 | 5.9 |
| 30.089 | 2.9675 | 4.7 |

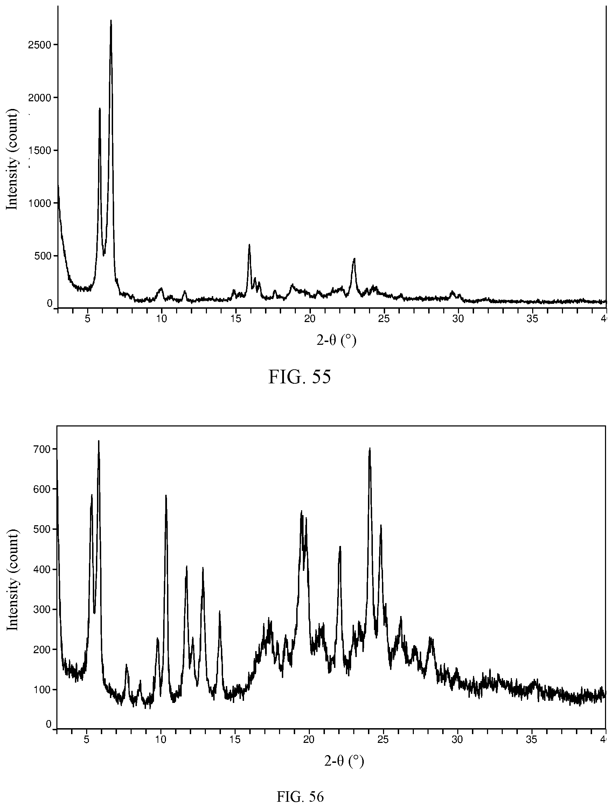

In one embodiment, the crystalline form C of the sulfate salt of the compound of formula I has an X-ray powder diffraction pattern substantially as shown in FIG. 55.

The present invention further provides a preparation method for the crystalline form C of the sulfate salt of the compound of formula I, which preferably comprises the following steps: mixing and stirring a compound of formula I, tetrahydrofuran, and sulfuric acid, adding n-heptane to precipitate a solid, and separating and drying the solid to obtain the crystalline form C of the sulfate salt of the compound of formula I, wherein the stirring is performed at a temperature of 20-35 °C; the stirring is preferably performed for a period of 3-5 h; the compound of formula I and the sulfuric acid are in a molar ratio of (1-1.5):1; the compound of formula I and the tetrahydrofuran are in a mass-to-volume ratio of (40 mg-50 mg):1 mL; and the compound of formula I and the n-heptane are in a mass-to-volume ratio of (15 mg-35 mg):1 mL.

In one embodiment, in the preparation method for the crystalline form C of the sulfate salt of the compound of formula I, the compound of formula I is preferably a crystalline form A of a compound of formula I. The stirring is preferably performed at a temperature of 25 °C. The stirring is preferably performed for a period of 4 h. The compound of the formula I and the sulfuric acid are preferably in a molar ratio of 1.1:1. The compound of formula I and the tetrahydrofuran are preferably in a mass-to-volume ratio of 45.45 mg: 1 mL. The compound of formula I and the n-heptane are preferably in a mass-to-volume ratio of 27.32 mg:1 mL.

The present invention provides a crystalline form of a toluene solvate of a sulfate salt of a compound of formula I, which has an X-ray powder diffraction (XRPD) pattern having at least three, at least four, at least five, at least six, or at least seven characteristic peaks at the following 2θ angles: 5.351° ± 0.2°, 5.819° ± 0.2°, 10.347° ± 0.2°, 19.48° ± 0.2°, 19.792° ± 0.2°, 24.086° ± 0.2°, and 24.826° ± 0.2°;

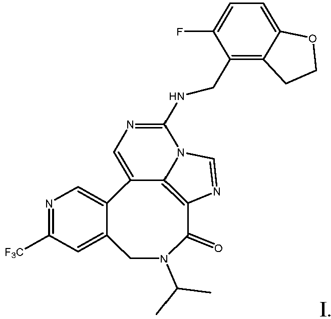

In one embodiment, the crystalline form of the toluene solvate of the sulfate salt of the compound of formula I has an X-ray powder diffraction pattern having characteristic peaks at the following 2Θ angles: 5.351° ± 0.2°, 5.819° ± 0.2°, and 24.086° ± 0.2°.

In one embodiment, the crystalline form of the toluene solvate of the sulfate salt of the compound of formula I has an X-ray powder diffraction pattern having characteristic peaks at the following 2Θ angles: 5.351° ± 0.2°, 5.819° ± 0.2°, 10.347° ± 0.2°, and 24.086° ± 0.2°.

In one embodiment, the crystalline form of the toluene solvate of the sulfate salt of the compound of formula I has an X-ray powder diffraction pattern having characteristic peaks at the following 2Θ angles: 5.351° ± 0.2°, 5.819° ± 0.2°, 10.347° ± 0.2°, 19.48° ± 0.2°, and 24.086° ± 0.2°.

In one embodiment, the crystalline form of the toluene solvate of the sulfate salt of the compound of formula I has an X-ray powder diffraction pattern having characteristic peaks at the following 2θ angles: 5.351° ± 0.2°, 5.819° ± 0.2°, 10.347° ± 0.2°, 19.48° ± 0.2°, 19.792° ± 0.2°, and 24.086° ± 0.2°.

In one embodiment, the crystalline form of the toluene solvate of the sulfate salt of the compound of formula I has an X-ray powder diffraction pattern having characteristic peaks at the following 2θ angles: 5.351° ± 0.2°, 5.819° ± 0.2°, 10.347° ± 0.2°, 19.48° ± 0.2°, 19.792° ± 0.2°, 24.086° ± 0.2°, and 24.826° ± 0.2°.

In one embodiment, the crystalline form of the toluene solvate of the sulfate salt of the compound of formula I has an X-ray powder diffraction pattern having characteristic peaks at the following 2θ angles: 5.351° ± 0.2°, 5.819° ± 0.2°, 9.765° ± 0.2°, 10.347° ± 0.2°, 11.744° ± 0.2°, 12.154° ± 0.2°, 12.834° ± 0.2°, 13.962° ± 0.2°, 16.933° ± 0.2°, 17.439° ± 0.2°, 18.407° ± 0.2°, 19.48° ± 0.2°, 19.792° ± 0.2°, 20.941° ± 0.2°, 22.066° ± 0.2°, 22.966° ± 0.2°, 23.38° ± 0.2°, 24.086° ± 0.2°, 24.826° ± 0.2°, 25.154° ± 0.2°, 26.167° ± 0.2°, and 28.344° ± 0.2°.

In one embodiment, the crystalline form of the toluene solvate of the sulfate salt of the compound of formula I has an X-ray powder diffraction pattern having characteristic peaks at the following 2θ angles: 5.351° ± 0.2°, 5.819° ± 0.2°, 7.684° ± 0.2°, 8.615° ± 0.2°, 9.765° ± 0.2°, 10.347° ± 0.2°, 11.744° ± 0.2°, 12.154° ± 0.2°, 12.834° ± 0.2°, 13.962° ± 0.2°, 16.414° ± 0.2°, 16.933° ± 0.2°, 17.439° ± 0.2°, 18.407° ± 0.2°, 19.48° ± 0.2°, 19.792° ± 0.2°, 20.941° ± 0.2°, 22.066° ± 0.2°, 22.966° ± 0.2°, 23.38° ± 0.2°, 24.086° ± 0.2°, 24.826° ± 0.2°, 25.154° ± 0.2°, 26.167° ± 0.2°, 27.172° ± 0.2°, 28.344° ± 0.2°, 29.901° ± 0.2°, and 32.168° ± 0.2°.

In one embodiment, the crystalline form of the toluene solvate of the sulfate salt of the compound of formula I has X-ray powder diffraction pattern analysis data substantially as shown in Table 17:

**Table 17. X-ray powder diffraction pattern analysis data for the crystalline form of the toluene solvate of the sulfate salt of the compound of formula I**

| Position [°2θ] ± 0.2° | d-spacing [Å] | Relative intensity [%] |
|---|---|---|
| 5.351 | 16.5021 | 81.3 |
| 5.819 | 15.1763 | 100 |
| 7.684 | 11.4953 | 22.3 |
| 8.615 | 10.2556 | 16.8 |
| 9.765 | 9.0504 | 31.4 |
| 10.347 | 8.542 | 81 |
| 11.744 | 7.5289 | 56.5 |
| 12.154 | 7.2763 | 31.7 |
| 12.834 | 6.892 | 56 |
| 13.962 | 6.3375 | 40.9 |
| 16.414 | 5.396 | 26.7 |
| 16.933 | 5.2317 | 36.4 |
| 17.439 | 5.081 | 37.4 |
| 18.407 | 4.816 | 32.5 |
| 19.48 | 4.5532 | 75.6 |
| 19.792 | 4.4821 | 73.3 |
| 20.941 | 4.2386 | 36.7 |
| 22.066 | 4.025 | 63.3 |
| 22.966 | 3.8693 | 35.9 |
| 23.38 | 3.8016 | 37 |
| 24.086 | 3.6918 | 97.5 |
| 24.826 | 3.5835 | 70.6 |
| 25.154 | 3.5374 | 43.5 |
| 26.167 | 3.4028 | 38.9 |
| 27.172 | 3.2791 | 28.4 |
| 28.344 | 3.1462 | 31 |
| 29.901 | 2.9858 | 21.1 |
| 32.168 | 2.7803 | 20.6 |

In one embodiment, the crystalline form of the toluene solvate of the sulfate salt of the compound of formula I has an X-ray powder diffraction pattern substantially as shown in FIG. 56.

In one embodiment, the crystalline form of the toluene solvate of the sulfate salt of the compound of formula I has a thermogravimetric analysis (TGA) curve of showing a weight loss of 0.5679% ± 0.2% during heating from 34.88 °C ± 3 °C to 109.33 °C ± 3 °C; a weight loss of 3.645% ± 0.5% during heating from 109.33 °C ± 3 °C to 192.37 °C ± 3 °C; and a weight loss of 1.535% ± 0.5% during heating from 192.37 °C ± 3 °C to 222.48 °C ± 3 °C.

In one embodiment, the crystalline form of the toluene solvate of the sulfate salt of the compound of formula I has a thermogravimetric analysis curve showing a weight loss of 0.5679% during heating from 34.88 °C to 109.33 °C; a weight loss of 3.645% during heating from 109.33 °C to 192.37 °C; and a weight loss of 1.535% during heating from 192.37 °C to 222.48 °C.

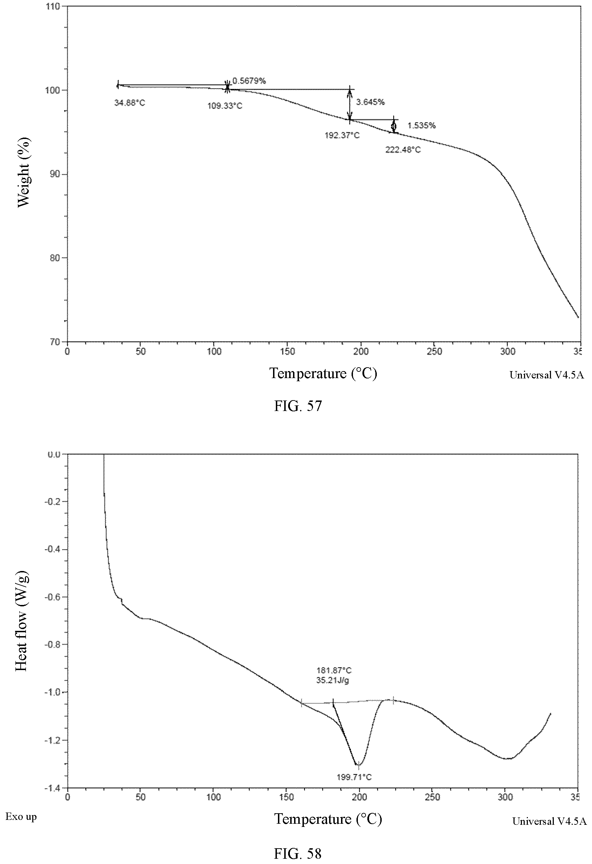

In one embodiment, the crystalline form of the toluene solvate of the sulfate salt of the compound of formula I has a thermogravimetric analysis curve substantially as shown in FIG. 57.

In one embodiment, the crystalline form of the toluene solvate of the sulfate salt of the compound of formula I has a differential scanning calorimetry (DSC) curve having an endothermic peak with an initial temperature of 181.87 °C ± 3 °C.

In one embodiment, the crystalline form of the toluene solvate of the sulfate salt of the compound of formula I has a differential scanning calorimetry curve having an endothermic peak with an initial temperature of 181.87 °C.

In one embodiment, the crystalline form of the toluene solvate of the sulfate salt of the compound of formula I has a differential scanning calorimetry curve having an endothermic peak with a peak temperature of 199.71 °C ± 3 °C.

In one embodiment, the crystalline form of the toluene solvate of the sulfate salt of the compound of formula I has a differential scanning calorimetry curve having an endothermic peak with a peak temperature of 199.71 °C.

In one embodiment, the crystalline form of the toluene solvate of the sulfate salt of the compound of formula I has a differential scanning calorimetry curve substantially as shown in FIG. 58.

The present invention further provides a preparation method for the crystalline form of the toluene solvate of the sulfate salt of the compound of formula I, which preferably comprises the following steps: mixing a crystalline form A of a sulfate salt of a compound of formula I with methanol, filtering the mixture to obtain a filtrate, adding toluene to the filtrate to precipitate the solid, and separating and drying the solid to obtain the crystalline form of the toluene solvate of the sulfate salt of the compound of formula I, wherein the crystalline form A of the sulfate salt of the compound of formula I and the methanol are preferably in a mass-to-volume ratio of (30 mg-40 mg):1 mL; and the crystalline form A of the sulfate salt of the compound of formula I and the toluene are preferably in a mass-to-volume ratio of (35 mg-45 mg):1 mL.

In one embodiment, in the preparation method for the crystalline form of the toluene solvate of the sulfate salt of the compound of formula I, the filtering is preferably performed using a 0.22 µm nylon filter membrane. The crystalline form A of the sulfate salt of the compound of formula I and the methanol are preferably in a mass-to-volume ratio of 35.71 mg: 1 mL. The crystalline form A of the sulfate salt of the compound of formula I and the toluene are preferably in a mass-to-volume ratio of 41.67 mg: 1 mL.

The present invention provides a crystalline form A of a p-toluenesulfonate salt of a compound of formula I, which has an X-ray powder diffraction (XRPD) pattern having at least three characteristic peaks at the following 2θ angles: 4.788° ± 0.2°, 5.839° ± 0.2°, and 17.48° ± 0.2°;

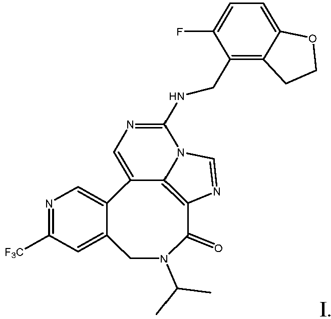

In one embodiment, the crystalline form A of the p-toluenesulfonate salt of the compound of formula I has an X-ray powder diffraction pattern having characteristic peaks at the following 2Θ angles: 4.788° ± 0.2°, 5.839° ± 0.2°, 16.624° ± 0.2°, 17.48° ± 0.2°, 18.354° ± 0.2°, 20.295° ± 0.2°, and 20.954° ± 0.2°.

In one embodiment, the crystalline form A of the p-toluenesulfonate salt of the compound of formula I has an X-ray powder diffraction pattern having characteristic peaks at the following 2θ angles: 4.788° ± 0.2°, 5.839° ± 0.2°, 7.235° ± 0.2°, 9.529° ± 0.2°, 10.656° ± 0.2°, 11.646° ± 0.2°, 12.275° ± 0.2°, 12.931° ± 0.2°, 13.878° ± 0.2°, 14.307° ± 0.2°, 14.598° ± 0.2°, 15.309° ± 0.2°, 15.57° ± 0.2°, 16.136° ± 0.2°, 16.624° ± 0.2°, 17.48° ± 0.2°, 18.354° ± 0.2°, 18.649° ± 0.2°, 19.348° ± 0.2°, 19.655° ± 0.2°, 20.295° ± 0.2°, 20.954° ± 0.2°, 21.385° ± 0.2°, 21.771° ± 0.2°, 21.988° ± 0.2°, 22.397° ± 0.2°, 23.019° ± 0.2°, 23.505° ± 0.2°, 23.911° ± 0.2°, 25.758° ± 0.2°, 26.126° ± 0.2°, 26.573° ± 0.2°, 27.78° ± 0.2°, 29.412° ± 0.2°, 29.76° ± 0.2°, 30.614° ± 0.2°, 30.827° ± 0.2°, 32.215° ± 0.2°, 32.517° ± 0.2°, 33.632° ± 0.2°, 35.063° ± 0.2°, and 37.779° ± 0.2°.

In one embodiment, the crystalline form A of the p-toluenesulfonate salt of the compound of formula I has X-ray powder diffraction pattern analysis data substantially as shown in Table 18:

**Table 18. X-ray powder diffraction pattern analysis data of the crystalline form A of the p-toluenesulfonate salt of the compound of formula I**

| Position [°2θ] ± 0.2° | d-spacing [Å] | Relative intensity [%] |
|---|---|---|
| 4.788 | 18.4391 | 86 |
| 5.839 | 15.1243 | 100 |
| 7.235 | 12.2076 | 8.1 |
| 9.529 | 9.2738 | 7.8 |
| 10.656 | 8.2954 | 3.5 |
| 11.646 | 7.5921 | 4.7 |
| 12.275 | 7.2047 | 4 |
| 12.931 | 6.8403 | 3.9 |
| 13.878 | 6.3758 | 5.6 |
| 14.307 | 6.1858 | 5 |
| 14.598 | 6.063 | 3.5 |
| 15.309 | 5.7828 | 3.9 |
| 15.57 | 5.6865 | 4.6 |
| 16.136 | 5.4882 | 6 |
| 16.624 | 5.3282 | 18 |
| 17.48 | 5.0691 | 19.1 |
| 18.354 | 4.8298 | 13.7 |
| 18.649 | 4.754 | 6.4 |
| 19.348 | 4.5838 | 8.8 |
| 19.655 | 4.5129 | 4.8 |
| 20.295 | 4.372 | 10.6 |
| 20.954 | 4.2359 | 10.4 |
| 21.385 | 4.1516 | 6.3 |
| 21.771 | 4.0788 | 6.6 |
| 21.988 | 4.0391 | 7.7 |
| 22.397 | 3.9662 | 7.3 |
| 23.019 | 3.8605 | 9.8 |
| 23.505 | 3.7817 | 9 |
| 23.911 | 3.7184 | 6.4 |
| 25.758 | 3.4558 | 8.5 |
| 26.126 | 3.408 | 5.7 |
| 26.573 | 3.3517 | 5.1 |
| 27.78 | 3.2087 | 6.8 |
| 29.412 | 3.0343 | 5.2 |
| 29.76 | 2.9996 | 3.6 |
| 30.614 | 2.9178 | 3.8 |
| 30.827 | 2.8981 | 3.8 |
| 32.215 | 2.7764 | 3.8 |
| 32.517 | 2.7513 | 3.3 |
| 33.632 | 2.6626 | 3.6 |
| 35.063 | 2.5571 | 2.7 |
| 37.779 | 2.3793 | 3 |

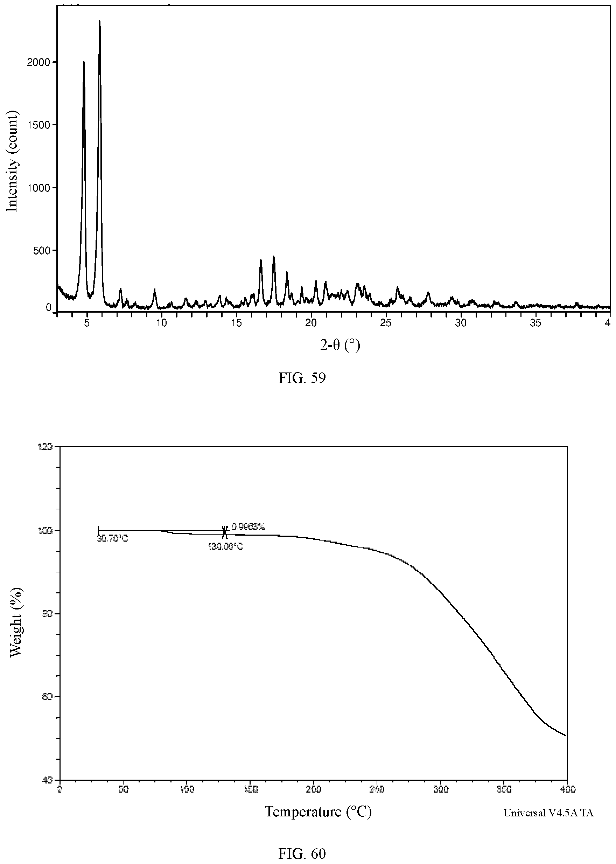

In one embodiment, the crystalline form A of the p-toluenesulfonate salt of the compound of formula I has an X-ray powder diffraction pattern substantially as shown in FIG. 59.

In one embodiment, the crystalline form A of the p-toluenesulfonate salt of the compound of formula I has a thermogravimetric analysis (TGA) curve showing a weight loss of 0.9963% ± 0.2% during heating from 30.7 °C ± 3 °C to 130 °C ± 3 °C.

In one embodiment, the crystalline form A of the p-toluenesulfonate salt of the compound of formula I has a thermogravimetric analysis curve showing a weight loss of 0.9963% during heating from 30.7 °C to 130 °C. In one embodiment, the crystalline form A of the p-toluenesulfonate salt of the compound of formula I has a thermogravimetric analysis curve substantially as shown in FIG. 60.

In one embodiment, the crystalline form A of the p-toluenesulfonate salt of the compound of formula I has a differential scanning calorimetry (DSC) curve having endothermic peaks with initial temperatures of 85.28 °C ± 3 °C, 193.65 °C ± 3 °C, and 223.74 °C ± 3 °C, respectively.

In one embodiment, the crystalline form A of the *p*-toluenesulfonate salt of the compound of formula I has a differential scanning calorimetry curve having endothermic peaks with initial temperatures of 85.28 °C, 193.65 °C, and 223.74 °C, respectively.

In one embodiment, the crystalline form A of the *p*-toluenesulfonate salt of the compound of formula I has a differential scanning calorimetry curve having endothermic peaks with peak temperatures of 105.14 °C ± 3 °C, 204.26 °C ± 3 °C, and 235.34 °C ± 3 °C, respectively.

In one embodiment, the crystalline form A of the *p*-toluenesulfonate salt of the compound of formula I has a differential scanning calorimetry curve having endothermic peaks with peak temperatures of 105.14 °C, 204.26 °C, and 235.34 °C, respectively.

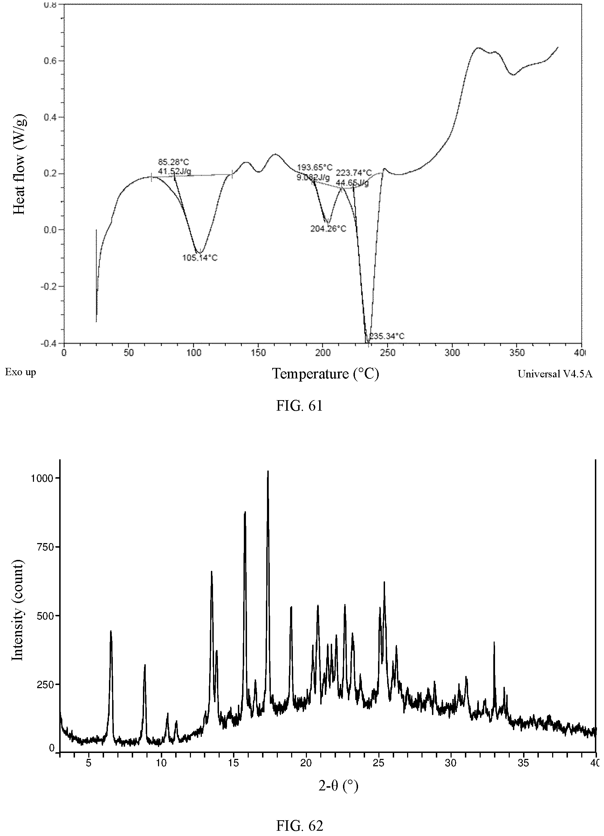

In one embodiment, the crystalline form A of the p-toluenesulfonate salt of the compound of formula I has a differential scanning calorimetry curve substantially as shown in FIG. 61.

The present invention further provides a preparation method for the crystalline form A of the *p-*toluenesulfonate salt of the compound of formula I, which preferably comprises the following steps: mixing and stirring a compound of formula I, tetrahydrofuran, and *p*-toluenesulfonic acid, adding n-heptane to precipitate a solid, and separating and drying the solid to obtain the crystalline form A of the p-toluenesulfonate salt of the compound of formula I, wherein the stirring is preferably performed at a temperature of 20-30 °C; the stirring is preferably performed for a period of 0.2-0.8 h; the compound of formula I and the *p-*toluenesulfonic acid are preferably in a molar ratio of (1-1.5):1; the compound of formula I and the tetrahydrofuran are preferably in a mass-to-volume ratio of (40 mg-60 mg):1 mL; and the compound of formula I and the n-heptane are preferably in a mass-to-volume ratio of (70 mg-90 mg):1 mL.

In one embodiment, in the preparation method for the crystalline form A of the p-toluenesulfonate salt of the compound of formula I, the compound of formula I is preferably a crystalline form A of a compound of formula I. The stirring is preferably performed at a temperature of 25 °C. The stirring is preferably performed for a period of 0.5 h; and the compound of formula I and the p-toluenesulfonic acid are preferably in a mass ratio of 1.1:1. The compound of formula I and the tetrahydrofuran are preferably in a mass-to-volume ratio of 50.7 mg: 1 mL. The compound of formula I and the n-heptane are preferably in a mass-to-volume ratio of 84.5 mg: 1 mL.

The present invention provides a crystalline form A of a benzenesulfonate salt of a compound of formula I, which has an X-ray powder diffraction (XRPD) pattern having at least three or at least four characteristic peaks at the following 2Θ angles: 13.477° ± 0.2°, 15.787° ± 0.2°, 17.362° ± 0.2°, and 25.371° ± 0.2°;

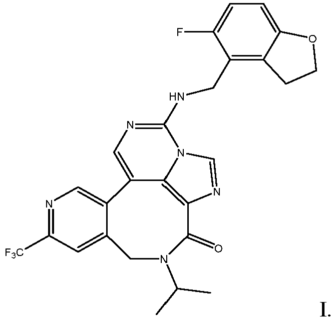

In one embodiment, the crystalline form A of the benzenesulfonate salt of the compound of formula I has an X-ray powder diffraction pattern having characteristic peaks at the following 2Θ angles: 13.477° ± 0.2°, 15.787° ± 0.2°, and 17.362° ± 0.2°.

In one embodiment, the crystalline form A of the benzenesulfonate salt of the compound of formula I has an X-ray powder diffraction pattern having characteristic peaks at the following 2Θ angles: 13.477° ± 0.2°, 15.787° ± 0.2°, 17.362° ± 0.2°, and 25.371° ± 0.2°.

In one embodiment, the crystalline form A of the benzenesulfonate salt of the compound of formula I has an X-ray powder diffraction pattern having characteristic peaks at the following 2Θ angles: 6.519° ± 0.2°, 8.866° ± 0.2°, 13.477° ± 0.2°, 13.842° ± 0.2°, 15.787° ± 0.2°, 17.362° ± 0.2°, 18.971° ± 0.2°, 20.452° ± 0.2°, 20.802° ± 0.2°, 21.48° ± 0.2°, 21.735° ± 0.2°, 22.067° ± 0.2°, 22.668° ± 0.2°, 23.175° ± 0.2°, 25.077° ± 0.2°, 25.371° ± 0.2°, 25.973° ± 0.2°, 26.206° ± 0.2°, and 32.971° ± 0.2°.

In one embodiment, the crystalline form A of the benzenesulfonate salt of the compound of formula I has an X-ray powder diffraction pattern having characteristic peaks at the following 2Θ angles: 6.519° ± 0.2°, 8.866° ± 0.2°, 10.437° ± 0.2°, 11.044° ± 0.2°, 13.059° ± 0.2°, 13.477° ± 0.2°, 13.842° ± 0.2°, 14.814° ± 0.2°, 15.787° ± 0.2°, 16.036° ± 0.2°, 16.488° ± 0.2°, 17.362° ± 0.2°, 18.971° ± 0.2°, 20.452° ± 0.2°, 20.802° ± 0.2°, 21.246° ± 0.2°, 21.48° ± 0.2°, 21.735° ± 0.2°, 22.067° ± 0.2°, 22.668° ± 0.2°, 23.175° ± 0.2°, 23.717° ± 0.2°, 25.077° ± 0.2°, 25.371° ± 0.2°, 25.973° ± 0.2°, 26.206° ± 0.2°, 26.453° ± 0.2°, 28.4° ± 0.2°, 28.851° ± 0.2°, 30.269° ± 0.2°, 30.538° ± 0.2°, 31.026° ± 0.2°, 31.864° ± 0.2°, 32.359° ± 0.2°, 32.971° ± 0.2°, 33.356° ± 0.2°, 33.652° ± 0.2°, and 38.822° ± 0.2°.

In one embodiment, the crystalline form A of the benzenesulfonate salt of the compound of formula I has X-ray powder diffraction pattern analysis data substantially as shown in Table 19:

**Table 19. X-ray powder diffraction pattern analysis data for the crystalline form A of the benzenesulfonate salt of the compound of formula I**

| Position [°2θ] ± 0.2° | d-spacing [Å] | Relative intensity [%] |
|---|---|---|
| 6.519 | 13.5469 | 43.1 |
| 8.866 | 9.9658 | 31 |
| 10.437 | 8.4687 | 13.8 |
| 11.044 | 8.0051 | 11.1 |
| 13.059 | 6.7739 | 14.6 |
| 13.477 | 6.5647 | 64.3 |
| 13.842 | 6.3925 | 36.1 |
| 14.814 | 5.9749 | 14.6 |
| 15.787 | 5.609 | 85.5 |
| 16.036 | 5.5224 | 22.4 |
| 16.488 | 5.3721 | 25.7 |
| 17.362 | 5.1035 | 100 |
| 18.971 | 4.674 | 51.7 |
| 20.452 | 4.3388 | 38 |
| 20.802 | 4.2667 | 52.1 |
| 21.246 | 4.1785 | 28.1 |
| 21.48 | 4.1334 | 38.4 |
| 21.735 | 4.0855 | 38.3 |
| 22.067 | 4.0247 | 41.6 |
| 22.668 | 3.9195 | 52.4 |
| 23.175 | 3.8348 | 42.3 |
| 23.717 | 3.7484 | 27.7 |
| 25.077 | 3.548 | 51.5 |
| 25.371 | 3.5076 | 60.5 |
| 25.973 | 3.4278 | 32.4 |
| 26.206 | 3.3977 | 37.8 |
| 26.453 | 3.3665 | 24.6 |
| 28.4 | 3.1401 | 22.8 |
| 28.851 | 3.092 | 25.2 |
| 30.269 | 2.9503 | 20.3 |
| 30.538 | 2.925 | 24.2 |
| 31.026 | 2.88 | 26.8 |
| 31.864 | 2.8061 | 18.1 |
| 32.359 | 2.7644 | 18.9 |
| 32.971 | 2.7144 | 39.1 |
| 33.356 | 2.684 | 16.1 |
| 33.652 | 2.661 | 23 |
| 38.822 | 2.3177 | 10.3 |

In one embodiment, the crystalline form A of the benzenesulfonate salt of the compound of formula I has an X-ray powder diffraction pattern substantially as shown in FIG. 62.

In one embodiment, the crystalline form A of the benzenesulfonate salt of the compound of formula I has a thermogravimetric analysis (TGA) curve showing a weight loss of 0.02869% ± 0.0005% during heating from 30.43 °C ± 3 °C to 149.29 °C ± 3 °C.

In one embodiment, the crystalline form A of the benzenesulfonate salt of the compound of formula I has a thermogravimetric analysis curve showing a weight loss of 0.02869% during heating from 30.43 °C to 149.29 °C.

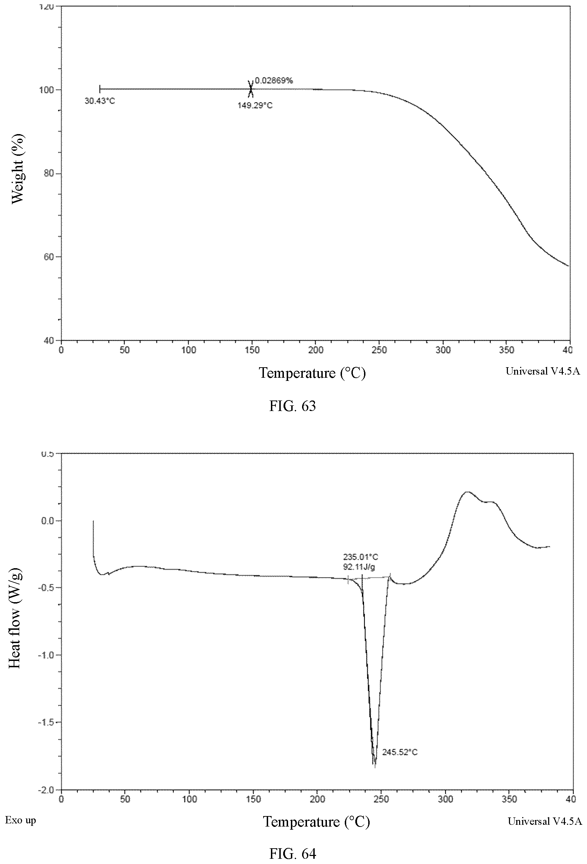

In one embodiment, the crystalline form A of the benzenesulfonate salt of the compound of formula I has a thermogravimetric analysis curve substantially as shown in FIG. 63.

In one embodiment, the crystalline form A of the benzenesulfonate salt of the compound of formula I has a differential scanning calorimetry (DSC) curve having an endothermic peak with an initial temperature of 235.01 °C ± 3 °C.

In one embodiment, the crystalline form A of the benzenesulfonate salt of the compound of formula I has a differential scanning calorimetry curve having an endothermic peak with an initial temperature of 235.01 °C. In one embodiment, the crystalline form A of the benzenesulfonate salt of the compound of formula I has a differential scanning calorimetry curve having an endothermic peak with a peak temperature of 245.52 °C ± 3 °C.

In one embodiment, the crystalline form A of the benzenesulfonate salt of the compound of formula I has a differential scanning calorimetry curve having an endothermic peak with a peak temperature of 245.52 °C. In one embodiment, the crystalline form A of the benzenesulfonate salt of the compound of formula I has a differential scanning calorimetry curve substantially as shown in FIG. 64.

The present invention further provides a preparation method for the crystalline form A of the benzenesulfonate salt of the compound of formula I, which preferably comprises the following steps: mixing and stirring a compound of formula I, tetrahydrofuran, and benzenesulfonic acid, adding n-heptane to precipitate a solid, and separating and drying the solid to obtain the crystalline form A of the benzenesulfonate salt of the compound of formula I, wherein the stirring is preferably performed at a temperature of 20-30 °C; the stirring is preferably performed for a period of 0.2-0.8 h; the compound of formula I and the benzenesulfonic acid are preferably in a molar ratio of (1-1.5):1; the compound of formula I and the tetrahydrofuran are preferably in a mass-to-volume ratio of (40 mg-60 mg):1 mL; and the compound of formula I and the n-heptane are preferably in a mass-to-volume ratio of (55 mg-75 mg):1 mL.

In one embodiment, in the preparation method for the crystalline form A of the benzenesulfonate salt of the compound of formula I, the compound of formula I is preferably a crystalline form A of a compound of formula I. The stirring is preferably performed at a temperature of 25 °C. The stirring is preferably performed for a period of 0.5 h; and the compound of formula I and the benzenesulfonic acid are preferably in a mass ratio of 1.1:1. The compound of formula I and the tetrahydrofuran are preferably in a mass-to-volume ratio of 52.8 mg: 1 mL. The compound of formula I and the n-heptane are preferably in a mass-to-volume ratio of 66 mg:1 mL.

The present invention provides a crystalline form A of a methanesulfonate salt of a compound of formula I, which has an X-ray powder diffraction (XRPD) pattern having at least three characteristic peaks at the following 2Θ angles: 7.548° ± 0.2°, 15.087° ± 0.2°, and 15.554° ± 0.2°;

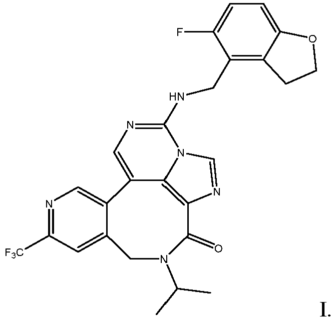

In one embodiment, the crystalline form A of the methanesulfonate salt of the compound of formula I has an X-ray powder diffraction pattern having characteristic peaks at the following 2Θ angles: 7.548° ± 0.2°, 9.084° ± 0.2°, 10.268° ± 0.2°, 12.268° ± 0.2°, 13.505° ± 0.2°, 15.087° ± 0.2°, 15.554° ± 0.2°, 16.917° ± 0.2°, 18.994° ± 0.2°, 19.853° ± 0.2°, 20.515° ± 0.2°, 22.185° ± 0.2°, 22.785° ± 0.2°, 23.075° ± 0.2°, 24.146° ± 0.2°, 25.038° ± 0.2°, 26.533° ± 0.2°, 27.082° ± 0.2°, 28.572° ± 0.2°, 29.68° ± 0.2°, and 30.167° ± 0.2°.

In one embodiment, the crystalline form A of the methanesulfonate salt of the compound of formula I has X-ray powder diffraction pattern analysis data substantially as shown in Table 20:

**Table 20. X-ray powder diffraction pattern analysis data for the crystalline form A of the methanesulfonate salt of the compound of formula I**

| Position [°2θ] ± 0.2° | d-spacing [Å] | Relative intensity [%] |
|---|---|---|
| 7.548 | 11.7025 | 100 |
| 9.084 | 9.7266 | 7.3 |
| 10.268 | 8.6082 | 1.3 |
| 12.268 | 7.2085 | 1.4 |
| 13.505 | 6.5508 | 1.4 |
| 15.087 | 5.8677 | 19.7 |
| 15.554 | 5.6924 | 21 |
| 16.917 | 5.2366 | 2 |
| 18.994 | 4.6685 | 1.8 |
| 19.853 | 4.4684 | 1.9 |
| 20.515 | 4.3257 | 4.5 |
| 22.185 | 4.0037 | 5.6 |
| 22.785 | 3.8996 | 5.2 |
| 23.075 | 3.8511 | 4.4 |
| 24.146 | 3.6828 | 2.2 |
| 25.038 | 3.5535 | 3.5 |
| 26.533 | 3.3566 | 1.7 |
| 27.082 | 3.2898 | 2 |
| 28.572 | 3.1215 | 1.8 |
| 29.68 | 3.0075 | 2.5 |
| 30.167 | 2.96 | 3 |

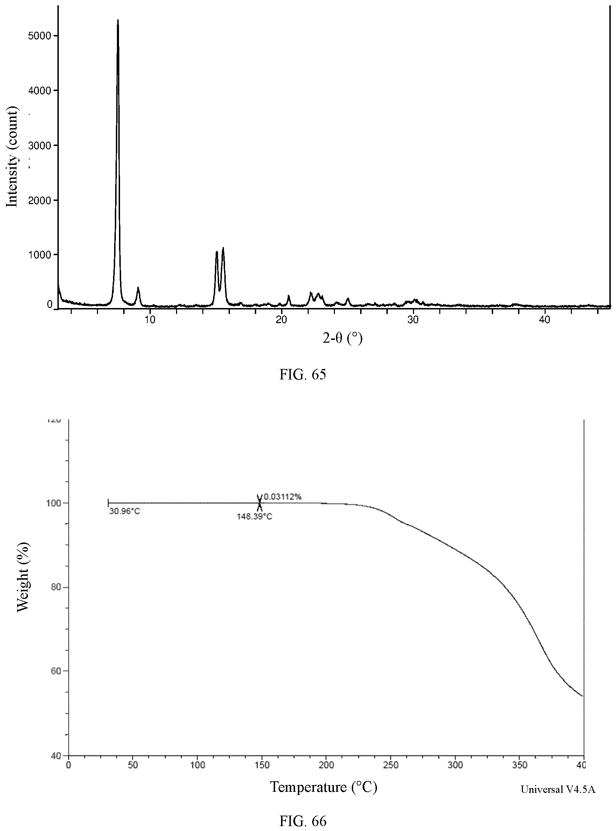

In one embodiment, the crystalline form A of the methanesulfonate salt of the compound of formula I has an X-ray powder diffraction pattern substantially as shown in FIG. 65.

In one embodiment, the crystalline form A of the methanesulfonate salt of the compound of formula I has a thermogravimetric analysis (TGA) curve showing a weight loss of 0.03112% ± 0.0005% during heating from 30.96 °C ± 3 °C to 148.39 °C ± 3 °C.

In one embodiment, the crystalline form A of the methanesulfonate salt of the compound of formula I has a thermogravimetric analysis curve showing a weight loss of 0.03112% during heating from 30.96 °C to 148.39 °C.

In one embodiment, the crystalline form A of the methanesulfonate salt of the compound of formula I has a thermogravimetric analysis curve substantially as shown in FIG. 66.

In one embodiment, the crystalline form A of the methanesulfonate salt of the compound of formula I has a differential scanning calorimetry (DSC) curve having an endothermic peak with an initial temperature of 250.75 °C ± 3 °C.

In one embodiment, the crystalline form A of the methanesulfonate salt of the compound of formula I has a differential scanning calorimetry curve having an endothermic peak with an initial temperature of 250.75 °C. In one embodiment, the crystalline form A of the methanesulfonate salt of the compound of formula I has a differential scanning calorimetry curve having an endothermic peak with a peak temperature of 262.7 °C ± 3°C.

In one embodiment, the crystalline form A of the methanesulfonate salt of the compound of formula I has a differential scanning calorimetry curve having an endothermic peak with a peak temperature of 262.7 °C.

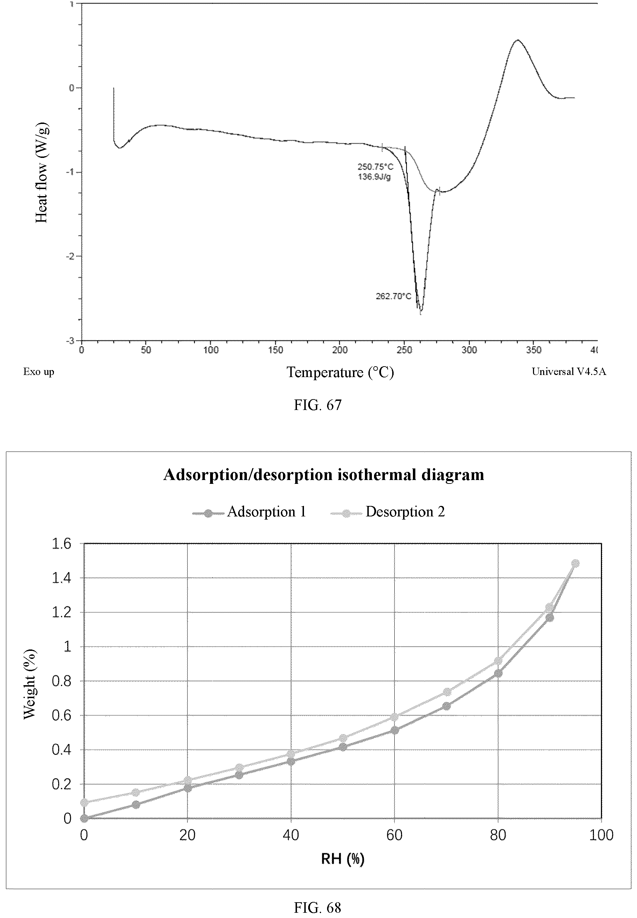

In one embodiment, the crystalline form A of the methanesulfonate salt of the compound of formula I has a differential scanning calorimetry curve substantially as shown in FIG. 67.

In one embodiment, the crystalline form A of the methanesulfonate salt of the compound of formula I has a dynamic vapor sorption (DVS) curve showing a hygroscopic weight gain of 0.84% ± 0.005% at 25 °C and 80% RH.

In one embodiment, the crystalline form A of the methanesulfonate salt of the compound of formula I has a dynamic vapor sorption curve showing a hygroscopic weight gain of 0.84% at 25 °C and 80% RH.

In one embodiment, the crystalline form A of the methanesulfonate salt of the compound of formula I has a dynamic vapor sorption curve as shown in FIG. 68.

The present invention further provides a preparation method for the crystalline form A of the methanesulfonate salt of the compound of formula I, preferably any one of the following methods:
method I: mixing and stirring a compound of formula I, tetrahydrofuran and methanesulfonic acid to precipitate a solid, and separating and drying the solid to obtain the crystalline form A of the methanesulfonate salt of the compound of formula I, wherein the stirring is performed at a temperature of 20-30 °C; the stirring is preferably performed for a period of 0.2-0.8 h; the compound of formula I and the methanesulfonic acid are preferably in a mass ratio of (1 to 1.5): 1; and the compound of formula I and the tetrahydrofuran are preferably in a mass-to-volume ratio of (20 mg-30 mg): 1 mL; and
method II: stirring an amorphous form of a methanesulfonate salt of a compound of formula I in an organic solvent to precipitate a solid, and separating and drying the solid to obtain the crystalline form A of the methanesulfonate salt of the compound of formula I, wherein the organic solvent is selected from one or more of dichloromethane, methyl isobutyl ketone, heptane, acetone, acetonitrile, ethyl acetate, tetrahydrofuran, and toluene; the stirring is preferably performed at 20-60 °C; the stirring is preferably performed for a period of (4-8) days; and the crystalline form A of the compound of formula I and the organic solvent are preferably in a mass-to-volume ratio of (150 mg-250 mg): 1 mL.

In one embodiment, in the method I, the compound of formula I is preferably a crystalline form A of a compound of formula I. The stirring is preferably performed at a temperature of 25 °C. The stirring is preferably performed for a period of 0.5 h; and the compound of formula I and the methanesulfonic acid are preferably in a mass ratio of 1.1:1. The compound of formula I and the tetrahydrofuran are preferably in a mass-to-volume ratio of 25.05 mg: 1 mL.

In one embodiment, in the method II, the stirring is preferably performed at 25 °C or 50 °C. The stirring is preferably performed for a period of 6 days; and the crystalline form A of the compound of formula I and the organic solvent are preferably in a mass-to-volume ratio of 200 mg: 1 mL.

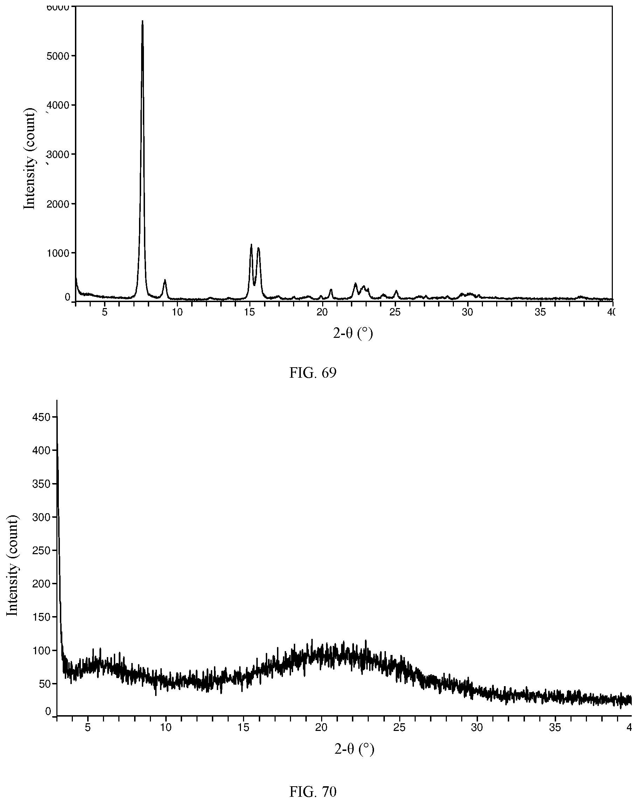

The present invention provides an amorphous form of a methanesulfonate salt of a compound of formula I, which has an X-ray powder diffraction pattern substantially as shown in FIG. 70;

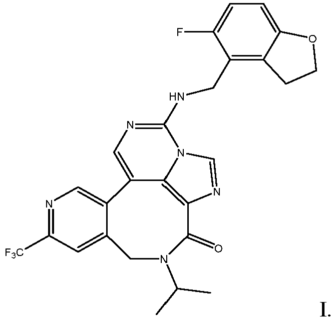

In one embodiment, the amorphous form of the methanesulfonate salt of the compound of formula I has a thermogravimetric analysis (TGA) curve showing a weight loss of 0.8804% ± 0.005% during heating from 41.74 °C ± 3 °C to 107.12 °C ± 3 °C.

In one embodiment, the amorphous form of the methanesulfonate salt of the compound of formula I has a thermogravimetric analysis curve showing a weight loss of 0.8804% during heating from 41.74 °C to 107.12 °C.

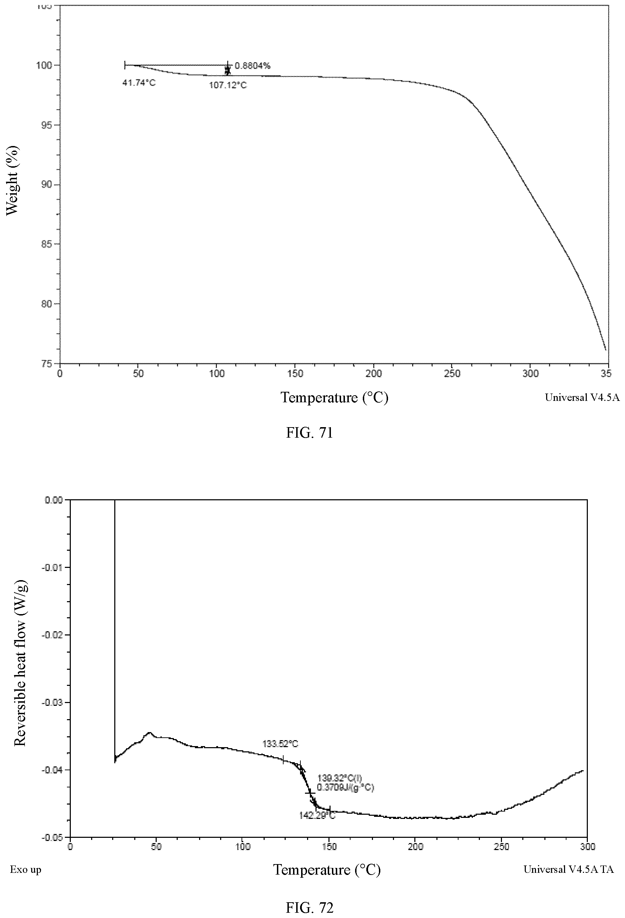

In one embodiment, the amorphous form of the methanesulfonate salt of the compound of formula I has a thermogravimetric analysis curve substantially as shown in FIG. 71.

In one embodiment, the amorphous form of the methanesulfonate salt of the compound of formula I has a modulated differential scanning calorimetry (mDSC) curve showing a glass transition temperature of 139.32 °C ± 3 °C.

In one embodiment, the amorphous form of the methanesulfonate salt of the compound of formula I has a modulated differential scanning calorimetry curve showing a glass transition temperature of 139.32 °C.

In one embodiment, the amorphous form of the methanesulfonate salt of the compound of formula I has a modulated differential scanning calorimetry curve as shown in FIG. 72.

The present invention further provides a preparation method for the amorphous form of the methanesulfonate salt of the compound of the formula I, which comprises the following steps: mixing a crystalline form A of a methanesulfonate of a compound of formula I with a mixed solvent of dichloromethane and methanol, and performing spray drying and vacuum drying sequentially to obtain the amorphous form of the methanesulfonate salt of the compound of formula I, wherein the dichloromethane and the methanol are preferably in a volume ratio of (1.5-2.5):1; the spray drying is preferably performed at a temperature of 80-100 °C; the vacuum drying is preferably performed at a temperature of 40-60 °C; and the crystalline form A of the methanesulfonate salt of the compound of formula I and the mixed solvent are preferably in a mass-to-volume ratio of (1.5-2.5):1.

In a certain embodiment, in the preparation method for the amorphous form of the methanesulfonate salt of the compound of formula I, the dichloromethane and the methanol are preferably in a volume ratio of 2:1. The spray drying is preferably performed at a temperature of 90 °C; and the vacuum drying is preferably performed at a temperature of 50 °C. The crystalline form A of the methanesulfonate salt of the compound of formula I and the mixed solvent are preferably in a mass-to-volume ratio of 2:1.

The present invention provides a crystalline form of a methanol solvate of a methanesulfonate salt of the compound of formula I, which has an X-ray powder diffraction (XRPD) pattern having at least three characteristic peaks at the following 2Θ angles: 7.467° ± 0.2°, 21.736° ± 0.2°, and 23.056° ± 0.2°;

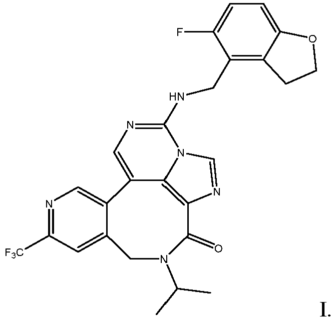

In one embodiment, the crystalline form of the methanol solvate of the methanesulfonate salt of the compound of formula I has an X-ray powder diffraction pattern having characteristic peaks at the following 2Θ angles: 7.467° ± 0.2°, 8.749° ± 0.2°, 10.402° ± 0.2°, 12.311° ± 0.2°, 13.029° ± 0.2°, 14.797° ± 0.2°, 15.301° ± 0.2°, 16.972° ± 0.2°, 17.749° ± 0.2°, 18.506° ± 0.2°, 19.113° ± 0.2°, 19.731° ± 0.2°, 20.376° ± 0.2°, 20.765° ± 0.2°, 21.736° ± 0.2°, 22.341° ± 0.2°, 23.056° ± 0.2°, 24.415° ± 0.2°, 25.173° ± 0.2°, 25.485° ± 0.2°, 25.855° ± 0.2°, 26.711° ± 0.2°, 27.292° ± 0.2°, 28.226° ± 0.2°, 28.516° ± 0.2°, 29.02° ± 0.2°, 29.745° ± 0.2°, 30.886° ± 0.2°, 31.318° ± 0.2°, 32.755° ± 0.2°, 33.111° ± 0.2°, 33.972° ± 0.2°, 34.666° ± 0.2°, 35.143° ± 0.2°, 36.057° ± 0.2°, 37.334° ± 0.2°, and 39.149° ± 0.2°.

In one embodiment, the crystalline form of the methanol solvate of the methanesulfonate salt of the compound of formula I has X-ray powder diffraction pattern analysis data substantially as shown in Table 21:

**Table 21. X-ray powder diffraction pattern analysis data for the crystalline form of the methanol solvate of the methanesulfonate salt of the compound of formula I**

| Position [°2θ] ± 0.2° | d-spacing [Å] | Relative intensity [%] |
|---|---|---|
| 7.467 | 11.83 | 100 |
| 8.749 | 10.0987 | 6.9 |
| 10.402 | 8.4973 | 1.6 |
| 12.311 | 7.1837 | 5.7 |
| 13.029 | 6.7893 | 3.4 |
| 14.797 | 5.9818 | 19.1 |
| 15.301 | 5.7859 | 4.2 |
| 16.972 | 5.2197 | 2.6 |
| 17.749 | 4.993 | 9.2 |
| 18.506 | 4.7904 | 5.4 |
| 19.113 | 4.6396 | 4.7 |
| 19.731 | 4.4958 | 3.7 |
| 20.376 | 4.3549 | 4 |
| 20.765 | 4.2742 | 10.5 |
| 21.736 | 4.0853 | 23.8 |
| 22.341 | 3.976 | 6.7 |
| 23.056 | 3.8544 | 20.1 |
| 24.415 | 3.6428 | 4.7 |
| 25.173 | 3.5348 | 8.9 |
| 25.485 | 3.4923 | 5 |
| 25.855 | 3.4431 | 4.5 |
| 26.711 | 3.3347 | 4.6 |
| 27.292 | 3.2649 | 3 |
| 28.226 | 3.159 | 4 |
| 28.516 | 3.1275 | 4.8 |
| 29.02 | 3.0744 | 4.9 |
| 29.745 | 3.0011 | 2.7 |
| 30.886 | 2.8927 | 3.3 |
| 31.318 | 2.8538 | 3.7 |
| 32.755 | 2.7318 | 2 |
| 33.111 | 2.7033 | 2.4 |
| 33.972 | 2.6367 | 2.2 |
| 34.666 | 2.5855 | 2.3 |
| 35.143 | 2.5515 | 4 |
| 36.057 | 2.4889 | 2.4 |
| 37.334 | 2.4066 | 2.3 |
| 39.149 | 2.2991 | 2 |

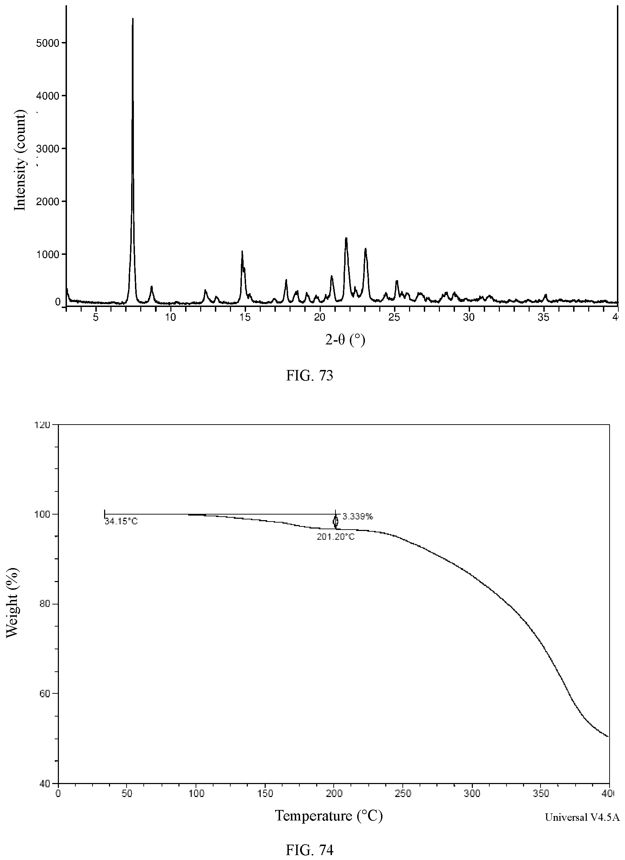

In one embodiment, the crystalline form of the methanol solvate of the methanesulfonate salt of the compound of formula I has an X-ray powder diffraction pattern substantially as shown in FIG. 73.

In one embodiment, the crystalline form of the methanol solvate of the methanesulfonate salt of the compound of formula I has a thermogravimetric analysis (TGA) curve showing a weight loss of 3.339% ± 0.5% during heating from 34.15 °C ± 3 °C to 201.2 °C ± 3 °C.

In one embodiment, the crystalline form of the methanol solvate of the methanesulfonate salt of the compound of formula I has a thermogravimetric analysis curve showing a weight loss of 3.339% during heating from 34.15 °C to 201.2 °C.

In one embodiment, the crystalline form of the methanol solvate of the methanesulfonate salt of the compound of formula I has a thermogravimetric analysis curve substantially as shown in FIG. 74.

In one embodiment, the crystalline form of the methanol solvate of the methanesulfonate salt of the compound of formula I has a differential scanning calorimetry (DSC) curve having endothermic peaks with initial temperatures of 162.9 °C ± 3 °C and 248.02 °C ± 3 °C, respectively.

In one embodiment, the crystalline form of the methanol solvate of the methanesulfonate salt of the compound of formula I has a differential scanning calorimetry curve having endothermic peaks with initial temperatures of 162.9 °C and 248.02 °C, respectively.

In one embodiment, the crystalline form of the methanol solvate of the methanesulfonate salt of the compound of formula I has a differential scanning calorimetry curve having endothermic peaks with peak temperatures of 176.43 °C ± 3 °C and 260.81 °C ± 3 °C, respectively.

In one embodiment, the crystalline form of the methanol solvate of the methanesulfonate salt of the compound of formula I has a differential scanning calorimetry curve having endothermic peaks with peak temperatures of 176.43 °C and 260.81 °C, respectively.

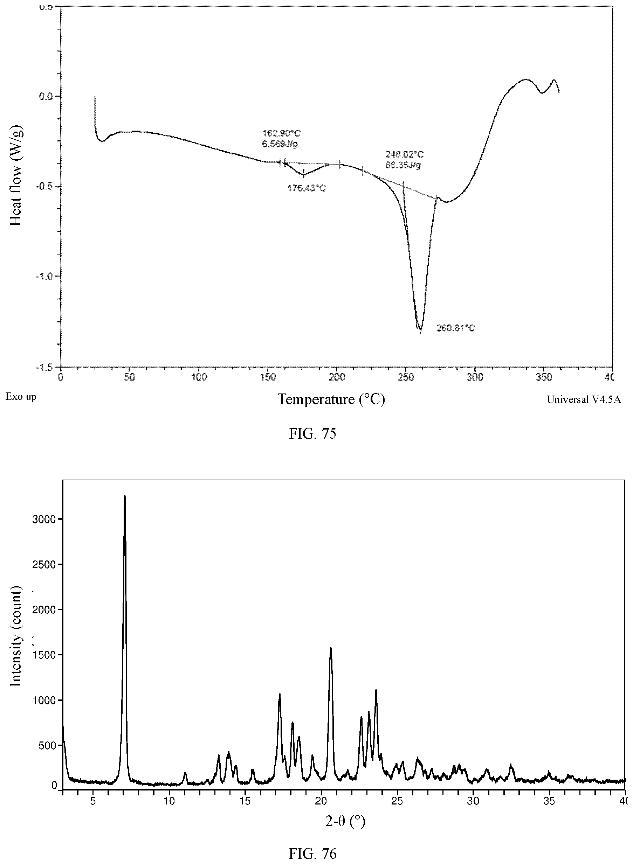

In one embodiment, the crystalline form of the methanol solvate of the methanesulfonate salt of the compound of formula I has a differential scanning calorimetry curve substantially as shown in FIG. 75.

The present invention further provides a preparation method for the crystalline form of the methanol solvate of the methanesulfonate salt of the compound of formula I, which preferably comprises the following steps: stirring an amorphous form of a methanesulfonate salt of a compound of formula I in a solvent to precipitate a solid, and separating and drying the solid to obtain the crystalline form of the methanol solvate of the methanesulfonate salt of the compound of formula I, wherein the solvent is methanol or a mixed solvent of methanol and an organic solvent; the organic solvent is selected from one or more of tetrahydrofuran, ethyl acetate, and acetonitrile; the stirring is preferably performed at 20-60 °C; the stirring is preferably performed for period of 4-8 days; the amorphous form of the methanesulfonate salt of the compound of formula I and the solvent are preferably in a mass-to-volume ratio of (150 mg-250 mg):1 mL; and the methanol and the organic solvent are preferably in a volume ratio of (0.5-1.5):1.

In one embodiment, in the preparation method for the crystalline form of the methanol solvate of the methanesulfonate salt of the compound of formula I, the stirring is preferably performed at 25 °C, 40 °C, or 50 °C. The stirring is preferably performed for a period of 6 days. The amorphous form of the methanesulfonate salt of the compound of formula I and the solvent are preferably in a mass-to-volume ratio of 200 mg: 1 mL. The methanol and the organic solvent are preferably in a volume ratio of 1:1.

The present invention provides a crystalline form of a 1,4-dioxane solvate of a methanesulfonate salt of a compound of formula I, which has an X-ray powder diffraction (XRPD) pattern having three or at least four characteristic peaks at the following 2θ angles: 7.1° ± 0.2°, 17.283° ± 0.2°, 20.608° ± 0.2°, and 23.6° ± 0.2°;

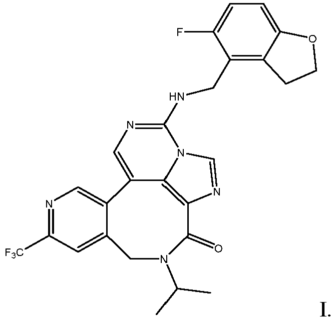

In one embodiment, the crystalline form of the 1,4-dioxane solvate of the methanesulfonate salt of the compound of formula I has an X-ray powder diffraction pattern having characteristic peaks at the following 2Θ angles: 7.1° ± 0.2°, 20.608° ± 0.2°, and 23.6° ± 0.2°.

In one embodiment, the crystalline form of the 1,4-dioxane solvate of the methanesulfonate salt of the compound of formula I has an X-ray powder diffraction pattern having characteristic peaks at the following 2Θ angles: 7.1° ± 0.2°, 17.283° ± 0.2°, 20.608° ± 0.2°, and 23.6° ± 0.2°.

In one embodiment, the crystalline form of the 1,4-dioxane solvate of the methanesulfonate salt of the compound of formula I has an X-ray powder diffraction pattern having characteristic peaks at the following 2Θ angles: 7.1° ± 0.2°, 13.26° ± 0.2°, 13.923° ± 0.2°, 17.283° ± 0.2°, 17.596° ± 0.2°, 18.121° ± 0.2°, 18.547° ± 0.2°, 19.424° ±0.2°, 20.608° ± 0.2°, 22.628° ± 0.2°, 23.116° ±0.2°, 23.6° ±0.2°, 23.913° ± 0.2°, and 26.322° ± 0.2°.

In one embodiment, the crystalline form of the 1,4-dioxane solvate of the methanesulfonate salt of the compound of formula I has an X-ray powder diffraction pattern having characteristic peaks at the following 2Θ angles: 7.1° ± 0.2°, 11.029° ± 0.2°, 12.504° ± 0.2°, 13.26° ± 0.2°, 13.923° ± 0.2°, 14.389° ± 0.2°, 15.55° ± 0.2°, 17.283° ± 0.2°, 17.596° ± 0.2°, 18.121° ± 0.2°, 18.547° ± 0.2°, 19.424° ± 0.2°, 20.608° ± 0.2°, 21.428° ± 0.2°, 21.718° ± 0.2°, 22.628° ± 0.2°, 23.116° ± 0.2°, 23.6° ± 0.2°, 23.913° ± 0.2°, 24.958° ± 0.2°, 25.384° ± 0.2°, 26.322° ± 0.2°, 26.843° ± 0.2°, 27.274° ± 0.2°, 28.05° ± 0.2°, 28.732° ± 0.2°, 29.064° ± 0.2°, 29.412° ± 0.2°, 30.056° ± 0.2°, 30.85° ± 0.2°, 31.763° ± 0.2°, 32.444° ± 0.2°, 34.933° ± 0.2°, 36.254° ± 0.2°, 36.489° ± 0.2°, and 37.421° ± 0.2°.

In one embodiment, the crystalline form of the 1,4-dioxane solvate of the methanesulfonate salt of the compound of formula I has X-ray powder diffraction pattern analysis data substantially as shown in Table 22:

**Table 22. X-ray powder diffraction pattern analysis data for the crystalline form of the 1,4-dioxane solvate of the methanesulfonate salt of the compound of formula I**

| Position [°2θ] ± 0.2° | d-spacing [Å] | Relative intensity [%] |
|---|---|---|
| 7.1 | 12.4399 | 100 |
| 11.029 | 8.0153 | 5.8 |
| 12.504 | 7.0731 | 3.4 |
| 13.26 | 6.6715 | 11.7 |
| 13.923 | 6.3555 | 12.9 |
| 14.389 | 6.1507 | 8.2 |
| 15.55 | 5.6939 | 6.8 |
| 17.283 | 5.1265 | 32.6 |
| 17.596 | 5.0362 | 11.7 |
| 18.121 | 4.8915 | 22.9 |
| 18.547 | 4.7799 | 18 |
| 19.424 | 4.5662 | 11.8 |
| 20.608 | 4.3063 | 48.2 |
| 21.428 | 4.1435 | 5 |
| 21.718 | 4.0888 | 6.8 |
| 22.628 | 3.9262 | 24.9 |
| 23.116 | 3.8444 | 26.6 |
| 23.6 | 3.7667 | 33.9 |
| 23.913 | 3.7181 | 12.2 |
| 24.958 | 3.5647 | 8.9 |
| 25.384 | 3.5059 | 9.5 |
| 26.322 | 3.3831 | 11 |
| 26.843 | 3.3185 | 6.8 |
| 27.274 | 3.2671 | 7.1 |
| 28.05 | 3.1784 | 5.8 |
| 28.732 | 3.1046 | 8.1 |
| 29.064 | 3.0698 | 8.6 |
| 29.412 | 3.0342 | 7 |
| 30.056 | 2.9707 | 3.9 |
| 30.85 | 2.8961 | 7.1 |
| 31.763 | 2.8149 | 4.6 |
| 32.444 | 2.7573 | 8.7 |
| 34.933 | 2.5663 | 6.5 |
| 36.254 | 2.4758 | 5 |
| 36.489 | 2.4604 | 4.7 |
| 37.421 | 2.4012 | 4.2 |

In one embodiment, the crystalline form of the 1,4-dioxane solvate of the methanesulfonate salt of the compound of formula I has an X-ray powder diffraction pattern substantially as shown in FIG. 76.

In one embodiment, the crystalline form of the 1,4-dioxane solvate of the methanesulfonate salt of the compound of formula I has a thermogravimetric analysis (TGA) curve showing a weight loss of 1.573% ± 0.5% during heating from 33.54 °C ± 3 °C to 117.32 °C ± 3 °C; and a weight loss of 5.341% ± 0.5% during heating from 117.32°C ± 3 °C to 200 °C ± 3 °C.

In one embodiment, the crystalline form of the 1,4-dioxane solvate of the methanesulfonate salt of the compound of formula I has a thermogravimetric analysis curve showing a weight loss of 1.573% during heating to 117.32 °C at 33.54 °C; and a weight loss of 5.341% during heating from 117.32 °C to 200 °C.

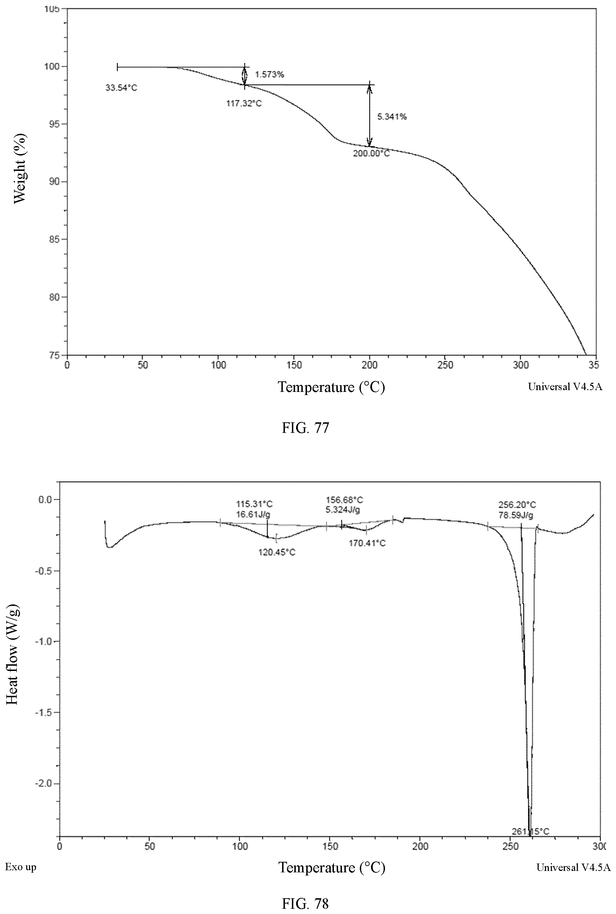

In one embodiment, the crystalline form of the 1,4-dioxane solvate of the methanesulfonate salt of the compound of formula I has a thermogravimetric analysis curve substantially as shown in FIG. 77.

In one embodiment, the crystalline form of the 1,4-dioxane solvate of the methanesulfonate salt of the compound of formula I has a differential scanning calorimetry (DSC) curve having endothermic peaks with initial temperatures of 115.31 °C ± 3 °C, 156.68 °C ± 3 °C, and 256.2 °C ± 3 °C, respectively.

In one embodiment, the crystalline form of the 1,4-dioxane solvate of the methanesulfonate salt of the compound of formula I has a differential scanning calorimetry curve having endothermic peaks with initial temperatures of 115.31 °C, 156.68 °C, and 256.2 °C, respectively.

In one embodiment, the crystalline form of the 1,4-dioxane solvate of the methanesulfonate salt of the compound of formula I has a differential scanning calorimetry curve having endothermic peaks with peak temperatures of 120.45 °C ± 3 °C, 170.41 °C ± 3 °C, and 261.15 °C ± 3 °C, respectively.

In one embodiment, the crystalline form of the 1,4-dioxane solvate of the methanesulfonate salt of the compound of formula I has a differential scanning calorimetry curve having endothermic peaks with peak temperatures of 120.45 °C, 170.41 °C, and 261.15 °C, respectively.

In one embodiment, the crystalline form of the 1,4-dioxane solvate of the methanesulfonate salt of the compound of formula I has a differential scanning calorimetry curve substantially as shown in FIG. 78.

The present invention further provides a preparation method for the crystalline form of the 1,4-dioxane solvate of the methanesulfonate salt of the compound of formula I, which preferably comprises the following steps: stirring an amorphous form of a methanesulfonate salt of a compound of formula I in 1,4-dioxane to precipitate a solid, and separating and drying the solid to obtain the crystalline form of the 1,4-dioxane solvate of the methanesulfonate salt of the compound of formula I, wherein the stirring is preferably performed at 20-60 °C; the stirring is preferably performed for a period of 4-8 days; and the amorphous form of the methanesulfonate salt of the compound of formula I and the 1,4-dioxane are preferably in a mass-to-volume ratio of (150 mg-250 mg):1 mL.

In one embodiment, in the preparation method for the crystalline form of the 1,4-dioxane solvate of the methanesulfonate salt of the compound of formula I, the stirring is preferably performed at 25 °C, 40 °C, or 50 °C. The stirring is preferably performed for a period of 6 days. The amorphous form of the methanesulfonate salt of the compound of formula I and the 1,4-dioxane are preferably in a mass-to-volume ratio of 200 mg: 1 mL.

The present invention further provides a pharmaceutical composition comprising a crystalline form or an amorphous form of the macrocyclic compound, or a salt or solvate thereof of the present invention.

The present invention further provides a method for treating or preventing a disease or condition for which inhibition of EED provides a benefit, which comprises administering to a subject in need the crystalline form or the amorphous form of the macrocyclic compound, or the salt or solvate or thereof of the present invention. Preferably, the disease or condition for which inhibition of EED provides a benefit is cancer or a proliferative disease.

In one embodiment, the cancer is preferably EED-mediated cancer. The EED-mediated cancer is a cancer known in the art.

In particular, the EED-mediated cancer may be found in WO2021011713A1, which is incorporated herein by reference in its entirety.

The crystalline or amorphous form of the macrocyclic compound or the salt or solvate thereof of the present invention include, but is not limited to, a crystalline form A of a compound of formula I, a crystalline form B of a compound of formula I, a crystalline form C of a compound of formula I, a crystalline form of a toluene solvate of a compound of formula I, a crystalline form E of a compound of formula I, a crystalline form F of a compound of formula I, a crystalline form G of a compound of formula I, a crystalline form of an N-methyl-2-pyrrolidone solvate of a compound of formula I, a crystalline form of a DMF solvate of a compound of formula I, a crystalline form K of a compound of formula I, a crystalline form A of a hydrochloride salt of a compound of formula I, a crystalline form A of an ethanol solvate of a hydrochloride salt of a compound of formula I, a crystalline form of an isopropanol solvate of a hydrochloride salt of a compound of formula I, a crystalline form A of a sulfate salt of a compound of formula I, a crystalline form B of a sulfate salt of a compound of formula I, a crystalline form C of a sulfate salt of a compound of formula I, a crystalline form of a toluene solvate of a sulfate salt of a compound of formula I, a crystalline form A of a p-toluenesulfonate salt of a compound of formula I, a crystalline form A of a benzenesulfonate salt of a compound of formula I, a crystalline form A of a methanesulfonate salt of a compound of formula I, an amorphous form of a methanesulfonate salt of a compound of formula I, a crystalline form of a methanol solvate of a methanesulfonate salt of a compound of formula I, and a crystalline form of a 1,4-dioxane solvate of a methanesulfonate salt of a compound of formula I, in the present invention.

In the present invention, the X-ray powder diffraction pattern is obtained by detection under a Cu-Kα ray source.

In the present invention, the differential scanning calorimetry (DSC) curve is measured at a heating rate of 10 °C/min.

In the present invention, the thermogravimetric analysis (TGA) curve is measured at a heating rate of 10 °C/min.

In the present invention, the modulated differential scanning calorimetry (mDSC) curve is measured at a heating rate of 3 °C/min.

In the present invention, the nuclear magnetic resonance hydrogen spectrum (¹H-NMR) is measured using deuterated DMSO as a solvent.

The above preferred conditions may be combined arbitrarily to obtain preferred embodiments of the present invention without departing from the general knowledge in the art.

The reagents and starting materials used in the present invention are commercially available.

The positive and progressive effects of the present invention are as follows: the crystalline or amorphous form of the macrocyclic compound or the salt or solvate thereof of the present invention has one or more advantages of high crystallinity, good stability, low hygroscopicity, easy formation of solvates, and difficulty in preparation.

### BRIEF DESCRIPTION OF THE DRAWINGS

FIG. 1 is an XRPD pattern of an amorphous form of a compound of formula I;
FIG. 2 is a TGA pattern of an amorphous form of a compound of formula I;
FIG. 3 is an mDSC pattern of an amorphous form of a compound of formula I;
FIG. 4 is an XRPD pattern of a crystalline form A of a compound of formula I;
FIG. 5 is a TGA pattern of a crystalline form A of a compound of formula I;
FIG. 6 is a DSC pattern of a crystalline form A of a compound of formula I;
FIG. 7 is a DVS profile of a crystalline form A of a compound of formula I;
FIG. 8 is an XRPD pattern of a crystalline form B of a compound of formula I;
FIG. 9 is a TGA pattern of a crystalline form B of a compound of formula I;
FIG. 10 is a DSC pattern of a crystalline form B of a compound of formula I;
FIG. 11 is a DVS profile of a crystalline form B of a compound of formula I;
FIG. 12 is an XRPD pattern of a crystalline form C of a compound of formula I;
FIG. 13 is an XRPD pattern of a toluene solvate of a compound of formula I;
FIG. 14 is a TGA profile of a toluene solvate of a compound of formula I;
FIG. 15 is a DSC pattern of a toluene solvate of a compound of formula I;
FIG. 16 is a ¹H-NMR spectrum of a toluene solvate of a compound of formula I;
FIG. 17 is an XRPD pattern of a crystalline form E of a compound of formula I;
FIG. 18 is a TGA pattern of a crystalline form E of a compound of formula I;
FIG. 19 is a DSC pattern of a crystalline form E of a compound of formula I;
FIG. 20 is an XRPD pattern of a crystalline form F of a compound of formula I;
FIG. 21 is a TGA pattern of a crystalline form F of a compound of formula I;
FIG. 22 is a DSC pattern of a crystalline form F of a compound of formula I;
FIG. 23 is an XRPD pattern of a crystalline form G of a compound of formula I;
FIG. 24 is a TGA pattern of a crystalline form G of a compound of formula I;
FIG. 25 is a DSC pattern of a crystalline form G of a compound of formula I;
FIG. 26 is an XRPD pattern of an *N*-methyl-2-pyrrolidone solvate of a compound of formula I;
FIG. 27 is a TGA pattern of an *N*-methyl-2-pyrrolidone solvate of a compound of formula I;
FIG. 28 is a DSC pattern of an *N*-methyl-2-pyrrolidone solvate of a compound of formula I;
FIG. 29 is a ¹H-NMR spectrum of an *N*-methyl-2-pyrrolidone solvate of a compound of formula I;
FIG. 30 is an XRPD pattern of a DMF solvate of a compound of formula I;
FIG. 31 is a TGA pattern of a DMF solvate of a compound of formula I;
FIG. 32 is a DSC pattern of a DMF solvate of a compound of formula I;
FIG. 33 is a ¹H-NMR spectrum of a DMF solvate of a compound of formula I;
FIG. 34 is an XRPD pattern of a crystalline form K of a compound of formula I;
FIG. 35 is a DSC pattern of a crystalline form K of a compound of formula I;
FIG. 36 is an XPRD spectrum of a crystalline form A of a hydrochloride salt of a compound of formula I;
FIG. 37 is a TGA pattern of a crystalline form A of a hydrochloride salt of a compound of formula I;
FIG. 38 is a DSC pattern of a crystalline form A of a hydrochloride salt of a compound of formula I;
FIG. 39 is a DVS profile of a crystalline form A of a hydrochloride salt of a compound of formula I;
FIG. 40 is an XPRD pattern of a crystalline form A sample of a hydrochloride salt of a compound of formula I after DVS;
FIG. 41 is an XRPD pattern of an ethanol solvate of a hydrochloride salt of a compound of formula I;
FIG. 42 is a TGA pattern of an ethanol solvate of a hydrochloride salt of a compound of formula I;
FIG. 43 is a DSC pattern of an ethanol solvate of a hydrochloride salt of a compound of formula I;
FIG. 44 is an XRPD pattern of an isopropanol solvate of a hydrochloride salt of a compound of formula I;
FIG. 45 is a TGA pattern of an isopropanol solvate of a hydrochloride salt of a compound of formula I;
FIG. 46 is a DSC pattern of an isopropanol solvate of a hydrochloride salt of a compound of formula I;
FIG. 47 is an XPRD pattern of a crystalline form A of a sulfate salt of a compound of formula I;
FIG. 48 is a TGA pattern of a crystalline form A of a sulfate salt of a compound of formula I;
FIG. 49 is a DSC pattern of a crystalline form A of a sulfate salt of a compound of formula I;
FIG. 50 is a DVS profile of a crystalline form A of a sulfate salt of a compound of formula I;
FIG. 51 is an XPRD pattern of a crystalline form A sample of a sulfate salt of a compound of formula I after DVS;
FIG. 52 is an XRPD pattern of a crystalline form B of a sulfate salt of a compound of formula I;
FIG. 53 is a TGA pattern of a crystalline form B of a sulfate salt of a compound of formula I;
FIG. 54 is a DSC pattern of a crystalline form B of a sulfate salt of a compound of formula I;
FIG. 55 is an XRPD pattern of a crystalline form C of a sulfate salt of a compound of formula I;
FIG. 56 is an XRPD pattern of a toluene solvate of a sulfate salt of a compound of formula I;
FIG. 57 is a TGA pattern of a toluene solvate of a sulfate salt of a compound of formula I;
FIG. 58 is a DSC pattern of a toluene solvate of a sulfate salt of a compound of formula I;
FIG. 59 is an XRPD pattern of a crystalline form A of a p-toluenesulfonate salt of a compound of formula I;
FIG. 60 is a TGA pattern of a crystalline form A of a p-toluenesulfonate salt of a compound of formula I;
FIG. 61 is a DSC pattern of a crystalline form A of a p-toluenesulfonate salt of a compound of formula I;
FIG. 62 is an XRD pattern of a crystalline form A of a benzenesulfonate salt of a compound of formula I;
FIG. 63 is a TGA pattern of a crystalline form A of a benzenesulfonate salt of a compound of formula I;
FIG. 64 is a DSC pattern of a crystalline form A of a benzenesulfonate salt of a compound of formula I;
FIG. 65 is an XRPD pattern of a crystalline form A of a methanesulfonate salt of a compound of formula I;
FIG. 66 is a TGA pattern of a crystalline form A of a methanesulfonate salt of a compound of formula I;
FIG. 67 is a DSC pattern of a crystalline form A of a methanesulfonate salt of a compound of formula I;
FIG. 68 is a DVS profile of a crystalline form A of a methanesulfonate salt of a compound of formula I;
FIG. 69 is an XRPD pattern of a crystalline form A sample of a methanesulfonate salt of a compound of formula I after DVS;
FIG. 70 is an XRPD pattern of an amorphous form of a methanesulfonate salt of a compound of formula I;
FIG. 71 is a TGA pattern of an amorphous form of a methanesulfonate salt of a compound of formula I;
FIG. 72 is an mDSC pattern of an amorphous form of a methanesulfonate salt of a compound of formula I;
FIG. 73 is an XRPD pattern of a methanol solvate of a methanesulfonate salt of a compound of formula I;
FIG. 74 is a TGA pattern of a methanol solvate of a methanesulfonate salt of a compound of formula I;
FIG. 75 is a DSC pattern of a methanol solvate of a methanesulfonate salt of a compound of formula I;
FIG. 76 is an XRPD pattern of a 1,4-dioxane solvate of a methanesulfonate salt of a compound of formula I;
FIG. 77 is a TGA pattern of a 1,4-dioxane solvate of a methanesulfonate salt of a compound of formula I; and
FIG. 78 is a DSC pattern of a 1,4-dioxane solvate of a methanesulfonate salt of a compound of formula I.

### DETAILED DESCRIPTION

In the following examples, the experimental procedures were performed according to conventional conditions or conventional test conditions, and the compounds used in the examples were commercially available or prepared in-house. The compound of formula I was prepared as the method described in WO2021011713A1. All solvents used in the present invention are commercially available and can be used without further purification.

Unless otherwise stated, in the present invention, the 88% acetone is a mixed solvent of acetone and water in a volume ratio of 4:1.

Unless otherwise stated, in the present invention, each example is performed at room temperature, which is generally 20-30 °C, preferably 25 °C.

Unless otherwise stated, in the present invention, the overnight stirring is generally performed for a period of 12-16 h, preferably 14 h.

The Chinese references for the English abbreviations of the solvents selected in the present invention are as follows:

**Table 38**

| **In English** | **In Chinese** | **In English** | **In Chinese** |
|---|---|---|---|
| MeOH | Methanol | EtOAc | Ethyl acetate |
| EtOH | Ethanol | DCM | Dichloromethane |
| IPA | Isopropanol | MTBE | Methyl *tert*-butyl ether |
| 2-Me THF | 2-methyl-tetrahydrofuran | DMF | *N*,*N*-dimethylformamide |
| THF | Tetrahydrofuran | NMP | *N*-methyl-2-pyrrolidone |
| MIBK | Methyl isobutyl ketone | FA | Methyl acetate |

In the present invention, the XRPD test parameters are set as follows:

**Table 39**

| **Parameter** | **Instrument 1** |
|---|---|
| Model | Lynxeye detector |
| X-ray | Cu, Kα, Kα (Å):1.54 |
| X-ray light tube settings | 45 kV, 40 mA |
| Step length (° 2θ) | 0.02 |
| Scanning speed (s/step) | 3-40 |
| Scanning range (° 2θ) | 0.1 |

In the present invention, the DVS test parameters are set as follows:

**Table 40**

| **Parameter** | | **TGA** |
|---|---|---|
| Operating temperature (°C) | | 25 |
| Equilibration standard (dm/dt in 5 min) | | 0.01 %wt |
| Maximum equilibration time (min) | | 180 min |
| Hygroscopic range | | 0-95-0%RH |
| Data acquisition interval (min) | | 2 |
| Hygroscopic gradient | 0-90% | 10%RH |
| | 90-95% | 5%RH |

TGA and DSC patterns were acquired on a TA Q5000/5500 thermogravimetric analyzer and a TA Q2000/2500 differential scanning calorimeter, respectively. The parameters are set as follows:

**Table 41. DSC and TGA test parameters**

| **Parameter** | **TGA** | **DSC** |
|---|---|---|
| Method | Linear heating | Linear heating |
| Sample dish | Aluminum dish, open | Aluminum pan, gland/non-gland |
| Temperature range | Room temperature-setting endpoint temperature | 25 °C-setting endpoint temperature |
| Heating rate (C/min) | 10 | 10 |
| Protective gas | Nitrogen | Nitrogen |

In the present invention, the mDSC test parameters are set as follows: amplitude: ±1 °C, modulation period: 60s, heating rate: 3 °C/min, and temperature range: 25-350 °C.

In the present invention, ¹H-NMR test was performed using Qone-WNMR-I-AS400. The parameters are set as follows: solvent: deuterated DMSO, number of scanning: 18, temperature: 293.4 K, and magnetic field strength: 400 MHz.

In the present invention, the HPLC (high performance liquid chromatography) test parameters are set as follows:

**Table 42**

| | | | |
|---|---|---|---|
| Chromatographic column | Waters-Xselect CSHTM C18 (150mm ×4.6 mm, 3.5µm) | | |
| Column temperature | 30 °C | | |
| Flow rate | 1.0 mL/min | | |
| Injection volume | 10 µL | | |
| Detector | UV | | |
| Detection wavelength | 254 nm | | |
| Run time | 33 min | | |
| Mobile phase A | 10 mmol/L monopotassium phosphate + 0.05% perchloric acid (pH = 4.4 ± 0.1)/ACN = 9/1 (v/v) | | |
| Mobile phase B | Acetonitrile | | |
| Elution gradient | Time | %A | %B |
| | 0.0 | 70 | 30 |
| | 25.0 | 35 | 65 |
| | 25.1 | 70 | 30 |
| | 33.0 | 70 | 30 |

### Example 1:

Preparation of amorphous form of compound of formula I:
5 g of a crystalline form B of a compound of formula I was dissolved in 500 mL of dichloromethane/methanol (2/1 v/v) with stirring at 800 rpm for complete dissolution to prepare a 10 mg/mL solution. The solution was subjected to spray drying, with an air inlet temperature of 85 °C and an outlet temperature of 55 °C, to obtain 3.7 g of powder, which was subjected to vacuum drying at 50 °C for 4 h to obtain the final product.

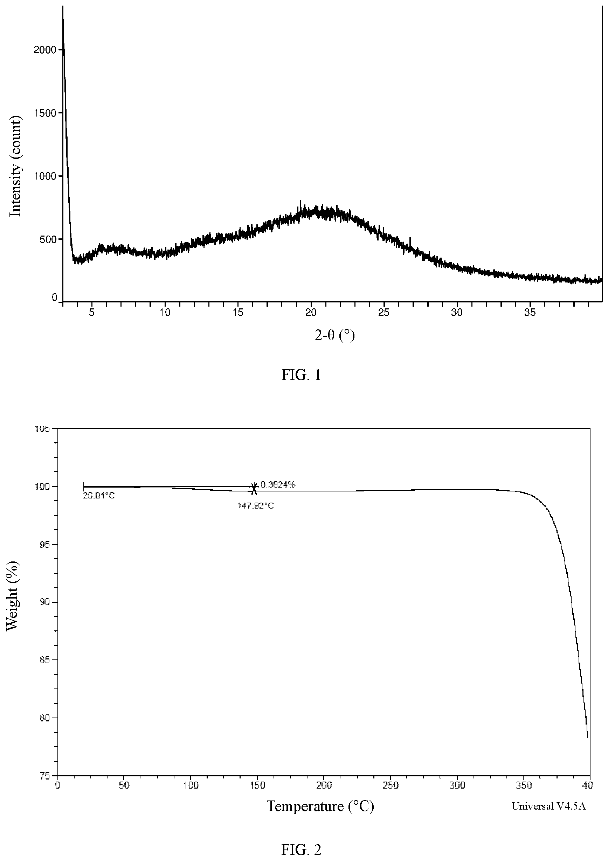

Characterization of amorphous form of compound of formula I:
1) the XRPD characterization results are shown in FIG. 1;
2) the TGA characterization results are shown in FIG. 2; and
3) the DSC characterization results are shown in FIG. 3, with a glass transition temperature of 178.67 °C for the amorphous form of the compound of formula I.

### Example 2:

Preparation of crystalline form A of compound of formula I:
0.2 mL of solvent such as methanol or acetonitrile was added to 50 mg of an amorphous form sample of a compound of formula I, and the mixture was suspended and stirred at room temperature or 50 °C for 2 days. The resulting sample was quickly centrifuged to remove a supernatant, and the resulting solid was subjected to vacuum drying at room temperature.

Characterization of crystalline form A of compound of formula I:
1) the XRPD characterization results are shown in FIG. 4 and Table 1;
2) the TGA characterization results are shown in FIG. 5;
3) the DSC characterization results are shown in FIG. 6; and
4) the DVS characterization results are shown in FIG. 7, with a hygroscopic weight gain of 0.44% at 80% RH, showing slight hygroscopicity.

### Example 3:

Preparation of crystalline form B of compound of formula I:
0.6 mL of solvent such as ethyl acetate or acetone was added to 60 mg of a crystalline form A of a compound of formula I, and the mixture was suspended and stirred at room temperature or 50 °C for 2 days. The resulting sample was quickly centrifuged to remove a supernatant, and the resulting solid was subjected to vacuum drying at room temperature.

Characterization of crystalline form B of compound of formula I:
1) the XRPD characterization results are shown in FIG. 8 and Table 2;
2) the TGA characterization results are shown in FIG. 9;
3) the DSC characterization results are shown in FIG. 10; and
4) the DVS characterization results are shown in FIG. 11, with a hygroscopic weight gain of 0.84% at 80% RH, showing slight hygroscopicity.

### Example 4:

Preparation of crystalline form C of compound of formula I:
3 mL of tetrahydrofuran was added to 100 mg of a crystalline form A of a compound of formula I, and the mixture was suspended and stirred at room temperature for 1 day. The resulting sample was quickly centrifuged to remove a supernatant, and the resulting solid was directly tested, wherein the crystalline form C sample was a wet product and was not subjected to a TGA-DSC test.

Characterization of crystalline form C of compound of formula I: the XRPD characterization results are shown in FIG. 12 and Table 3.

### Example 5:

Preparation of toluene solvate of compound of formula I:
0.6 mL of toluene was added to 60 mg of a crystalline form B of a compound of formula I, and the mixture was suspended and stirred at room temperature for 2 days. The resulting sample was quickly centrifuged to remove a supernatant, and the resulting solid was subjected to vacuum drying at room temperature and directly tested.

Characterization of toluene solvate of compound of formula I:
1) the XRPD characterization results are shown in FIG. 13 and Table 4;
2) the TGA characterization results are shown in FIG. 14;
3) the DSC characterization results are shown in FIG. 15; and
4) the ¹H-NMR characterization results are shown in FIG. 16, showing the remaining of toluene with a weight percentage of 3.27%.

### Example 6:

Preparation of crystalline form E of compound of formula I:
0.6 mL of tert-butyl methyl ether was added to 60 mg of a crystalline form B of a compound of formula I, and the mixture was suspended and stirred at room temperature for 2 days. The resulting sample was quickly centrifuged to remove a supernatant, and the resulting solid was subjected to vacuum drying at room temperature and directly tested.

Characterization of crystalline form E of compound of formula I:
1) the XRPD characterization results are shown in FIG. 17 and Table 5;
2) the TGA characterization results are shown in FIG. 18; and
3) the DSC characterization results are shown in FIG. 19.

### Example 7:

Preparation of crystalline form F of compound of formula I:
0.6 mL of MeOH/H₂O (1:1, v/v) was added to 60 mg of a crystalline form B of a compound of formula I, and the mixture was suspended and stirred at room temperature for 2 days. The resulting sample was quickly centrifuged to remove a supernatant, and the resulting solid was subjected to vacuum drying at room temperature and directly tested.

Characterization of crystalline form F of the compound of formula I:
1) the XRPD characterization results are shown in FIG. 20 and Table 6;
2) the TGA characterization results are shown in FIG. 21; and
3) the DSC characterization results are shown in FIG. 22.

### Example 8: Preparation of crystalline form G of compound of formula I

0.2 mL of toluene was added to 40 mg of a crystalline form A of a compound of formula I, and the mixture was suspended and stirred at room temperature for 2 days. The resulting sample was quickly centrifuged to remove a supernatant, and the resulting solid was subjected to vacuum drying at room temperature and directly tested.

Characterization of crystalline form G of compound of formula I:
1) the XRPD characterization results are shown in FIG. 23 and Table 7;
2) the TGA characterization results are shown in FIG. 24; and
3) the DSC characterization results are shown in FIG. 25.

### Example 9:

Preparation of N-methyl-2-pyrrolidone solvate of compound of formula I:
0.2 mL of NMP was added to 50 mg of an amorphous form sample of a compound of formula I, and the mixture was suspended and stirred at room temperature for 2 days. The resulting sample was quickly centrifuged to remove a supernatant, and the resulting solid was subjected to vacuum drying at room temperature and directly tested.

Characterization of *N*-methyl-2-pyrrolidone solvate of compound of formula I:
1) the XRPD characterization results are shown in FIG. 26 and Table 8;
2) the TGA characterization results are shown in FIG. 27, with no weight loss before 114 °C, and with a weight loss of 15.56% between 114.32 °C and 209.84 °C, which is an NMP residue;
3) the DSC characterization results are shown in FIG. 28; and
4) the ¹H-NMR characterization results are shown in FIG. 29, showing the presence of solvent NMP residue with a molar ratio of 1:0.96 to the compound of formula I and with a weight percentage of 15.3%.

Example 10: Preparation of DMF solvate of compound of formula I:
0.2 mL of DMF was added to 50 mg of an amorphous form sample of the compound of formula I, and the mixture was suspended and stirred at room temperature for 2 days. The resulting sample was quickly centrifuged to remove a supernatant, and the resulting solid was subjected to vacuum drying at room temperature and directly tested.

Characterization of DMF solvate of compound of formula I:
1) the XRPD characterization results are shown in FIG. 30 and Table 9;
2) the TGA characterization results are shown in FIG. 31, with a weight loss of 3.381% before 196.62 °C, which is a DMF residue;
3) the DSC characterization results are shown in FIG. 32; and
4) the ¹H-NMR characterization results are shown in FIG. 33, showing the presence of solvent DMF residue with a molar ratio of 1:0.83 to the compound of formula I and with a weight percentage of 10.3%.

### Example 11:

Preparation of crystalline form K of compound of formula I:
10 mg of a crystalline form B of a compound of formula I was placed on a TGA platinum pan, heated to 280 °C, maintained for 2 min, and cooled to room temperature to obtain a sample, namely the crystalline form K.

Characterization of crystalline form K of compound of formula I:
1) the XRPD characterization results are shown in FIG. 34 and Table 10; and
2) the DSC characterization results are shown in FIG. 35.

### Example 12:

Preparation of crystalline form A of hydrochloride salt of compound of formula I:
50.2 mg of a crystalline form A of a compound of formula I was weighed and added to a 4 mL glass flask, and 2 mL of tetrahydrofuran was added to obtain a suspension. 1.1 equivalent (105 µL, 1 M HCl-THF solution) of hydrochloric acid was added to obtain a clear solution, which was stirred at room temperature for half an hour. The solid was precipitated, and the mixture was stirred overnight and quickly centrifuged. The resulting solid precipitate was subjected to vacuum drying at 40 °C to obtain a white solid.

Characterization of crystalline form A of hydrochloride salt of compound of formula I:
1) the XRPD characterization results are shown in FIG. 36 and Table 11;
2) the TGA characterization results are shown in FIG. 37, with a weight loss of 1.03% before heating to 108.66 °C, which is presumed to be a tetrahydrofuran solvent residue, and with a weight loss of 6.68% during heating from 108 °C to 209.57 °C, which is presumed to be a loss of hydrochloric acid (1 eq of hydrochloric acid accounted for about 6.34%);
3) the DSC characterization results are shown in FIG. 38;
4) the DVS characterization results are shown in FIG. 39, with a hygroscopic weight gain of 0.31% at 80% RH, showing slight hygroscopicity; and
5) the crystalline form did not change before and after a hygroscopic test, with the XRPD characterization results shown in FIG. 40.

### Example 13:

Preparation of ethanol solvate of hydrochloride salt of compound of formula I:
0.4 mL of ethanol was added to 40 mg of a crystalline form A of a hydrochloride salt of a compound of formula I, and the mixture was suspended and stirred at 30 °C for 4 days. The resulting sample was quickly centrifuged to remove a supernatant, and the resulting solid was subjected to vacuum drying at room temperature and directly tested.

Characterization of ethanol solvate of hydrochloride salt of compound of formula I:
1) the XRPD characterization results are shown in FIG. 41 and Table 12;
2) the TGA characterization results are shown in FIG. 42; and
3) the DSC characterization results are shown in FIG. 43.

### Example 14:

Preparation of isopropanol solvate of hydrochloride salt of compound of formula **I:**
0.3 mL of isopropanol was added to 40 mg of a crystalline form A of a hydrochloride salt of a compound of formula I, and the mixture was suspended and stirred at 30 °C for 4 days. The resulting sample was quickly centrifuged to remove a supernatant, and the resulting solid was subjected to vacuum drying at room temperature and directly tested.

Characterization of isopropanol solvate of hydrochloride salt of compound of formula **I:**
1) the XRPD characterization results are shown in FIG. 44 and Table 13;
2) the TGA characterization results are shown in FIG. 45; and
3) the DSC characterization results are shown in FIG. 46.

### Example 15:

Preparation of crystalline form A of sulfate salt of compound of formula I:
49.4 mg of a crystalline form A of a compound of formula I was weighed and added to a 4 mL glass flask, and 1 mL of tetrahydrofuran was added to obtain a suspension. 1.1 equivalent (105 µL, 1 M H₂SO₄-THF solution) of sulfuric acid was added to obtain a clear solution, which was stirred at room temperature for half an hour, followed by the addition of 0.6 mL of an anti-solvent n-heptane. The solid was precipitated, and the mixture was stirred overnight and quickly centrifuged. The resulting solid precipitate was subjected to vacuum drying to obtain a white solid.

Characterization of crystalline form A of sulfate salt of compound of formula I:
1) the XRPD characterization results are shown in FIG. 47 and Table 14;
2) the TGA characterization results are shown in FIG. 48;
3) the DSC characterization results are shown in FIG. 49, showing an endothermic peak at 210.46 °C that is presumed to be the melting of a sulfate salt with concomitant degradation;
4) the DVS characterization results are shown in FIG. 50, with a hygroscopic weight gain of 1.43% at 25 °C and 80% RH, showing slight hygroscopicity; and
5) the crystalline form did not change before and after hygroscopic test, with the XRPD characterization results shown in FIG. 51.

### Example 16:

Preparation of crystalline form B of sulfate salt of compound of formula **I**:
1.51 g of crystalline form A of the compound of formula I was weighed and added to a 40 mL glass flask, and 30 mL of tetrahydrofuran was added to obtain a suspension. 1.2 equivalent (342.9 mg, diluted with 5 mL of THF) of sulfuric acid to obtain a clear solution, which was stirred at room temperature for 4 h to precipitate a small amount of solid. The mixture was stirred overnight and quickly centrifuged. The resulting solid precipitate was subjected to vacuum drying to obtain a white solid.

Characterization of crystalline form B of sulfate salt of compound of formula I:
1) the XRPD characterization results are shown in FIG. 52 and Table 15;
2) the TGA characterization results are shown in FIG. 53; and
3) the DSC characterization results are shown in FIG. 54.

### Example 17:

Preparation of crystalline form C of sulfate salt of compound of formula **I**:
0.5 g of crystalline form A of the compound of formula **I** was weighed and added to a 40 mL glass flask, and 10 mL of tetrahydrofuran was added to obtain a suspension. 1.2 equivalent (107 mg, diluted with 1 mL of THF) of sulfuric acid to obtain a clear solution, which was stirred at room temperature for 4 h. A small amount of solid was precipitate, and the mixture was stirred overnight and quickly centrifuged, followed by the addition of n-heptane. The mixture was stirred overnight, filtered, and washed with n-heptane. The resulting solid precipitate was subjected to vacuum drying to obtain a white solid.

Characterization of crystalline form C of sulfate salt of compound of formula I: the XRPD characterization results are shown in FIG. 55 and Table 16.

### Example 18:

Preparation of toluene solvate of sulfate salt of compound of formula **I:**
7 mL of methanol was added to 250 mg of a crystalline form A of a sulfate salt of a compound of formula **I,** and sonication was performed at room temperature to obtain a clear solution, which was filtered through a 0.22 µm nylon filter membrane to obtain a filtrate. 1 mL of filtrate was added to a 40 mL glass flask, and 6 mL of toluene solvent was added to obtain a precipitate. The resulting sample was quickly centrifuged to remove a supernatant, and the resulting solid was subjected to vacuum drying at room temperature and directly tested.

Characterization of toluene solvate of sulfate salt of compound of formula I:
1) the XRPD characterization results are shown in FIG. 56 and Table 17;
2) the TGA characterization results are shown in FIG. 57; and
3) the DSC characterization results are shown in FIG. 58.

### Example 19:

Preparation of crystalline form A of p-toluenesulfonate salt of compound of formula **I:**
50.7 mg of a crystalline form A of a compound of formula I was weighed and added to a 4 mL glass flask, and 1 mL of tetrahydrofuran was added to obtain a suspension. 1.1 equivalent (23.8 mg) of *p*-toluenesulfonic acid was added to obtain a clear solution, which was stirred at room temperature for half an hour, followed by the addition of 0.6 mL of an anti-solvent *n*-heptane. The solid was precipitated, and the mixture was stirred overnight and quickly centrifuged. The resulting solid was subjected to vacuum drying to obtain a yellow solid. Characterization of crystalline form A of *p*-toluenesulfonate salt of compound of formula I:
1) the XRPD characterization results are shown in FIG. 59 and Table 18;
2) the TGA characterization results are shown in FIG. 60; and
3) the DSC characterization results are shown in FIG. 61.

### Example 20:

Preparation of crystalline form A of benzenesulfonate salt of compound of formula I:
52.8 mg of a crystalline form A of a compound of formula I was added to a 4 mL glass flask, and 1 mL of tetrahydrofuran was added to obtain a suspension. 1.1 equivalent (18.1 mg) of benzenesulfonic acid was added to obtain a clear solution, which was stirred at room temperature for half an hour, followed by the addition of 0.8 mL of an anti-solvent n-heptane. The solid was precipitated, and the mixture was stirred overnight and quickly centrifuged. The resulting solid precipitate was subjected to vacuum drying to obtain a yellow solid. Characterization of crystalline form A of benzenesulfonate salt of compound of formula I:
1) the XRPD characterization results are shown in FIG. 62 and Table 19;
2) the TGA characterization results are shown in FIG. 63; and
3) the DSC characterization results are shown in FIG. 64.

### Example 21:

Preparation of crystalline form A of methanesulfonate salt of compound of formula I:
50.1 mg of crystalline form A of compound of formula I was weighed and added to a 4 mL glass flask, and 2 mL of tetrahydrofuran was added to obtain a suspension. 1.1 equivalent (10.8 mg) of methanesulfonic acid was added to obtain a clear solution, which was stirred at room temperature for half an hour. The solid was precipitated, and the mixture was stirred overnight and quickly centrifuged. The resulting solid was subjected to vacuum drying to obtain a light yellow solid.

Preparation of crystalline form A of methanesulfonate salt of compound of formula I:
1) the XRPD characterization results are shown in FIG. 65 and Table 20;
2) the TGA characterization results are shown in FIG. 66;
3) the DSC characterization results are shown in FIG. 67, showing the presence of an endothermic peak at 250.75 °C that is presumed to be the melting of a methanesulfonate salt with concomitant degradation;
4) the DVS characterization results are shown in FIG. 68, with a hygroscopic weight gain of 0.84% at 80% RH, showing slight hygroscopicity; and
5) the crystalline form did not change before and after hygroscopic test, with the XRPD characterization results shown in FIG. 69.

### Example 22:

Preparation of amorphous form of methanesulfonate salt of compound of formula I:
5 g of a crystalline form A of a methanesulfonate salt of a compound of formula I was added to 250 mL of DCM/MeOH (2-1 (v/v)) solution, and sonication was performed for 2 min to completely dissolve the starting material to obtain a light yellow clear solution, which was subjected to spray drying at an air inlet temperature of 90 °C. The resulting solid powder was subjected to vacuum drying at 50 °C for 2 h to obtain 3.92 g of light yellow powder.

Characterization of amorphous form of methanesulfonate salt of compound of formula I:
1) the XRPD characterization results are shown in FIG. 70;
2) the TGA characterization results are shown in FIG. 71; and
3) the mDSC characterization results are shown in FIG. 72, with a glass transition temperature of 139.32 °C for the amorphous form of the compound of formula I.

### Example 23:

Preparation of methanol solvate of methanesulfonate salt of compound of formula I:
0.2 mL of methanol was added to 40 mg of an amorphous form sample of a methanesulfonate salt of a compound of formula I, and the mixture was suspended and stirred at 40 °C for 6 days. The resulting sample was quickly centrifuged to remove a supernatant, and the resulting solid was subjected to vacuum drying at room temperature and directly tested.

Characterization of methanol solvate of methanesulfonate salt of compound of formula I:
1) the XRPD characterization results are shown in FIG. 73 and Table 21;
2) the TGA characterization results are shown in FIG. 74; and
3) the DSC characterization results are shown in FIG. 75.

### Example 24:

Preparation of 1,4-dioxane solvate of methanesulfonate salt of compound of formula I:
0.2 mL of 1,4-dioxane was added to 40 mg of an amorphous form sample of a methanesulfonate salt of a compound of formula I, and the mixture was suspended and stirred at 40 °C for 6 days. The resulting sample was quickly centrifuged to remove a supernatant, and the resulting solid was subjected to vacuum drying at room temperature and directly tested.

Characterization of 1,4-dioxane solvate of methanesulfonate salt of compound of formula I:
1) the XRPD characterization results are shown in FIG. 76 and Table 22;
2) the TGA characterization results are shown in FIG. 77; and
3) the DSC characterization results are shown in FIG. 78.

### Example 25: Polymorph screening test with crystalline form A of compound of formula I as starting material (suspension method)

30-40 mg of a crystalline form A of a compound of formula I was weighed and placed in a 2 mL glass flask, and 200 µL of solvent was added to obtain a suspension, which was stirred at 40 °C for 2 days. The sample was quickly centrifuged (6000 rpm, 5 min) to remove a supernatant. The resulting precipitate was subjected to vacuum drying (40 °C, -0.1 MPa) for 20 h, followed by physical characterization. The results are shown in Table 23.

**Table 23. Polymorph screening results with a crystalline form A as a starting material**

| **No.** | **Solvent** | **Crystalline form results** |
|---|---|---|
| - | Initial crystalline form | Crystalline form A |
| 1 | MeOH | Crystalline form A |
| 2 | EtOH | Crystalline form A |
| 3 | IPA | Crystalline form A |
| 4 | ACN | Crystalline form A |
| 5 | Acetone | Crystalline form B |
| 6 | EtOAc | Crystalline form B |
| 7 | THF | Crystalline form B |
| 8 | Toluene | Crystalline form G |
| 9 | MeOH-H₂O=1-1 | Crystalline form A |
| 10 | EtOH-H₂O=1-1 | Crystalline form A |
| 11 | ACN-H₂O=1-1 | Crystalline form A |
| 12 | 88% acetone | Crystalline form B |

### Example 26: Polymorph screening test with crystalline form B of compound of formula I as starting material (suspension method)

60 mg of a crystalline form B of a compound of formula I was weighed and placed in a 2 mL glass flask, and 200 µL of solvent was added to obtain a suspension, which was stirred at 25 °C or 50 °C for 2 days and quickly centrifuged (6000 rpm, 5 min) to remove a supernatant. The resulting precipitate was subjected to vacuum drying at 40 °C for 20 h, followed by physical characterization.

**Table 24. Polymorph screening results with a crystalline form B as a starting material**

| **No.** | **Solvent** | **Crystalline form results 25 °C** | **Crystalline form results 50 °C** |
|---|---|---|---|
| - | Initial crystalline form | Crystalline form B | |
| 1 | MeOH | Crystalline form A | Crystalline form A |
| 2 | EtOH | Crystalline form B | Crystalline form A |
| 3 | ACN | Crystalline form A | Crystalline form A |
| 4 | Acetone | Crystalline form B | Crystalline form B |
| 5 | EtOAc | Crystalline forms C + B | Crystalline form B |
| 6 | THF | Crystalline form B | Crystalline form B |
| 7 | Heptane | Crystalline form B | Crystalline forms G + B |
| 8 | 1,4-dioxane | Crystalline forms C + B | Crystalline forms C + B |
| 9 | H₂O | Crystalline form B | Crystalline form B |
| 10 | MeOH-H₂O=3-1 | Crystalline form A | Crystalline forms A + B |
| 11 | EtOH-H₂O=3-1 | Crystalline form B | Crystalline form A |
| 12 | ACN-H₂O=1-1 | Crystalline form A | Crystalline form A |
| 13 | Acetone-H₂O = 1-2 | Crystalline form B | Crystalline form B |
| 14 | IPA | Crystalline forms C + B | Crystalline forms C + B |
| 15 | Toluene | Toluene solvate | Crystalline form B + toluene solvate |
| 16 | MIBK | Crystalline forms C + B | Crystalline forms C + B |
| 17 | 2-Me THF | Crystalline forms C + B | Crystalline forms C + B |
| 18 | MTBE | Crystalline form E | Crystalline form G |
| 19 | MeOH-H₂O 1-1 | Crystalline form F | Crystalline form A |
| 20 | EtOH-H₂O 1-1 | Crystalline form B | Crystalline form A |

Example 27: Polymorph screening test with compound of formula I as starting material (suspension method) 50 mg of a crystalline form B of a compound of formula I was weighed and added to a 4 mL glass flask, and 500 µL of solvent (DMF and NMP) was added. No clear solution was obtained, and the sample was still undissolved after 1 mL of solvent was added. Then, 500 µL of poor solvent (acetone, ACN, MeOH, EtOAc, THF, or H₂O) was added, and 40 mg of the sample was added to perform an experiment by a suspension method. All suspension samples were stirred at 50 °C for 2 days and quickly centrifuged (6000 rpm, 5 min) to remove a supernatant. The resulting solid was subjected to vacuum drying at 40 °C for 20 h, followed by physical characterization.

The compound of formula I was subjected to polymorph screening with DCM as a good solvent and with MeOH, EtOH, THF, or EA as an anti-solvent, and was dissolved in both DCM and MeOH systems. The suspension sample was stirred for 2 days and quickly centrifuged to remove a supernatant. The resulting precipitate was subjected to vacuum drying at 25 °C for 20 h, followed by physical characterization.

**Table 25. Polymorph screening results for the compound of formula I in a mixed solvent of NMP and DMF 50 °C, suspension method**

| **No.** | **Solvent** | **Crystalline form** |
|---|---|---|
| 1 | NMP/acetone = 1/3 | *N*-methyl-2-pyrrolidone solvate |
| 2 | NMP/MeOH=1/3 | *N*-methyl-2-pyrrolidone solvate |
| 3 | NMP/ACN=1/3 | *N*-methyl-2-pyrrolidone solvate |
| 4 | NMP/EtOAc=1/3 | *N*-methyl-2-pyrrolidone solvate |
| 5 | NMP/THF=1/3 | *N*-methyl-2-pyrrolidone solvate |
| 6 | NMP/H₂O=1/3 | Crystalline forms A + B |
| 7 | DMF/H₂O=1/3 | Crystalline form B + DMF solvate |
| 8 | DMF | Crystalline form B + DMF solvate |
| 9 | NMP | *N*-methyl-2-pyrrolidone solvate |
| 10 | DCM | Crystalline form A |
| 11 | DCM/EtOH=2-1 | Crystalline form A |
| 12 | DCM/THF=1-1 | Crystalline form A |
| 13 | DCM/EA=1-1 | Crystalline form A |

Example 28: Polymorph screening with amorphous form of compound of formula I as starting material (suspension method)
60 mg of an amorphous form of a compound of formula I was weighed and placed in a 2 mL glass flask, and 200 µL of solvent was added to obtain a suspension, which was stirred at 25 °C or 50 °C for 2 days and quickly centrifuged (6000 rpm, 5 min) to remove a supernatant. The resulting precipitate was subjected to vacuum drying at room temperature for 20 h, followed by physical characterization.
Table 26. Polymorph screening results with an amorphous form sample as a starting material (suspension method)

| **No.** | **Solvent** | **Crystalline form results 25°C** | **Crystalline form results 50°C** |
|---|---|---|---|
| - | Initial crystalline form | Amorphous form | |
| 1 | MeOH | Crystalline form A | Crystalline form A |
| 2 | EtOH | Crystalline form B | Crystalline form A |
| 3 | ACN | Crystalline form A | Crystalline form A |
| 4 | Acetone | Crystalline forms C + B | Crystalline forms C + B |
| 5 | EtOAc | Crystalline forms C + B | Crystalline form B |
| 6 | THF | Crystalline forms C + B | Crystalline forms C + B |
| 7 | Heptane | Amorphous form | Crystalline form G |
| 8 | 1,4-dioxane | Crystalline forms C + B | Crystalline forms C + B |
| 9 | H₂O | Crystalline form G | Crystalline form G |
| 10 | MeOH-H₂O=3-1 | Crystalline form A | Crystalline form A |
| 11 | EtOH-H₂O=3-1 | Crystalline form A | Crystalline form A |
| 12 | ACN-H₂O=1-1 | Crystalline form A | Crystalline form A |
| 13 | Acetone-H₂O = 1-2 | Crystalline form B | Crystalline form B |
| 14 | IPA | Crystalline form A | Crystalline form A |
| 15 | Toluene | Crystalline form G | Crystalline form G |
| 16 | MIBK | Crystalline form B | Crystalline forms C + B |
| 17 | 2-Me THF | Crystalline form E | Crystalline forms G + B |
| 18 | MTBE | Crystalline form E | Crystalline form B |
| 19 | MeOH-H₂O 1-1 | Crystalline forms F + A | Crystalline form G |
| 20 | EtOH-H₂O 1-1 | Crystalline forms F + A | Crystalline form A |
| 21 | NMP | *N*-methyl-2-pyrrolidone solvate | *N*-methyl-2-pyrrolidone solvate |
| 22 | DMF | DMF solvate | DMF solvate |
| 23 | DCM | Crystalline form A | Crystalline form A |

Example 29: Polymorph screening study on hydrochloride salt of compound of formula I (suspension method) 30 mg of a crystalline form A of a hydrochloride salt of a compound of formula I was weighed and added to a 2 mL glass flask, and 200 µL of solvent was added to obtain a suspension, which was stirred at 40 °C for 3 days and quickly centrifuged (6000 rpm, 6 min) to remove a supernatant. The resulting precipitate was subjected to vacuum drying for 20 h.

**Table 27. Polymorph screening results for the hydrochloride salt of the compound of formula I**

| **No.** | **Solvent** | **Crystalline form results** |
|---|---|---|
| 1 | MeOH | Partial dissociation into crystalline form A |
| 2 | EtOH | Ethanol solvate of hydrochloride salt |
| 3 | IPA | Isopropanol solvate of hydrochloride salt |
| 4 | *n*-butanol | Crystalline form A of hydrochloride salt |
| 5 | ACN | |
| 6 | Acetone | |
| 7 | EtOAc | |
| 8 | Toluene | |
| 9 | 1,4-dioxane | |
| 10 | H₂O | |
| 11 | MeOH-H₂O=3-1 | Crystalline form A |
| 12 | EtOH-H₂O=3-1 | |
| 13 | 88% acetone | Crystalline form B |

Example 30: Polymorph screening study on sulfate salt of compound of formula I (suspension method, 40 °C) 30 mg of a crystalline form A of a sulfate form of the compound of formula I was weighed and added to a 2 mL glass flask, and 200 µL of solvent was added to obtain a suspension, which was stirred at 40 °C for 3 days and quickly centrifuged (6000 rpm, 6 min) to remove a supernatant. The resulting precipitate was subjected to vacuum drying for 20 h.

**Table 28. Polymorph screening test results for the sulfate salt of the compound of formula I**

| **No.** | **Solvent** | **Crystalline form results** |
|---|---|---|
| 1 | MeOH | Crystalline form A |
| 2 | EtOH | Sulfate salt forming into sulfate |
| 3 | IPA | Sulfate salt forming into sulfate |
| 4 | ACN | Crystalline form A of sulfate salt |
| 5 | Acetone | Crystalline form A of sulfate salt |
| 6 | EtOAc | Sulfate salt forming into sulfate |
| 7 | THF | Crystalline form A of sulfate salt |
| 8 | Toluene | Crystalline form A of sulfate salt |
| 9 | MeOH-H₂O=3:1 | Crystalline form A |
| 10 | 88% acetone | Crystalline form B |
| 11 | ACN-H₂O=1:1 | Crystalline form A |
| 12 | EtOH-H₂O=3:1 | Crystalline form A |
| 13 | DCM/MeOH=1:1 | Crystalline form A |

Example 31: Polymorph screening test of sulfate salt of compound of formula I (anti-solvent method) 250 mg of a crystalline form A of a sulfate salt of a compound of formula I was weighed and added to a 40 mL glass flask, and 7 mL of methanol was added. Sonication was performed for 1 min to obtain a clear solution, which was filtered through a 0.22 µm filter membrane and aliquoted into seven 8 mL glass flasks. An anti-solvent was added until precipitation occurred. The sample was stirred at room temperature for 1 day and quickly centrifuged. The resulting solid precipitate was subjected to vacuum drying.

**Table 29. Polymorph screening test results for the sulfate salt of the compound of formula I**

| **No.** | **Anti-solvent** | **Solution behavior** | **XRPD results** |
|---|---|---|---|
| 1 | IPA | 6 mL of a corresponding anti-solvent was added, and precipitation occurred in the solution | Crystalline form A + crystalline form B |
| 2 | MTBE | | Crystalline form A of sulfate salt |
| 3 | EtOAc | | Sulfate salt forming into sulfate |
| 4 | Toluene | | Possibly a toluene solvate of the sulfate salt |
| 5 | Acetone | No precipitation, open-to-air volatilization | Crystalline form B |
| 6 | Heptane | No precipitation, open-to-air volatilization | Crystalline form A |
| 7 | ACN | No precipitation, volatilization into oil | Oil, not tested |

Example 32: Polymorph screening study on methanesulfonate salt of compound of formula I (suspension method)
30 mg of a crystalline form A of a methanesulfonate salt was weighed and added to a 2 mL glass flask, and 200 µL of solvent was added to obtain a suspension, which was stirred at 40 °C for 3 days and quickly centrifuged (6000 rpm, 6 min) to remove a supernatant. The resulting precipitate was subjected to vacuum drying for 20 h.

**Table 30. Polymorph screening results for the methanesulfonate salt of the compound of formula I**

| **No.** | **Solvent** | **Crystalline form results** |
|---|---|---|
| - | Initial crystalline form | Crystalline form A of methanesulfonate salt |
| 1 | MeOH | Methanol solvate of methanesulfonate salt |
| 2 | EtOH | Crystalline form A of methanesulfonate salt |
| 3 | Acetone | Crystalline form A of methanesulfonate salt |
| 4 | ACN | Crystalline form A of methanesulfonate salt |
| 5 | EtOAc | Crystalline form A of methanesulfonate salt |
| 6 | H₂O | Crystalline form A |
| 7 | MeOH-H₂O 1-1 | Crystalline form A |
| 8 | ACN-H₂O 1-1 | Crystalline form A |

Example 33: Polymorph screening study on methanesulfonate salt of compound of formula I (anti-solvent method)

A certain amount of crystalline form A of a methanesulfonate salt of a compound of formula I was weighed and added to an 8 mL glass flask, and the following solvent was added to prepare a clear solution at a certain concentration, which was divided into 6 parts, with each part in 0.5 mL. Then, an anti-solvent was added until precipitation occurred, and the mixture was stirred at room temperature overnight and quickly centrifuged (6000 rpm, 6 min) to remove a supernatant. The resulting precipitate was subjected to vacuum drying for 20 h.

**Table 31. Polymorph screening results for the methanesulfonate salt of the compound of formula I**

| **Good solvent** | **Anti-solvent** | **Crystalline form results** |
|---|---|---|
| DCM/ MeOH=2-1 (20mg/mL) | Ethyl acetate | Methanol solvate of methanesulfonate salt |
| | *Tert*-butyl methyl ether | |
| | Acetonitrile | |
| | *n*-heptane | |
| | Cyclohexane | |
| | Methyl acetate | No precipitation |
| NMP (50mg/mL) | Ethyl acetate | Dissociation into a free base Presumably an NMP solvate |
| | *Tert*-butyl methyl ether | |
| | Acetonitrile | |
| | *n*-heptane | |
| | Dimethyl tetrahydrofuran | |
| | Methyl acetate | |
| DMF (50mg/mL) | Ethyl acetate | Crystalline form A of methanesulfonate salt |
| | *Tert*-butyl methyl ether | |
| | Acetonitrile | Crystalline form A |
| | *n*-heptane | No precipitation |
| | Dimethyl tetrahydrofuran | |
| | Methyl acetate | Dissociation into a free base |
| MeOH (10mg/mL) | Ethyl acetate | Methanol solvate of methanesulfonate salt |
| | *Tert*-butyl methyl ether | |
| | Acetonitrile | No precipitation |
| | *n*-heptane | |
| | Dimethyl tetrahydrofuran | |
| | Methyl acetate | |

Example 34: Polymorph screening study on methanesulfonate salt of compound of formula I (gas-liquid diffusion method)

A certain amount of crystalline form A of a methanesulfonate salt of a compound of formula I was weighed and added to an 8 mL glass flask, and the following solvent was added to prepare a clear solution at a certain concentration, which was divided into 6 parts, with each part in 0.5 mL in a 1.5 mL glass flask. Then, the solution was added to a 20 mL glass flask containing 2 mL of an anti-solvent (shown in the following table), and the flask was tightly capped at room temperature and placed in a fume hood for diffusion. When precipitation occurred, the flask was taken out, and the resulting precipitate was subjected to vacuum drying for 20 h.

**Table 32. Polymorph screening results for the methanesulfonate salt of the compound of formula I**

| **Good solvent** | **Anti-solvent** | **Crystalline form results** |
|---|---|---|
| NMP (50mg/mL) | EtOAc | Dissociation into a free base Possibly an NMP solvate |
| | MTBE | |
| | ACN | |
| | Heptane | |
| | 2-MeTHF | |
| | FA | |
| DMF (50mg/mL) | EtOAc | No precipitation |
| | MTBE | |
| | ACN | |
| | Heptane | |
| | 2-MeTHF | |
| | FA | |
| MeOH (10mg/mL) | EtOAc | Methanol solvate of methanesulfonate salt |
| | MTBE | |
| | ACN | No precipitation |
| | Heptane | |
| | 2-MeTHF | |
| | FA | |

Example 35: Polymorph screening study on methanesulfonate salt of compound of formula I (slow volatilization method)

A certain amount of crystalline form A of a methanesulfonate salt of a compound of formula I was weighed and added to an 8 mL glass flask, and the following solvent was added to prepare a clear solution at a certain concentration. The flask placed in a fume hood at room temperature for diffusion. When precipitation occurred, the flask was taken out, and the resulting precipitate was subjected to vacuum drying for 20 h.

**Table 33. Polymorph screening results for the methanesulfonate salt of the compound of formula I**

| **Solvent** | Crystalline form results |
|---|---|
| DCM/MeOH=2-1 | Crystalline form B |
| MeOH | Crystalline form A |
| EtOH | Crystalline form B |
| EtOH/EA=2-1 | Crystalline form A |
| MeOH/FA=2-1 | Crystalline form B |

Example 36: Polymorph screening study on amorphous form of methanesulfonate salt of compound of formula I (suspension method)
40 mg of an amorphous form sample of a methanesulfonate salt was weighed and added to a 2 mL glass flask, and 200 µL of solvent was added. The mixture was stirred at 50 °C or room temperature for 6 days and quickly centrifuged (6000 rpm, 6 min) to remove a supernatant. The resulting precipitate was subjected to vacuum drying for 20 h.

**Table 34. Polymorph screening results for the amorphous form of the methanesulfonate salt of the compound of formula I**

| **No.** | **Solvent** | **Crystalline form results Room temperature** | **Crystalline form results 50°C** |
|---|---|---|---|
| - | Initial crystalline form | Amorphous form (possibly containing very small amount of crystalline form A of methanesulfonate salt) | |
| 1 | IPA | Mixture of crystalline form A with crystalline form A of methanesulfonate salt | |
| 2 | DCM | Crystalline form A of methanesulfonate salt | Crystalline form A of methanesulfonate salt |
| 3 | MIBK | Crystalline form A of methanesulfonate salt | Crystalline form A of methanesulfonate salt |
| 4 | 1,4-Dixoane | 1,4-dioxane solvate | |
| 5 | Toluene | Crystalline form A of methanesulfonate salt | Crystalline form A of methanesulfonate salt |
| 6 | MeOH/THF=1-1 | Methanol solvate of methanesulfonate salt | Methanol solvate of methanesulfonate salt |
| 7 | MeOH/EA=1-1 | Methanol solvate of methanesulfonate salt | Methanol solvate of methanesulfonate salt |
| 8 | MeOH/ACN=1-1 | Methanol solvate of methanesulfonate salt | Methanol solvate of methanesulfonate salt + crystalline form A of free base |
| 9 | Acetone/ACN = 1-1 | Crystalline form A of methanesulfonate salt | Crystalline form A of methanesulfonate salt |
| 10 | Acetone/EA = 1-1 | Crystalline form A of methanesulfonate salt | Crystalline form A of methanesulfonate salt |
| 11 | Acetone/THF = 1-1 | Crystalline form A of methanesulfonate salt | Crystalline form A of methanesulfonate salt |
| 12 | DCM/Hepatane= 1-1 | Crystalline form A of methanesulfonate salt | Crystalline form A of methanesulfonate salt |
| 13 | DCM/EA=1-1 | Crystalline form A of methanesulfonate salt | Crystalline form A of methanesulfonate salt |
| 14 | DCM/ACN=1-1 | Crystalline form A of methanesulfonate salt | Crystalline form A of methanesulfonate salt |
| 15 | DCM/THF=1-1 | Crystalline form A of methanesulfonate salt | Crystalline form A of methanesulfonate salt |

Example 37: Stability study on compound of formula 1, hydrochloride salt, methanesulfonate salt, and sulfate salt

An appropriate amount of corresponding compounds (a crystalline form A of a compound of formula I, a crystalline form B of a compound of formula I, a crystalline form A of a hydrochloride salt of a compound of formula I, a crystalline form A of a methanesulfonate salt of a compound of formula I, and a crystalline form A of a sulfate salt of a compound of formula I) were separately added to a 20 mL glass flask, and uniformly spread at the bottom of the flask. Then, the sample flasks were opened to air (sealed with aluminum foil punctured with small holes) and placed in constant temperature and humidity cabinets at high temperature (60 °C), at room temperature and high humidity (RT/92.5% RH), at RT/75% RH, and at 40 °C/75% RH for 30 days, respectively, and samples were taken at days 5, 10 and 30 for XRPD and HPLC detection.

XRPD results showed that the crystalline form A of the compound of formula I, the crystalline form B of the compound of formula I, the crystalline form A of the hydrochloride salt of the compound of formula I, the crystalline form A of the methanesulfonate salt of the compound of formula I, and the crystalline form A of the sulfate salt of the compound of formula I showed no change in the crystalline form after being stored for 30 days, thus having good physical stability.

HPLC results (Table 35, TRS for total impurity content) showed that the crystalline form A of the compound of formula I, the crystalline form B of the compound of formula I, and the crystalline form A of the hydrochloride salt of the compound of formula I all had good chemical stability.

**Table 35. Stability test results for content and related substances of the compound of formula I**

| Condition | Crystalline form A | Crystalline form B | Crystalline form A of hydrochloride salt |
|---|---|---|---|
| | TRS(%) | TRS(%) | TRS(%) |
| Initial | 0.21 | 0.89 | 0.31 |
| 60 °C-5d | 0.20 | 0.52 | 0.28 |
| 60 °C-10d | 0.25 | 0.53 | 0.34 |
| 60 °C-30d | 0.16 | 0.62 | 0.27 |
| RT/90RH%-5d | 0.24 | 0.55 | 0.28 |
| RT/90RH%-10d | 0.25 | 0.56 | 0.26 |
| RT/90RH%-30d | 0.17 | 0.58 | 0.27 |
| 40 °C/75RH%-5d | 0.16 | 0.54 | 0.30 |
| 40 °C/75RH%-10d | 0.16 | 0.46 | 0.28 |
| 40 °C/75RH%-30d | 0.16 | 0.56 | 0.28 |
| RT/75%RH 7d | 0.16 | / | 0.26 |
| RT/75%RH 10d | 0.16 | / | 0.26 |
| RT/75%RH 30d | 0.29 | / | 0.37 |

**Table 36. Summary of crystalline forms of the compound of formula I**

| Crystalline forms of the compound of formula I | Evaluation results |
|---|---|
| Crystalline form A | High stability, high crystallinity, and slight hygroscopicity |
| Crystalline form B | Good stability, high crystallinity, slight hygroscopicity, and easy formation of solvate |
| Crystalline form C | - |
| Toluene solvate | Crystalline form of toluene solvate |
| Crystalline form E | - |
| Crystalline form F | - |
| Crystalline form G | High crystallinity, slight hygroscopicity, and difficulty in preparation |
| *N*-methyl-2-pyrrolidone solvate | Crystalline form of NMP solvate |
| DMF solvate | Crystalline form of DMF solvate |
| Crystalline form K | - |

**Table 37. Summary of salt forms of compound of formula I**

| Salt form | Crystalline form | Evaluation results |
|---|---|---|
| hydrochloride | Crystalline form A of hydrochloride salt | DSC showing multiple endothermic peaks, with slight hygroscopicity |
| | Ethanol solvate of hydrochloride salt | Solvate |
| | Isopropanol solvate of hydrochloride salt | Solvate |
| Sulfate | Crystalline form A | Slight hygroscopicity |
| | Crystalline form B | TGA showing significant weight loss |
| | Crystalline form C | - |
| | Toluene solvate | Solvate |
| Methanesulfonate salt | Crystalline form A | High crystallinity and slight hygroscopicity |
| | Amorphous form | - |
| | Methanol solvate | Solvate |
| | 1,4-dioxane solvate | Solvate |
| *p*-toluenesulfonate | Crystalline form A | High crystallinity, DSC showing multiple endothermic peaks |
| Benzenesulfonate | Crystalline form A | - |

Although specific embodiments of the present invention have been described above, it will be understood by those skilled in the art that these embodiments are merely illustrative and that many changes or modifications can be made to these embodiments without departing from the principle and spirit of the present invention. Therefore, the protection scope of the present invention is defined by the appended claims.

## Claims

1. A crystalline form A of a compound of formula I, wherein the crystalline form A of the compound of formula I has an X-ray powder diffraction pattern having at least three, at least four, at least five, at least six, or at least seven characteristic peaks at the following 2Θ angles: 5.181° ± 0.2°, 6.254° ± 0.2°, 8.708° ± 0.2°, 11.496° ± 0.2°, 11.743° ± 0.2°, 16.538° ± 0.2°, and 20.361° ± 0.2°;

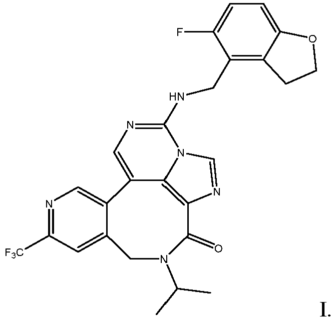

2. The crystalline form A of the compound of formula I according to claim 1, wherein the crystalline form A of the compound of formula I has an X-ray powder diffraction pattern having characteristic peaks at the following 2Θ angles: 5.181° ± 0.2°, 16.538° ± 0.2°, and 20.361° ± 0.2°; or
the crystalline form A of the compound of formula I has an X-ray powder diffraction pattern having characteristic peaks at the following 2Θ angles: 5.181° ± 0.2°, 11.743° ± 0.2°, 16.538° ± 0.2°, and 20.361° ± 0.2°; or
the crystalline form A of the compound of formula I has an X-ray powder diffraction pattern having characteristic peaks at the following 2Θ angles: 5.181° ± 0.2°, 6.254° ± 0.2°, 11.743° ± 0.2°, 16.538° ± 0.2°, and 20.361° ± 0.2°; or
the crystalline form A of the compound of formula I has an X-ray powder diffraction pattern having characteristic peaks at the following 2Θ angles: 5.181° ± 0.2°, 6.254° ± 0.2°, 11.496° ± 0.2°, 11.743° ± 0.2°, 16.538° ± 0.2°, and 20.361° ± 0.2°; or
the crystalline form A of the compound of formula I has an X-ray powder diffraction pattern having characteristic peaks at the following 2Θ angles: 5.181° ± 0.2°, 6.254° ± 0.2°, 8.708° ± 0.2°, 11.496° ± 0.2°, 11.743° ± 0.2°, 16.538° ± 0.2°, and 20.361° ± 0.2°; or
the crystalline form A of the compound of formula I has an X-ray powder diffraction pattern having characteristic peaks at the following 2Θ angles: 5.181° ± 0.2°, 6.254° ± 0.2°, 8.708° ± 0.2°, 10.304° ± 0.2°, 11.496° ± 0.2°, 11.743° ± 0.2°, 16.538° ± 0.2°, 18.275° ± 0.2°, 18.58° ± 0.2°, 20.361° ± 0.2°, 21.113° ± 0.2°, 23.495° ± 0.2°, 24.232° ± 02°, 26.337° ± 02°, and 26.767° ± 0.2°; or
the crystalline form A of the compound of formula I has an X-ray powder diffraction pattern having characteristic peaks at the following 2Θ angles: 5.181° ± 0.2°, 6.254° ± 0.2°, 7.052° ± 0.2°, 8.708° ± 0.2°, 10.304° ± 0.2°, 10.633° ± 0.2°, 11.496° ± 0.2°, 11.743° ± 0.2°, 12.466° ± 0.2°, 12.849° ± 0.2°, 13.224° ± 0.2°, 14.047° ± 0.2°, 14.784° ± 0.2°, 15.004° ± 0.2°, 15.917° ± 0.2°, 16.538° ± 0.2°, 17.529° ± 0.2°, 17.726° ± 0.2°, 18.275° ± 0.2°, 18.58° ± 0.2°, 19.083° ± 0.2°, 19.291° ± 0.2°, 19.848° ± 0.2°, 20.361° ± 0.2°, 21.113° ± 0.2°, 22.221° ± 0.2°, 22.458° ± 0.2°, 23.066° ± 0.2°, 23.495° ± 0.2°, 23.743° ± 0.2°, 24.232° ± 0.2°, 25.19° ± 0.2°, 25.885° ± 0.2°, 26.337° ± 0.2°, 26.767° ± 0.2°, 27.119° ± 0.2°, 27.832° ± 0.2°, 28.188° ± 0.2°, 29.263° ± 0.2°, and 30.363° ± 0.2°; or
the crystalline form A of the compound of formula I has X-ray powder diffraction pattern analysis data as shown in the following table:
| Position [°2θ] ± 0.2° | d-spacing [Å] | Relative intensity [%] |
|---|---|---|
| 5.181 | 17.042 | 100 |
| 6.254 | 14.1202 | 25.2 |
| 7.052 | 12.5243 | 6 |
| 8.708 | 10.1458 | 22.9 |
| 10.304 | 8.5777 | 18.8 |
| 10.633 | 8.3134 | 7 |
| 11.496 | 7.691 | 23.7 |
| 11.743 | 7.5296 | 29.6 |
| 12.466 | 7.0947 | 9.4 |
| 12.849 | 6.8842 | 4.3 |
| 13.224 | 6.6896 | 6.2 |
| 14.047 | 6.2996 | 6.5 |
| 14.784 | 5.9869 | 3.8 |
| 15.004 | 5.9 | 7.2 |
| 15.917 | 5.5632 | 8.8 |
| 16.538 | 5.3557 | 43 |
| 17.529 | 5.0553 | 9.2 |
| 17.726 | 4.9996 | 5.4 |
| 18.275 | 4.8505 | 13.6 |
| 18.58 | 4.7716 | 14.4 |
| 19.083 | 4.647 | 7.2 |
| 19.291 | 4.5973 | 4.6 |
| 19.848 | 4.4695 | 6.5 |
| 20.361 | 4.358 | 32.2 |
| 21.113 | 4.2044 | 13.9 |
| 22.221 | 3.9973 | 4.8 |
| 22.458 | 3.9556 | 5.4 |
| 23.066 | 3.8527 | 8.8 |
| 23.495 | 3.7834 | 17.4 |
| 23.743 | 3.7443 | 7.7 |
| 24.232 | 3.6699 | 13.8 |
| 25.19 | 3.5325 | 7.2 |
| 25.885 | 3.4392 | 8.3 |
| 26.337 | 3.3812 | 10.6 |
| 26.767 | 3.3278 | 12.4 |
| 27.119 | 3.2855 | 6.4 |
| 27.832 | 3.2028 | 3.7 |
| 28.188 | 3.1631 | 3.7 |
| 29.263 | 3.0494 | 4.3 |
| 30.363 | 2.9414 | 5.6 |
; or the crystalline form A of the compound of formula I has an X-ray powder diffraction pattern substantially as shown in FIG. 4.

3. The crystalline form A of the compound of formula I according to claim 1, wherein the crystalline form A of the compound of formula I has a thermogravimetric analysis curve showing a weight loss of 0.1264% ± 0.05% during heating from 29.6 °C ± 3 °C to 150.14 °C ± 3 °C; for example, the crystalline form A of the compound of formula I has a thermogravimetric analysis curve showing a weight loss of 0.1264% during heating from 29.6 °C to 150.14 °C; for another example, the crystalline form A of the compound of formula I has a thermogravimetric analysis curve substantially as shown in FIG. 5; and/or
the crystalline form A of the compound of formula I has a differential scanning calorimetry curve having endothermic peaks with initial temperatures of 327.1 °C ± 3 °C and 334.5 °C ± 3 °C, respectively; for example, the crystalline form A of the compound of formula I has a differential scanning calorimetry curve having endothermic peaks with initial temperatures of 327.1 °C and 334.5 °C, respectively; and/or the crystalline form A of the compound of formula I has a differential scanning calorimetry curve having endothermic peaks with peak temperatures of 330.45 °C ± 3 °C and 336.58 °C ± 3 °C, respectively; for example, the crystalline form A of the compound of formula I has a differential scanning calorimetry curve having endothermic peaks with peak temperatures of 330.45 °C and 336.58 °C, respectively; for another example, the crystalline form A of the compound of formula I has a differential scanning calorimetry curve substantially as shown in FIG. 6; and/or
the crystalline form A of the compound of formula I has a dynamic vapor sorption curve showing a hygroscopic weight gain of 0.44% ± 0.05% at 25 °C and 80% RH; for example, the crystalline form A of the compound of formula I has a dynamic vapor sorption curve showing an hygroscopic weight gain of 0.44% at 25 °C and 80% RH; for another example, the crystalline form A has a dynamic vapor sorption curve substantially as shown in FIG. 7.

4. A preparation method for the crystalline form A of the compound of formula I according to any one of claims 1-3, wherein the preparation method is selected from any one of the following methods:
method I comprising the following steps: stirring a crystalline form B of a compound of formula I in a solvent to precipitate a solid, and separating and drying the solid to obtain the crystalline form A of the compound of formula I, wherein the solvent is an organic solvent or a mixed solvent of an organic solvent and water; the organic solvent is selected from one or more of methanol, ethanol, dichloromethane, and acetonitrile; the stirring is preferably performed at a temperature of 20-60 °C; the stirring is preferably performed for a period of 1.5-2.5 days; the crystalline form B of the compound of formula I and the solvent are preferably in a mass-to-volume ratio of (250 mg-350 mg):1 mL; when the solvent is a mixed solvent of an organic solvent and
water, the organic solvent and the water are preferably in a volume ratio of (0.5-3.5):1; wherein when the organic solvent is ethanol, the stirring is performed at a temperature of 40-60 °C; when the solvent is a mixed solvent of methanol and water in a volume ratio of (2.5-3.5):1, the stirring is performed at a temperature of 20-30 °C; and when the solvent is a mixed solvent of methanol and water in a volume ratio of (0.5-1.5):1, the stirring is performed at a temperature of 40-60 °C;
method II comprising the following steps: stirring a crystalline form B of a compound of formula I in dichloromethane to obtain a mixed solution, adding an anti-solvent and stirring the solution to precipitate a solid, and separating and drying the solid to obtain the crystalline form A of the compound of formula I,
wherein the anti-solvent is selected from one or more of ethanol, tetrahydrofuran, and ethyl acetate; the stirring is preferably performed at a temperature of 20-60 °C; the stirring is preferably performed for a period of 1.5-2.5 days; the crystalline form B of the compound of formula I and the dichloromethane are preferably in a mass-to-volume ratio of (50 mg-200 mg):1 mL; and the dichloromethane and the anti-solvent are preferably in a volume ratio of (0.5-2.5):1;
method III comprising the following steps: stirring an amorphous form of a compound of formula I in a solvent to precipitate a solid, and separating and drying the solid to obtain the crystalline form A of the compound of formula I, wherein the solvent is an organic solvent or a mixed solvent of an organic solvent and water; the organic solvent is selected from one or more of methanol, ethanol, acetonitrile, isopropanol, and
dichloromethane; the stirring is preferably performed at a temperature of 20-60 °C; the stirring is preferably performed for a period of 1.5-2.5 days; the amorphous form of the compound of formula I and the solvent are preferably in a mass-to-volume ratio of (200 mg-350 mg):1 mL; when the solvent is a mixed solvent of an organic solvent and water, the organic solvent and the water are preferably in a volume ratio of (0.5-3.5):1;
wherein when the solvent is a mixed solvent of methanol and water, the methanol and the water are in a volume ratio of (2.5-3.5):1; and when the solvent is ethanol or a mixed solvent of ethanol and water in a volume ratio of 1:1, the stirring is performed at a temperature of 40-60 °C;
method IV comprising the following steps: stirring a crystalline form A of a hydrochloride salt of a compound of formula I in a mixed solvent of an organic solvent and water to precipitate a solid, and separating and drying the solid to obtain the crystalline form A of the compound of formula I, wherein the organic solvent is selected from one or two of methanol and ethanol; the stirring is preferably performed at a temperature of 35-45 °C;
the stirring is preferably performed for a period of 2.5-3.5 days; the hydrochloride salt of the compound of formula I and the mixed solvent are preferably in a mass-to-volume ratio of (100 mg-200 mg): 1 mL; and the organic solvent and the water are preferably in a volume ratio of (2.5-3.5): 1;
method V comprising the following steps: stirring a crystalline form A of a sulfate salt of a compound of formula I in a solvent to precipitate a solid, and separating and drying the solid to obtain the crystalline form A of the compound of formula I, wherein the solvent is a mixed solvent of methanol, an organic solvent and
water or a mixed solvent of methanol and dichloromethane; the organic solvent is selected from one or more of methanol, ethanol, and acetonitrile; the stirring is preferably performed at a temperature of 35-45 °C; the stirring is preferably performed for a period of 2.5-3.5 days; the crystalline form A of the sulfate salt of the compound of formula I and the solvent are preferably in a mass-to-volume ratio of (100 mg-200 mg): 1 mL; and the organic solvent and the water are preferably in a volume ratio of (0.5-3.5): 1;
method VI comprising the following steps: mixing a crystalline form A of a sulfate salt of a compound of formula I with methanol, adding heptane to precipitate a solid and stirring, and separating and drying the solid to obtain the crystalline form A of the compound of formula I, wherein the stirring is preferably performed at a temperature of 20-30 °C; the stirring is preferably performed for a period of 0.5-1.5 days; and the crystalline form A of the sulfate salt of the compound of formula I and the methanol are preferably in a mass-to-volume ratio of (30 mg-40 mg):1 mL;
method VII comprising the following steps: stirring a crystalline form A of a methanesulfonate salt of a compound of formula I in a solvent to precipitate a solid, and separating and drying the solid to obtain the crystalline form A of the compound of formula I, wherein the solvent is water or a mixed solvent of an organic solvent and water; the organic solvent is selected from one or two of methanol and acetonitrile; the stirring is preferably performed at a temperature of 35-45 °C; the stirring is preferably performed for a period of 2.5-3.5 days; and the crystalline form A of the methanesulfonate salt of the compound of formula I and the solvent are preferably in a mass-to-volume ratio of (100 mg-200 mg):1 mL;
method VIII comprising the following steps: mixing a crystalline form A of a methanesulfonate salt of a compound of formula I with DMF, adding acetonitrile to precipitate a solid and stirring, and separating and
drying the solid to obtain the crystalline form A of the compound of formula I, wherein the stirring is preferably performed at a temperature of 20-30 °C; the stirring is preferably performed for a period of 12-16 h; and the methanesulfonate salt of the compound of formula I and DMF are preferably in a mass-to-volume ratio of (30 mg-70 mg): 1 mL; and
method IX comprising the following steps: stirring a crystalline form A of a methanesulfonate salt of a compound of formula I in a solvent until a clear solution is obtained, and volatilizing and drying the solution to obtain the crystalline form A of the compound of formula I, wherein the solvent is methanol or a mixed solvent of ethanol and methyl acetate; the organic solvent is selected from one or two of methanol and acetonitrile; the volatilizing and drying are preferably performed at a temperature of 20-30 °C; and the ethanol and the methyl acetate are preferably in a volume ratio of (1.5-2.5): 1.

5. A crystalline form B of a compound of formula I, wherein the crystalline form B of the compound of formula I has an X-ray powder diffraction pattern having at least three, at least four, at least five, at least six, or at least seven characteristic peaks at the following 2Θ angles: 5.884° ± 0.2°, 14.747° ± 0.2°, 16.575° ± 0.2°, 18.116° ± 0.2°, 19.987° ± 0.2°, 22.225° ± 0.2°, and 26.884° ± 0.2°;

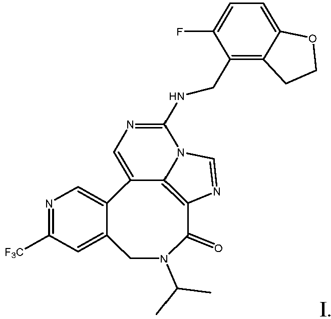

6. The crystalline form B of the compound of formula I according to claim 5, wherein the crystalline form B of the compound of formula I has an X-ray powder diffraction pattern having characteristic peaks at the following 2Θ angles: 5.884° ± 0.2°, 14.747° ± 0.2°, and 16.575° ± 0.2°; or
the crystalline form B of the compound of formula I has an X-ray powder diffraction pattern having characteristic peaks at the following 2Θ angles: 5.884° ± 0.2°, 14.747° ± 0.2°, 16.575° ± 0.2°, and 18.116° ± 0.2°; or
the crystalline form B of the compound of formula I has an X-ray powder diffraction pattern having characteristic peaks at the following 2Θ angles: 5.884° ± 0.2°, 14.747° ± 0.2°, 16.575° ± 0.2°, 18.116° ± 0.2°, and 26.884° ± 0.2°; or
the crystalline form B of the compound of formula I has an X-ray powder diffraction pattern having characteristic peaks at the following 2Θ angles: 5.884° ± 0.2°, 14.747° ± 0.2°, 16.575° ± 0.2°, 18.116° ± 0.2°, 19.987° ± 0.2°, and 26.884° ± 0.2°; or
the crystalline form B of the compound of formula I has an X-ray powder diffraction pattern having characteristic peaks at the following 2Θ angles: 5.884° ± 0.2°, 14.747° ± 0.2°, 16.575° ± 0.2°, 18.116° ± 0.2°, 19.987° ± 0.2°, 22.225° ± 02°, and 26.884° ± 0.2°; or
the crystalline form B of the compound of formula I has an X-ray powder diffraction pattern having characteristic peaks at the following 2Θ angles: 5.884° ± 0.2°, 13.263° ± 0.2°, 14.747° ± 0.2°, 16.575° ± 0.2°, 17.259° ± 0.2°, 18.116° ± 0.2°, 19.558° ± 0.2°, 19.987° ± 0.2°, 21.566° ± 0.2°, 22.225° ± 0.2°, 23.456° ± 0.2°, 23.999° ± 0.2°, and 26.884° ± 0.2°; or
the crystalline form B of the compound of formula I has an X-ray powder diffraction pattern having characteristic peaks at the following 2Θ angles: 5.884° ± 0.2°, 13.263° ± 0.2°, 13.868° ± 0.2°, 14.299° ± 0.2°, 14.747° ± 0.2°, 15.622° ± 0.2°, 16.575° ± 0.2°, 17.259° ± 0.2°, 18.116° ± 0.2°, 19.558° ± 0.2°, 19.987° ± 0.2°, 21.566° ± 0.2°, 22.225° ± 0.2°, 23.258° ± 0.2°, 23.456° ± 0.2°, 23.999° ± 0.2°, 24.894° ± 0.2°, 26.884° ± 0.2°, 29.551° ± 0.2°, 31.442° ± 02°, and 31.695° ± 0.2°; or
the crystalline form B of the compound of formula I has an X-ray powder diffraction pattern having characteristic peaks at the following 2Θ angles: 5.884° ± 0.2°, 10.54° ± 0.2°, 11.629° ± 0.2°, 12.427° ± 0.2°, 13.263° ± 0.2°, 13.868° ± 0.2°, 14.299° ± 0.2°, 14.747° ± 0.2°, 15.622° ± 0.2°, 16.575° ± 0.2°, 17.259° ± 0.2°, 18.116° ± 0.2°, 19.558° ± 0.2°, 19.987° ± 0.2°, 21.566° ± 0.2°, 22.225° ± 0.2°, 23.258° ± 0.2°, 23.456° ± 0.2°, 23.999° ± 0.2°, 24.894° ± 0.2°, 25.501° ± 0.2°, 26.884° ± 0.2°, 27.328° ± 0.2°, 27.7° ± 0.2°, 28.048° ± 0.2°, 28.557° ± 0.2°, 29.551° ± 0.2°, 30.497° ± 0.2°, 30.859° ± 0.2°, 31.442° ± 0.2°, 31.695° ± 0.2°, 32.436° ± 0.2°, 33.328° ± 0.2°, 34.071° ± 02°, 34.715° ± 02°, 35.553° ± 0.2°, 35.842° ± 0.2°, and 36.347° ± 0.2°; or
the crystalline form B of the compound of formula I has X-ray powder diffraction pattern analysis data as shown in the following table:
| Position [°2θ] ± 0.2° | d-spacing [Å] | Relative intensity [%] |
|---|---|---|
| 5.884 | 15.0089 | 83.1 |
| 10.54 | 8.3861 | 3.9 |
| 11.629 | 7.6036 | 5.5 |
| 12.427 | 7.1168 | 5.5 |
| 13.263 | 6.67 | 22.3 |
| 13.868 | 6.3803 | 11.6 |
| 14.299 | 6.1889 | 13.8 |
| 14.747 | 6.0021 | 100 |
| 15.622 | 5.6676 | 15.3 |
| 16.575 | 5.3439 | 56.6 |
| 17.259 | 5.1336 | 26.1 |
| 18.116 | 4.8927 | 50.7 |
| 19.558 | 4.5351 | 24 |
| 19.987 | 4.4388 | 45.8 |
| 21.566 | 4.1172 | 27 |
| 22.225 | 3.9966 | 30.9 |
| 23.258 | 3.8213 | 15.7 |
| 23.456 | 3.7896 | 26.4 |
| 23.999 | 3.7051 | 23.3 |
| 24.894 | 3.5738 | 14 |
| 25.501 | 3.4901 | 8.8 |
| 26.884 | 3.3136 | 47.9 |
| 27.328 | 3.2607 | 8.4 |
| 27.7 | 3.2178 | 6.8 |
| 28.048 | 3.1787 | 6.9 |
| 28.557 | 3.1231 | 7.9 |
| 29.551 | 3.0203 | 11.2 |
| 30.497 | 2.9288 | 6 |
| 30.859 | 2.8952 | 6.3 |
| 31.442 | 2.8428 | 14 |
| 31.695 | 2.8207 | 15.4 |
| 32.436 | 2.758 | 9.8 |
| 33.328 | 2.6861 | 8.2 |
| 34.071 | 2.6293 | 7.3 |
| 34.715 | 2.5819 | 8.1 |
| 35.553 | 2.523 | 7.5 |
| 35.842 | 2.5033 | 7.2 |
| 36.347 | 2.4697 | 7 |
; or the crystalline form B of the compound of formula I has an X-ray powder diffraction pattern substantially as shown in FIG. 8.

7. The crystalline form B of the compound of formula I according to claim 5, wherein the crystalline form B of the compound of formula I has a thermogravimetric analysis curve showing a weight loss of 0.1817% ± 0.05% during heating from 28.73 °C ± 3 °C to 149.63 °C ± 3 °C; for example, the crystalline form B of the compound of formula I has a thermogravimetric analysis curve showing a weight loss of 0.1817% during heating from 28.73 °C to 149.63 °C; for another example, the crystalline form B of the compound of formula I has a thermogravimetric analysis curve substantially as shown in FIG. 9; and/or the crystalline form B of the compound of formula I has a differential scanning calorimetry curve having endothermic peaks with initial temperatures of 249.1 °C ± 3 °C and 336.8 °C ± 3 °C, respectively; for example, the crystalline form B of the compound of formula I has a differential scanning calorimetry curve having endothermic peaks with initial temperatures of 249.1 °C and 336.8 °C, respectively; and/or the crystalline form B of the compound of formula I has a differential scanning calorimetry curve having endothermic peaks with peak temperatures of 252.54 °C ± 3 °C and 337.59 °C ± 3 °C, respectively; for example, the crystalline form B of the compound of formula I has a differential scanning calorimetry curve having endothermic peaks with peak temperatures of 252.54 °C and 337.59 °C, respectively; and/or the crystalline form B of the compound of formula I has a differential scanning calorimetry curve having an exothermic peak with a peak temperature of 255.28 °C ± 3 °C; for example, the crystalline form B of the compound of formula I has a differential scanning calorimetry curve having an exothermic peak with a peak temperature of 255.28 °C; and/or
the crystalline form B of the compound of formula I has a differential scanning calorimetry curve substantially as shown in FIG. 10; and/or
the crystalline form B of the compound of formula I has a dynamic vapor sorption curve showing a hygroscopic weight gain of 0.84% ± 0.05% at 25 °C and 80% RH; for example, the crystalline form B of the compound of formula I has a dynamic vapor sorption curve showing an hygroscopic weight gain of 0.84% at 25 °C and 80% RH; for another example, the crystalline form B of the compound of formula I has a dynamic vapor sorption curve as shown in FIG. 11.

8. A preparation method for the crystalline form B according to any one of claims 5-7, wherein the preparation method is selected from any one of the following methods:
method I comprising the following steps: stirring a crystalline form A of a compound of formula I in an organic solvent to precipitate a solid, and separating and drying the solid to obtain the crystalline form B of the compound of formula I, wherein the organic solvent is selected from one or more of ethyl acetate, acetone, 88% acetone, and tetrahydrofuran; the stirring is preferably performed at 20-60 °C; the stirring is preferably performed for a period of 1.5-2.5 days; the crystalline form A of the compound of formula I and the organic solvent are preferably in a mass-to-volume ratio of (80 mg-250 mg):1 mL; wherein when the organic solvent is tetrahydrofuran, the stirring is performed at a temperature of 35-45 °C;
method II comprising the following steps: stirring an amorphous form of a compound of formula I in a solvent to precipitate a solid, and separating and drying the solid to obtain the crystalline form B of the compound of formula I, wherein the solvent is an organic solvent or a mixed solvent of acetone and water in a volume ratio of (0.25-0.75):1; the organic solvent is selected from one or more of ethanol, ethyl acetate, acetone, methyl isobutyl ketone, and methyl *tert*-butyl ether; the stirring is preferably performed at a temperature of 20-60 °C; the stirring is preferably performed for a period of 1.5-2.5 days; the amorphous form of the compound of formula I and the solvent are preferably in a mass-to-volume ratio of (250 mg-350 mg):1 mL; wherein when the organic solvent is ethyl acetate or methyl *tert*-butyl ether, the stirring is performed at a temperature of 40-60 °C; and when the organic solvent is ethanol or methyl isobutyl ketone, the stirring is performed at a temperature of 20-30 °C;
method III comprising the following steps: stirring a crystalline form A of a hydrochloride salt of a compound of formula I or a crystalline form A of a sulfate salt of a compound of formula I in 88% acetone to precipitate a solid, and separating and drying the solid to obtain the crystalline form B of the compound of formula I, wherein the stirring is preferably performed at a temperature of 35-45 °C; the stirring is preferably performed for a period of 2.5-3.5 days; the crystalline form A of the hydrochloride salt of the compound of formula I or the crystalline form A of the sulfate salt of the compound of formula I and the 88% acetone are preferably in a mass-to-volume ratio of (100 mg-200 mg):1 mL;
method IV comprising the following steps: mixing a crystalline form A of a sulfate salt of a compound of formula I with methanol, adding acetone to precipitate a solid and stirring, and separating and drying the solid to obtain the crystalline form B of the compound of formula I, wherein the stirring is preferably performed at a temperature of 20-30 °C; the stirring is preferably performed for a period of 0.5-1.5 days; and the crystalline form A of the sulfate salt of the compound of formula I and the methanol are preferably in a mass-to-volume ratio of (30 mg-40 mg):1 mL; and
method V comprising the following steps: stirring a crystalline form A of a methanesulfonate salt of a compound of formula I in a solvent until a clear solution is obtained, and volatilizing and drying the solution to obtain the crystalline form B of the compound of formula I, wherein the solvent is ethanol or a mixed solvent of methanol and an organic solvent; the organic solvent is selected from one or two of methyl acetate and dichloromethane; the volatilizing and drying are preferably performed at a temperature of 20-30 °C; and the ethanol and the methyl acetate are preferably in a volume ratio of (1.5-2.5):1.

9. A crystalline form G of a compound of formula I, wherein the crystalline form G of the compound of formula I has an X-ray powder diffraction pattern having at least three, at least four, at least five, at least six, at least seven, or at least eight characteristic peaks at the following 2Θ angles: 5.279° ± 0.2°, 10.926° ± 0.2°, 11.843° ± 0.2°, 16.187° ± 0.2°, 16.831° ± 0.2°, 20.453° ± 0.2°, 23.55° ± 0.2°, and 26.825° ± 0.2°;

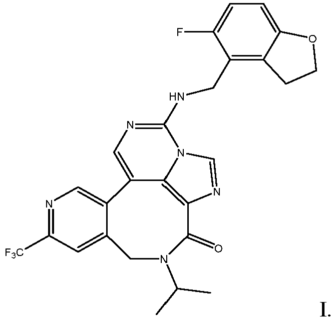

10. The crystalline form G of the compound of formula I according to claim 9, wherein the crystalline form G of the compound of formula I has an X-ray powder diffraction pattern having characteristic peaks at the following 2Θ angles: 5.279° ± 0.2°, 20.453° ± 0.2°, and 23.55° ± 02°; or
the crystalline form G of the compound of formula I has an X-ray powder diffraction pattern having characteristic peaks at the following 2Θ angles: 5.279° ± 0.2°, 11.843° ± 0.2°, 20.453° ± 0.2°, and 23.55° ± 0.2°; or
the crystalline form G of the compound of formula I has an X-ray powder diffraction pattern having characteristic peaks at the following 2Θ angles: 5.279° ± 0.2°, 11.843° ± 0.2°, 16.831° ± 0.2°, 20.453° ± 0.2°, and 23.55° ± 0.2°; or
the crystalline form G of the compound of formula I has an X-ray powder diffraction pattern having characteristic peaks at the following 2Θ angles: 5.279° ± 0.2°, 11.843° ± 0.2°, 16.187° ± 0.2°, 16.831° ± 0.2°, 20.453° ± 0.2°, and 23.55° ± 0.2°; or
the crystalline form G of the compound of formula I has an X-ray powder diffraction pattern having characteristic peaks at the following 2Θ angles: 5.279° ± 0.2°, 10.926° ± 0.2°, 11.843° ± 0.2°, 16.187° ± 0.2°, 16.831° ± 0.2°, 20.453° ± 0.2°, and 23.55° ± 0.2°; or
the crystalline form G of the compound of formula I has an X-ray powder diffraction pattern having characteristic peaks at the following 2Θ angles: 5.279° ± 0.2°, 10.926° ± 0.2°, 11.843° ± 0.2°, 16.187° ± 0.2°, 16.831° ± 0.2°, 20.453° ± 02°, 23.55° ± 0.2°, and 26.825° ± 0.2°; or
the crystalline form G of the compound of formula I has an X-ray powder diffraction pattern having characteristic peaks at the following 2Θ angles: 5.279° ± 0.2°, 6.388° ± 0.2°, 9.02° ± 0.2°, 10.926° ± 0.2°, 11.843° ± 0.2°, 12.68° ± 0.2°, 14.96° ± 0.2°, 15.758° ± 0.2°, 16.187° ± 0.2°, 16.831° ± 0.2°, 17.98° ± 0.2°, 18.352° ± 0.2°, 20.453° ± 0.2°, 20.881° ± 0.2°, 21.194° ± 0.2°, 22.732° ± 0.2°, 23.55° ± 0.2°, 25.208° ± 0.2°, 25.949° ± 0.2°, and 26.825° ± 0.2°; or
the crystalline form G of the compound of formula I has an X-ray powder diffraction pattern having characteristic peaks at the following 2Θ angles: 5.279° ± 0.2°, 6.388° ± 0.2°, 9.02° ± 0.2°, 10.926° ± 0.2°, 11.843° ± 0.2°, 12.68° ± 0.2°, 13.44° ± 0.2°, 14.162° ± 0.2°, 14.96° ± 0.2°, 15.758° ± 0.2°, 16.187° ± 0.2°, 16.831° ± 0.2°, 17.98° ± 0.2°, 18.352° ± 0.2°, 19.423° ± 0.2°, 20.453° ± 0.2°, 20.881° ± 0.2°, 21.194° ± 0.2°, 21.755° ± 0.2°, 22.732° ± 0.2°, 23.55° ± 0.2°, 24.217° ± 0.2°, 24.772° ± 0.2°, 25.208° ± 0.2°, 25.949° ± 0.2°, 26.825° ± 0.2°, 28.694° ± 0.2°, 29.143° ± 0.2°, 30.154° ± 0.2°, 30.774° ± 0.2°, 31.537° ± 0.2°, 32.318° ± 0.2°, and 33.834° ± 0.2°; or
the crystalline form G of the compound of formula I has an X-ray powder diffraction pattern having characteristic peaks at the following 2Θ angles: 5.279° ± 0.2°, 6.388° ± 0.2°, 7.147° ± 0.2°, 9.02° ± 0.2°, 10.926° ± 0.2°, 11.843° ± 0.2°, 12.68° ± 0.2°, 13.44° ± 0.2°, 14.162° ± 0.2°, 14.96° ± 0.2°, 15.758° ± 0.2°, 16.187° ± 0.2°, 16.831° ± 0.2°, 17.98° ± 0.2°, 18.352° ± 0.2°, 19.423° ± 0.2°, 20.453° ± 0.2°, 20.881° ± 0.2°, 21.194° ± 0.2°, 21.755° ± 0.2°, 22.732° ± 0.2°, 23.55° ± 0.2°, 24.217° ± 0.2°, 24.772° ± 0.2°, 25.208° ± 0.2°, 25.949° ± 0.2°, 26.825° ± 0.2°, 27.64° ± 0.2°, 28.694° ± 0.2°, 29.143° ± 0.2°, 30.154° ± 0.2°, 30.774° ± 0.2°, 31.537° ± 0.2°, 32.318° ± 0.2°, 33.834° ± 0.2°, 35.49° ± 0.2°, 36.347° ± 0.2°, 37.793° ± 0.2°, and 38.493° ± 0.2°; or
the crystalline form G of the compound of formula I has X-ray powder diffraction pattern analysis data as shown in the following table:
| Position [°2θ] ± 0.2° | d-spacing [Å] | Relative intensity [%] |
|---|---|---|
| 5.279 | 16.725 | 100 |
| 6.388 | 13.8245 | 29.5 |
| 7.147 | 12.3582 | 7.1 |
| 9.02 | 9.796 | 23.3 |
| 10.926 | 8.0908 | 38.7 |
| 11.843 | 7.4665 | 56.2 |
| 12.68 | 6.9755 | 28.4 |
| 13.44 | 6.5825 | 15 |
| 14.162 | 6.2485 | 14.2 |
| 14.96 | 5.917 | 29.8 |
| 15.758 | 5.6193 | 31.4 |
| 16.187 | 5.4711 | 41.1 |
| 16.831 | 5.2633 | 46.7 |
| 17.98 | 4.9295 | 33.2 |
| 18.352 | 4.8304 | 22.2 |
| 19.423 | 4.5663 | 18 |
| 20.453 | 4.3386 | 60.2 |
| 20.881 | 4.2507 | 21 |
| 21.194 | 4.1887 | 34.3 |
| 21.755 | 4.0818 | 11.7 |
| 22.732 | 3.9085 | 22.3 |
| 23.55 | 3.7746 | 66.7 |
| 24.217 | 3.6722 | 19.7 |
| 24.772 | 3.5912 | 17.3 |
| 25.208 | 3.53 | 29.6 |
| 25.949 | 3.4309 | 35.8 |
| 26.825 | 3.3208 | 36.7 |
| 27.64 | 3.2246 | 8.8 |
| 28.694 | 3.1086 | 11.1 |
| 29.143 | 3.0617 | 16 |
| 30.154 | 2.9613 | 13.6 |
| 30.774 | 2.903 | 12.8 |
| 31.537 | 2.8345 | 13 |
| 32.318 | 2.7677 | 13.1 |
| 33.834 | 2.6471 | 13.1 |
| 35.49 | 2.5273 | 8.8 |
| 36.347 | 2.4697 | 10 |
| 37.793 | 2.3784 | 8.6 |
| 38.493 | 2.3368 | 8.6 |
; or the crystalline form G has an X-ray powder diffraction pattern substantially as shown in FIG. 23.

11. The crystalline form G of the compound of formula I according to claim 9, wherein the crystalline form G of the compound of formula I has a thermogravimetric analysis curve showing a weight loss of 0.1261% ± 0.05% during heating from 36.93 °C ± 3 °C to 150.57 °C ± 3 °C; for example, the crystalline form G of the compound of formula I has a thermogravimetric analysis curve showing a weight loss of 0.1261% during heating from 36.93 °C to 150.57 °C; for another example, the crystalline form G of the compound of formula I has a thermogravimetric analysis curve as shown in FIG. 24; and/or
the crystalline form G of the compound of formula I has a differential scanning calorimetry curve having an endothermic peak with an initial temperature of 334.21 °C ± 3 °C; for example, the crystalline form G of the compound of formula I has a differential scanning calorimetry curve having an endothermic peak with an initial temperature of 334.21 °C; for another example, the crystalline form G of the compound of formula I has a differential scanning calorimetry curve as shown in FIG. 25.

12. A preparation method for the crystalline form G of the compound of formula I according to any one of claims 9-11, wherein the preparation method is selected from any one of the following methods:
method I comprising the following steps: stirring a crystalline form A of a compound of formula I in toluene to precipitate a solid, and separating and drying the solid to obtain the crystalline form G of the compound of formula I, wherein the stirring is preferably performed at a temperature of 20-45 °C; the stirring is preferably performed for a period of 1.5-2.5 days; and the crystalline form A of the compound of formula I and the toluene are preferably in a mass-to-volume ratio of (100 mg-250 mg): 1 mL;
method II comprising the following steps: stirring a crystalline form B of a compound of formula I in methyl *tert*-butyl ether to precipitate a solid, and separating and drying the solid to obtain the crystalline form G of the compound of formula I, wherein the stirring is performed at a temperature of 40-60 °C; the stirring is preferably performed for a period of 1.5-2.5 days; the crystalline form B of the compound of formula I and the methyl *tert*-butyl ether are preferably in a mass-to-volume ratio of (250 mg-350 mg): and
method III comprising the following steps: stirring an amorphous form of a compound of formula I in a solvent to precipitate a solid, and separating and drying the solid to obtain the crystalline form G of the compound of formula I, wherein the solvent is water, heptane, toluene, or a mixed solvent of methanol and water in a volume ratio of (0.5-1.5):1; the stirring is preferably performed at a temperature of 20-60 °C; the stirring is preferably performed for a period of 1.5-2.5 days; the amorphous form of the compound of formula I and the solvent are preferably in a mass-to-volume ratio of (250 mg-350 mg):1 mL; wherein when the solvent is heptane or a mixed solvent, the stirring is performed at a temperature of 40-60 °C.

13. A crystalline form A of a hydrochloride salt of a compound of formula I, wherein the crystalline form A of the hydrochloride salt of the compound of formula I has an X-ray powder diffraction pattern having at least three, at least four, at least five, at least six, at least seven, or at least eight characteristic peaks at the following 2Θ angles: 5.527° ± 0.2°, 11.026° ± 0.2°, 11.805° ± 0.2°, 16.39° ± 0.2°, 17.572° ± 0.2°, 23.853° ± 0.2°, 24.398° ± 0.2°, and 27.683° ± 0.2°;

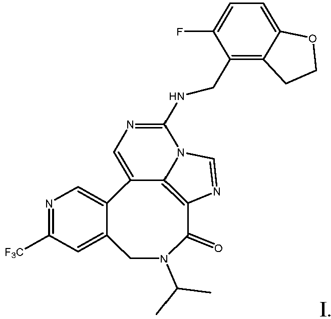

14. The crystalline form A of the hydrochloride salt of the compound of formula I according to claim 13, wherein the crystalline form A of the hydrochloride salt of the compound of formula I has an X-ray powder diffraction pattern having characteristic peaks at the following 2Θ angles: 5.527° ± 0.2°, 16.39° ± 0.2°, and 23.853° ± 0.2°; or
the crystalline form A of the hydrochloride salt of the compound of formula I has an X-ray powder diffraction pattern having characteristic peaks at the following 2Θ angles: 5.527° ± 0.2°, 16.39° ± 0.2°, 23.853° ± 0.2°, and 24.398° ± 0.2°; or
the crystalline form A of the hydrochloride salt of the compound of formula I has an X-ray powder diffraction pattern having characteristic peaks at the following 2Θ angles: 5.527° ± 0.2°, 16.39° ± 0.2°, 23.853° ± 0.2°, 24.398° ± 0.2°, and 27.683° ± 0.2°; or
the crystalline form A of the hydrochloride salt of the compound of formula I has an X-ray powder diffraction pattern having characteristic peaks at the following 2Θ angles: 5.527° ± 0.2°, 11.805° ± 0.2°, 16.39° ± 0.2°, 23.853° ± 0.2°, 24.398° ± 0.2°, and 27.683° ± 0.2°; or
the crystalline form A of the hydrochloride salt of the compound of formula I has an X-ray powder diffraction pattern having characteristic peaks at the following 2Θ angles: 5.527° ± 0.2°, 11.026° ± 0.2°, 11.805° ± 0.2°, 16.39° ± 0.2°, 23.853° ± 0.2°, 24.398° ± 0.2°, and 27.683° ± 0.2°; or
the crystalline form A of the hydrochloride salt of the compound of formula I has an X-ray powder diffraction pattern having characteristic peaks at the following 2Θ angles: 5.527° ± 0.2°, 11.026° ± 0.2°, 11.805° ± 0.2°, 16.39° ± 0.2°, 17.572° ± 0.2°, 23.853° ± 0.2°, 24.398° ± 0.2°, and 27.683° ± 0.2°; or
the crystalline form A of the hydrochloride salt of the compound of formula I has an X-ray powder diffraction pattern having characteristic peaks at the following 2Θ angles: 5.527° ± 0.2°, 11.026° ± 0.2°, 11.805° ± 0.2°, 12.655° ± 0.2°, 13.027° ± 0.2°, 16.39° ± 0.2°, 17.572° ± 0.2°, 20.57° ± 0.2°, 21.819° ± 0.2°, 22.358° ± 0.2°, 22.685° ± 0.2°, 23.853° ± 0.2°, 24.398° ± 0.2°, 27.037° ± 0.2°, 27.683° ± 0.2°, and 28.304° ± 0.2°; or
the crystalline form A of the hydrochloride salt of the compound of formula I has an X-ray powder diffraction pattern having characteristic peaks at the following 2Θ angles: 5.527° ± 0.2°, 5.911° ± 0.2°, 8.228° ± 0.2°, 11.026° ± 0.2°, 11.805° ± 0.2°, 12.655° ± 0.2°, 13.027° ± 0.2°, 16.39° ± 0.2°, 17.572° ± 0.2°, 18.876° ± 0.2°, 20.57° ± 0.2°, 21.819° ± 0.2°, 22.358° ± 0.2°, 22.685° ± 0.2°, 23.284° ± 0.2°, 23.853° ± 0.2°, 24.398° ± 0.2°, 27.037° ± 0.2°, 27.683° ± 0.2°, 28.304° ± 0.2°, 29.471° ± 0.2°, 30.051° ± 0.2°, 33.574° ± 0.2°, 36.726° ± 0.2°, and 38.297° ± 0.2°; or
the crystalline form A of the hydrochloride salt of the compound of formula I has X-ray powder diffraction pattern analysis data as shown in the following table:
| Position [°2θ] ± 0.2° | d-spacing [Å] | Relative intensity [%] |
|---|---|---|
| 5.527 | 15.976 | 91.7 |
| 5.911 | 14.9386 | 43.9 |
| 8.228 | 10.7364 | 39.5 |
| 11.026 | 8.0181 | 80.3 |
| 11.805 | 7.4902 | 80.7 |
| 12.655 | 6.9891 | 53.5 |
| 13.027 | 6.7903 | 52.2 |
| 16.39 | 5.4037 | 98.2 |
| 17.572 | 5.0428 | 77.2 |
| 18.876 | 4.6974 | 40.4 |
| 20.57 | 4.3142 | 64.5 |
| 21.819 | 4.07 | 52.6 |
| 22.358 | 3.9731 | 61.4 |
| 22.685 | 3.9165 | 51.8 |
| 23.284 | 3.8171 | 49.6 |
| 23.853 | 3.7273 | 100 |
| 24.398 | 3.6453 | 89.5 |
| 27.037 | 3.2952 | 50.4 |
| 27.683 | 3.2198 | 85.1 |
| 28.304 | 3.1505 | 71.1 |
| 29.471 | 3.0283 | 47.4 |
| 30.051 | 2.9712 | 43.9 |
| 33.574 | 2.667 | 33.8 |
| 36.726 | 2.445 | 29.4 |
| 38.297 | 2.3483 | 26.3 |
; or the crystalline form A of the hydrochloride salt of the compound of formula I has an X-ray powder diffraction pattern substantially as shown in FIG. 36.

15. The crystalline form A of the hydrochloride salt of the compound of formula I according to claim 13, wherein the crystalline form A of the hydrochloride salt of the compound of formula I has a thermogravimetric analysis curve showing a weight loss of 1.033% ± 0.05% during heating from 25.19 °C ± 3 °C to 108.66 °C ± 3 °C, and a weight loss of 6.683% ± 0.05% during heating from 108.66 °C ± 3 °C to 209.57 °C ± 3 °C; for example, the crystalline form A of the hydrochloride salt of the compound of formula I has a thermogravimetric analysis curve showing a weight loss of 1.033% during heating from 25.19 °C to 108.66 °C, and a weight loss of 6.683% during heating from 108.66 °C to 209.57 °C; for another example, the crystalline form A of the hydrochloride salt of the compound of formula I has a thermogravimetric analysis curve substantially as shown in FIG. 37; and/or
the crystalline form A of the hydrochloride salt of the compound of formula I has a differential scanning calorimetry curve having endothermic peaks with initial temperatures of 55.55 °C ± 3 °C and 190.92 °C ± 3 °C, respectively; for example, the crystalline form A of the hydrochloride salt of the compound of formula I has a differential scanning calorimetry curve having endothermic peaks with initial temperatures of 55.55 °C and 190.92 °C, respectively; and/or
the crystalline form A of the hydrochloride salt of the compound of formula I has a differential scanning calorimetry curve having endothermic peaks with peak temperatures of 97.51 °C ± 3 °C and 208.18 °C ± 3 °C, respectively; for example, the crystalline form A of the hydrochloride salt of the compound of formula I has a differential scanning calorimetry curve having endothermic peaks with peak temperatures of 97.51 °C and 208.18 °C, respectively; and/or
the crystalline form A of the hydrochloride salt of the compound of formula I has a differential scanning calorimetry curve substantially as shown in FIG. 38; and/or
the crystalline form A of the hydrochloride salt of the compound of formula I has a dynamic vapor sorption curve showing a hygroscopic weight gain of 0.31% ± 0.005% at 25 °C and 80% RH; for example, the crystalline form A of the hydrochloride salt has a dynamic vapor sorption curve showing a hygroscopic weight gain of 0.31% at 25 °C and 80% RH; for another example, the crystalline form A of the hydrochloride salt of the compound of formula I has a dynamic vapor sorption curve substantially as shown in FIG. 39.

16. A preparation method for the crystalline form A of the hydrochloride salt of the compound of formula I according to any one of claims 13-15, wherein the preparation method comprises the following steps: mixing and stirring a compound of formula I, hydrochloric acid, and tetrahydrofuran to precipitate a solid, and separating and drying the solid to obtain the crystalline form A of the hydrochloride salt of the compound of formula I, wherein the stirring is performed at a temperature of 20-45 °C; the stirring is preferably performed for a period of 0.2-0.8 h; the compound of formula I and the hydrochloric acid are preferably in a molar ratio of (1-1.5): 1; and the compound of formula I and the tetrahydrofuran are preferably in a mass-to-volume ratio of (20 mg-30 mg):1 mL.

17. A crystalline form A of a methanesulfonate salt of a compound of formula I, wherein the crystalline form A of the methanesulfonate salt of the compound of formula I has an X-ray powder diffraction pattern having at least three characteristic peaks at the following 2θ angles: 7.548° ± 0.2°, 15.087° ± 0.2°, and 15.554° ± 0.2°;

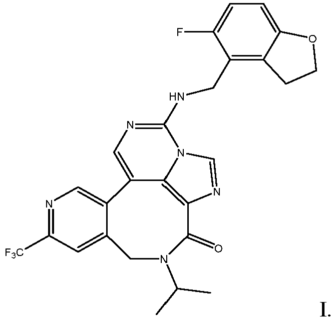

18. The crystalline form A of the methanesulfonate salt of the compound of formula I according to claim 17, wherein the crystalline form A of the methanesulfonate salt of the compound of formula I has an X-ray powder diffraction pattern having characteristic peaks at the following 2Θ angles: 7.548° ± 0.2°, 9.084° ± 0.2°, 10.268° ± 0.2°, 12.268° ± 0.2°, 13.505° ± 0.2°, 15.087° ± 0.2°, 15.554° ± 0.2°, 16.917° ± 0.2°, 18.994° ± 0.2°, 19.853° ± 0.2°, 20.515° ± 0.2°, 22.185° ± 0.2°, 22.785° ± 0.2°, 23.075° ± 0.2°, 24.146° ± 0.2°, 25.038° ± 0.2°, 26.533° ± 0.2°, 27.082° ± 0.2°, 28.572° ± 0.2°, 29.68° ± 0.2°, and 30.167° ± 0.2°; or
the crystalline form A of the methanesulfonate salt of the compound of formula I has X-ray powder diffraction pattern analysis data as shown in the following table:
| Position [°2θ] ± 0.2° | d-spacing [Å] | Relative intensity [%] |
|---|---|---|
| 7.548 | 11.7025 | 100 |
| 9.084 | 9.7266 | 7.3 |
| 10.268 | 8.6082 | 1.3 |
| 12.268 | 7.2085 | 1.4 |
| 13.505 | 6.5508 | 1.4 |
| 15.087 | 5.8677 | 19.7 |
| 15.554 | 5.6924 | 21 |
| 16.917 | 5.2366 | 2 |
| 18.994 | 4.6685 | 1.8 |
| 19.853 | 4.4684 | 1.9 |
| 20.515 | 4.3257 | 4.5 |
| 22.185 | 4.0037 | 5.6 |
| 22.785 | 3.8996 | 5.2 |
| 23.075 | 3.8511 | 4.4 |
| 24.146 | 3.6828 | 2.2 |
| 25.038 | 3.5535 | 3.5 |
| 26.533 | 3.3566 | 1.7 |
| 27.082 | 3.2898 | 2 |
| 28.572 | 3.1215 | 1.8 |
| 29.68 | 3.0075 | 2.5 |
| 30.167 | 2.96 | 3 |
; or the crystalline form A of the methanesulfonate salt of the compound of formula I has an X-ray powder diffraction pattern substantially as shown in FIG. 65.

19. The crystalline form A of the methanesulfonate salt of the compound of formula I according to claim 17, wherein the crystalline form A of the methanesulfonate salt of the compound of formula I has a thermogravimetric analysis curve showing a weight loss of 0.03112% ± 0.0005 % during heating from 30.96 °C ± 3 °C to 148.39 °C ± 3 °C; for example, the crystalline form A of the methanesulfonate salt of the compound of formula I has a thermogravimetric analysis curve showing a weight loss of 0.03112% during heating from 30.96 °C to 148.39 °C; for another example, the crystalline form A of the methanesulfonate salt of the compound of formula I has a thermogravimetric analysis curve as shown in FIG. 66; and/or the crystalline form A of the methanesulfonate salt of the compound of formula I has a differential scanning calorimetry curve having an endothermic peak with an initial temperature of 250.75 °C ± 3 °C; for example, the crystalline form A of the methanesulfonate salt of the compound of formula I has a differential scanning calorimetry curve having an endothermic peak with an initial temperature of 250.75 °C; and/or the crystalline form A of the methanesulfonate salt of the compound of formula I has a differential scanning calorimetry curve having an endothermic peak with a peak temperature of 262.7 °C ± 3 °C; for example, the crystalline form A of the methanesulfonate salt of the compound of formula I has a differential scanning calorimetry curve having an endothermic peak with a peak temperature of 262.7 °C; and/or the crystalline form A of the methanesulfonate salt of the compound of formula I has a differential scanning calorimetry curve as shown in FIG. 67; and/or
the crystalline form A of the methanesulfonate salt of the compound of formula I has a dynamic vapor sorption curve showing a hygroscopic weight gain of 0.84% ± 0.005% at 25 °C and 80% RH; for example, the crystalline form A of the methanesulfonate salt has a dynamic vapor sorption curve showing a hygroscopic weight gain of 0.84% at 25 °C and 80% RH; for another example, the crystalline form A of the methanesulfonate salt of the compound of formula I has a dynamic vapor sorption curve as shown in FIG. 68.

20. A preparation method for the crystalline form A of the methanesulfonate salt of the compound of formula I according to any one of claims 17-19, wherein the preparation method comprises the following steps: mixing and stirring a compound of formula I, tetrahydrofuran, and methanesulfonic acid, adding n-heptane to precipitate a solid, and separating and drying the solid to obtain the crystalline form A of the methanesulfonate salt of the compound of formula I, wherein the stirring is preferably performed at a temperature of 20-30 °C; the stirring is preferably performed for a period of 0.2-0.8 h; the compound of formula I and the methanesulfonic acid are preferably in a molar ratio of (1-1.5):1; the compound of formula I and the tetrahydrofuran are preferably in a mass-to-volume ratio of (40 mg-60 mg):1 mL; and the compound of formula I and the n-heptane are preferably in a mass-to-volume ratio of (70 mg-90 mg):1 mL.

21. A crystalline form A of a sulfate salt of a compound of formula I, wherein the crystalline form A of the sulfate salt of the compound of formula I has an X-ray powder diffraction pattern having at least three characteristic peaks at the following 2Θ angles: 7.645° ± 0.2°, 15.146° ± 0.2°, and 17.17° ± 0.2°;

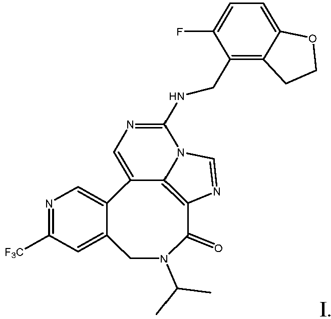

22. The crystalline form A of the sulfate salt of the compound of formula I according to claim 21, wherein the crystalline form A of the sulfate salt of the compound of formula I has an X-ray powder diffraction pattern having characteristic peaks at the following 2Θ angles: 5.468° ± 0.2°, 7.645° ± 0.2°, 15.146° ± 0.2°, 17.17° ± 0.2°, 19.25° ± 0.2°, 19.813° ± 0.2°, and 24.106° ± 0.2°; or
the crystalline form A of the sulfate salt of the compound of formula I has an X-ray powder diffraction pattern having characteristic peaks at the following 2Θ angles: 5.119° ± 0.2°, 5.468° ± 0.2°, 7.645° ± 0.2°, 8.424° ± 0.2°, 11.303° ± 0.2°, 15.146° ± 0.2°, 16.289° ± 0.2°, 16.701° ± 0.2°, 17.17° ± 0.2°, 18.176° ± 0.2°, 19.25° ± 0.2°, 19.813° ± 0.2°, 20.826° ± 0.2°, 21.138° ± 0.2°, 21.522° ± 0.2°, 22.476° ± 0.2°, 23.152° ± 0.2°, 24.106° ± 0.2°, 24.456° ± 0.2°, 25.351° ± 0.2°, 26.613° ± 0.2°, 27.41° ± 0.2°, 27.816° ± 0.2°, 28.324° ± 0.2°, and 29.084° ± 0.2°; or
the crystalline form A of the sulfate salt of the compound of formula I has X-ray powder diffraction pattern analysis data as shown in the following table:
| Position [°2θ] ± 0.2° | d-spacing [Å] | Relative intensity [%] |
|---|---|---|
| 5.119 | 17.2503 | 5.8 |
| 5.468 | 16.1496 | 11.7 |
| 7.645 | 11.5541 | 100 |
| 8.424 | 10.4877 | 9.2 |
| 11.303 | 7.8219 | 2.9 |
| 15.146 | 5.845 | 46.3 |
| 16.289 | 5.437 | 9.5 |
| 16.701 | 5.3039 | 7 |
| 17.17 | 5.1602 | 17.8 |
| 18.176 | 4.8767 | 3.9 |
| 19.25 | 4.6069 | 15.7 |
| 19.813 | 4.4774 | 10.4 |
| 20.826 | 4.2617 | 5.1 |
| 21.138 | 4.1995 | 4.7 |
| 21.522 | 4.1255 | 5.1 |
| 22.476 | 3.9525 | 8.4 |
| 23.152 | 3.8385 | 6.8 |
| 24.106 | 3.6888 | 10.8 |
| 24.456 | 3.6368 | 8.9 |
| 25.351 | 3.5104 | 9.3 |
| 26.613 | 3.3467 | 4 |
| 27.41 | 3.2512 | 7.4 |
| 27.816 | 3.2047 | 6.2 |
| 28.324 | 3.1484 | 4.7 |
| 29.084 | 3.0677 | 3.9 |
; or the crystalline form A of the sulfate salt of the compound of formula I has an X-ray powder diffraction pattern substantially as shown in FIG. 47.

23. The crystalline form A of the sulfate salt of the compound of formula I according to claim 21, wherein the crystalline form A of the sulfate salt of the compound of formula I has a thermogravimetric analysis curve showing a weight loss of 0.2336% ± 0.005% during heating from 32.19 ± 3 °C to 159.29 °C ± 3 °C, and a weight loss of 4.029% ± 0.005% during heating from 159.29 °C ± 3 °C to 252.51 °C ± 3 °C; for example, the crystalline form A of the sulfate salt of the compound of formula I has a thermogravimetric analysis curve showing a weight loss of 0.2336% during heating from 32.19 °C to 159.29 °C, and a weight loss of 4.029% during heating from 159.29 °C to 252.51 °C; for another example, the crystalline form A of the sulfate salt of the compound of formula I has a thermogravimetric analysis curve as shown in FIG. 48; and/or the crystalline form A of the sulfate salt of the compound of formula I has a differential scanning calorimetry curve having an endothermic peak with an initial temperature of 210.46 °C ± 3 °C; for example, the crystalline form A of the sulfate salt of the compound of formula I has a differential scanning calorimetry curve having an endothermic peak with an initial temperature of 210.46 °C; and/or
the crystalline form A of the sulfate salt of the compound of formula I has a differential scanning calorimetry curve having an endothermic peak with a peak temperature of 224.37 °C ± 3 °C; for example, the crystalline form A of the sulfate salt of the compound of formula I has a differential scanning calorimetry curve having an endothermic peak with a peak temperature of 224.37 °C; and/or
the crystalline form A of the sulfate salt of the compound of formula I has a differential scanning calorimetry curve as shown in FIG. 49; and/or
the crystalline form A of the sulfate salt of the compound of formula I has a dynamic vapor sorption curve showing a hygroscopic weight gain of 1.43% ± 0.05% at 25 °C and 80% RH; for example, the crystalline form A of the sulfate salt of the compound of formula I has a dynamic vapor sorption curve showing a hygroscopic weight gain of 1.43% at 25 °C and 80% RH; for example, the crystalline form A of the sulfate salt of the compound of formula I has a dynamic vapor sorption curve as shown in FIG. 50.

24. A preparation method for the crystalline form A of the sulfate salt of the compound of formula I according to any one of claims 22-23, wherein the preparation method comprises the following steps: mixing and stirring a compound of formula I, sulfuric acid, and tetrahydrofuran, adding n-heptane to precipitate a solid, and separating and drying the solid to obtain the crystalline form A of the sulfate salt of the compound of formula I, wherein the stirring is preferably performed at a temperature of 20-30 °C; the stirring is preferably performed for a period of 0.2-0.8 days; the compound of formula I and the sulfuric acid are preferably in a molar ratio of (1-1.5): 1; the compound of formula I and the tetrahydrofuran are preferably in a mass-to-volume ratio of (40 mg-50 mg): 1 mL; and the compound of formula I and the n-heptane are in a mass-to-volume ratio of (70 mg-90 mg):1 mL.

25. A pharmaceutical composition, wherein the pharmaceutical composition comprises a crystalline form A of a compound of formula I, a crystalline form B of a compound of formula I, a crystalline form C of a compound of formula I, a crystalline form of a toluene solvate of a compound of formula I, a crystalline form E of a compound of formula I, a crystalline form F of a compound of formula I, a crystalline form G of a compound of formula I, a crystalline form of an *N*-methyl-2-pyrrolidone solvate of a compound of formula I, a crystalline form of a DMF solvate of a compound of formula I, a crystalline form K of a compound of formula I, a crystalline form A of a hydrochloride salt of a compound of formula I, a crystalline form A of an ethanol solvate of a hydrochloride salt of a compound of formula I, a crystalline form of an isopropanol solvate of a hydrochloride salt of a compound of formula I, a crystalline form A of a sulfate salt of a compound of formula I, a crystalline form B of a sulfate salt of a compound of formula I, a crystalline form C of a sulfate salt of a compound of formula I, a crystalline form of a toluene solvate of a sulfate salt of a compound of formula I, a crystalline form A of a *p-*toluenesulfonate salt of a compound of formula I, a crystalline form A of a benzenesulfonate salt of a compound of formula I, a crystalline form A of a methanesulfonate salt of a compound of formula I, an amorphous form of a methanesulfonate salt of a compound of formula I, a crystalline form of a methanol solvate of a methanesulfonate salt of a compound of formula I, and a crystalline form of a 1,4-dioxane solvate of a methanesulfonate salt of a compound of formula I.

26. A method for treating or preventing a disease or condition for which inhibition of EED provides a benefit, wherein the method comprises administering a subjected in need a crystalline form A of a compound of formula I, a crystalline form B of a compound of formula I, a crystalline form C of a compound of formula I, a crystalline form of a toluene solvate of a compound of formula I, a crystalline form E of a compound of formula I, a crystalline form F of a compound of formula I, a crystalline form G of a compound of formula I, a crystalline form of an N-methyl-2-pyrrolidone solvate of a compound of formula I, a crystalline form of a DMF solvate of a compound of formula I, a crystalline form K of a compound of formula I, a crystalline form A of a hydrochloride salt of a compound of formula I, a crystalline form A of an ethanol solvate of a hydrochloride salt of a compound of formula I, a crystalline form of an isopropanol solvate of a hydrochloride salt of a compound of formula I, a crystalline form A of a sulfate salt of a compound of formula I, a crystalline form B of a sulfate salt of a compound of formula I, a crystalline form C of a sulfate salt of a compound of formula I, a crystalline form of a toluene solvate of a sulfate salt of a compound of formula I, a crystalline form A of a p-toluenesulfonate salt of a compound of formula I, a crystalline form A of a benzenesulfonate salt of a compound of formula I, a crystalline form A of a methanesulfonate salt of a compound of formula I, an amorphous form of a methanesulfonate salt of a compound of formula I, a crystalline form of a methanol solvate of a methanesulfonate salt of a compound of formula I, and a crystalline form of a 1,4-dioxane solvate of a methanesulfonate salt of a compound of formula I.
